# EUROPEAN PATENT SPECIFICATION

(11) **EP 2 091 556 B1**
(45) Date of publication and mention of the grant of the patent: **25.07.2018**
(21) Application number: 07868766.2
(22) Date of filing: 15.11.2007
(51) Int. Cl.: A61K 38/17, A61P 25/00, G01N 33/50, A61P 1/00, C12N 15/00, C12N 15/85

(54) **NON-HUMAN ANIMAL COMPRISING A MUTATION IN THE SREB2/GPR85 GENE AS ANIMAL MODEL OF METABOLIC DISEASE**
NICHT-MENSCHLICHES TIER MIT EINER MUTATION IM SREB2/GPR85 GEN UND TIERMODELL FÜR STOFFWECHSELKRANKHEITEN
MODÈLE ANIMAL NON-HUMAIN POUR DES MALADIES METABOLIQUES COMPRENANT UNE MUTATION DANS LE GÈNE SREB2/GPR85

(30) Priority: 15.11.2006 US 859469 P; 15.11.2006 US 859470 P; 15.11.2006 US 859473 P
(43) Date of publication of application: 26.08.2009
(73) Proprietor: Omeros Corporation, Seattle, WA 98119 (US)
(72) Inventor: GAITANARIS, George A., Seattle, Washington 98102 (US); GRAGEROV, Alexander, Seattle, Washington 98199 (US); HOHMANN, John G., La Conner, Washington 98257 (US); PAVLOVA, Maria N., Seattle, Washington 98105 (US); ZENG, Hongkui, Shoreline, Washington 98133 (US)
(74) Representative: Grünecker Patent- und Rechtsanwälte PartG mbB
(86) International application number: PCT/US2007/084877
(87) International publication number: WO 2008/061209

(56) References cited:
- EP-A- 1 690 939
- WO-A-01/31014
- WO-A-01/32865
- WO-A-02/061432
- WO-A-03/016553
- WO-A-03/065984
- WO-A-2005/047905
- WO-A-2005/050221
- WO-A-2005/075991
- US-A1- 2006 134 109
- MIZUSHIMA K ET AL: "A Novel G-Protein-Coupled Receptor Gene Expressed in Striatum" GENOMICS, ACADEMIC PRESS, SAN DIEGO, US, vol. 69, no. 3, 1 November 2000 (2000-11-01), pages 314-321, XP004437783 ISSN: 0888-7543
- STADEL J M ET AL: "Orphan G protein-coupled receptors: a neglected opportunity for pioneer drug discovery" TRENDS IN PHARMACOLOGICAL SCIENCES, ELSEVIER, HAYWARTH, GB, vol. 18, no. 11, 1 November 1997 (1997-11-01), pages 430-437, XP004099345 ISSN: 0165-6147
- HOWARD A D ET AL: "Orphan G-protein-coupled receptors and natural ligand discovery" TRENDS IN PHARMACOLOGICAL SCIENCES, ELSEVIER, HAYWARTH, GB, vol. 22, no. 3, 1 March 2001 (2001-03-01), pages 132-140, XP004317091 ISSN: 0165-6147
- ARITOSHI IIDA ET AL: "Identification of 156 novel SNPs in 29 genes encoding G-protein coupled receptors" JOURNAL OF HUMAN GENETICS, SPRINGER-VERLAG, TO, vol. 50, no. 4, 1 April 2005 (2005-04-01), pages 182-191, XP019374292 ISSN: 1435-232X
- BÜSCHER R ET AL: "PCR-based methods for identifying genetic variations in human alpha1B- and beta2-adrenergic receptors." MOLECULAR GENETICS AND METABOLISM AUG 1998, vol. 64, no. 4, August 1998 (1998-08), pages 266-270, XP002508965 ISSN: 1096-7192
- TAO ET AL: "Inactivating mutations of G protein-coupled receptors and diseases: Structure-function insights and therapeutic implications" PHARMACOLOGY AND THERAPEUTICS, ELSEVIER, GB, vol. 111, no. 3, 29 July 2006 (2006-07-29), pages 949-973, XP005799423 ISSN: 0163-7258
- SCHONEBERG T ET AL: "Mutant G-protein-coupled receptors as a cause of human diseases" PHARMACOLOGY AND THERAPEUTICS, ELSEVIER, GB, vol. 104, no. 3, 1 December 2004 (2004-12-01), pages 173-206, XP004647272 ISSN: 0163-7258
- HEIN L ET AL: "Adrenergic receptors: From molecular structure to in vivo function" TRENDS IN CARDIOVASCULAR MEDICINE 199707 US, vol. 7, no. 5, July 1997 (1997-07), pages 137-145, XP002508966 ISSN: 1050-1738

## Description

### STATEMENT REGARDING SEQUENCE LISTING

The Sequence Listing associated with this application is provided in text format in lieu of a paper copy, and is hereby incorporated by reference into the specification. The name of the text file containing the Sequence Listing is 700128_405PC_SEQUENCE_LISTING.txt. The text file is 47 KB, was created on November 15, 2007, and is being submitted electronically via EFS-Web to the U.S. PCT Receiving Office, concurrent with the filing of the specification.

### BACKGROUND OF THE INVENTION

### Field of the Invention

The present invention is related to G protein coupled receptors, including GPR88, GPR 22, and the Super-conserved Receptors Expressed in Brain (SREB) G protein coupled receptors, and compounds that modulate their biological activity or expression, as well as the use of these G protein coupled receptors and compounds in the diagnosis, treatment, and prevention of neurological and metabolic diseases and disorders.

### Description of the Related Art

Mammalian G protein coupled receptors (GPCRs) constitute a superfamily of diverse proteins with hundreds of members. GPCRs act as receptors for a multitude of different signals, and most GPCRs are selectively expressed in subsets of cells and tissues. For example, chemosensory GPCRs (csGPCR) are receptors for sensory signals of external origin that are sensed as odors, pheromones, or tastes. Most other GPCRs respond to endogenous signals, such as peptides, lipids, neurotransmitters, or nucleotides. GPCRs falling in the latter group are involved in numerous physiological processes, including the regulation of neuronal excitability, metabolism, reproduction, development, hormonal homeostasis, and behavior, and are differentially expressed in many cell types in the body.

A large number of GPCRs are found in the brain. Excluding the large family of odor receptors, over 89% of known GPCRs are active in the brain. It has been hypothesized that many of these receptors serve as modulators of behavior, memory, cognition, pain, and instinctive functions.

GPCRs, especially those in the nervous system, are ideal targets for drug development. The preference for GPCRs as specific drug targets derives, not only from their central role in biological processes, but also from the discriminating ability that these molecules have in recognizing and responding to their signals. Many GPCRs exist in several similar, but subtly distinct subtypes, which are found in different cells in the body. Such variety of sequence and location provides a high degree of selectivity, allowing the discovery of drugs which specifically affect one subtype of receptor, but not another. This selectivity substantially reduces the risk of unwanted side effects. In addition, most GPCRs are located in the plasma membranes of cells, where they can be easily accessed by pharmaceutical compounds. Given these properties, GPCRs, as a group, have emerged among the most coveted targets for drug development.

In the case of the histamine GPCRs, for instance, subtypes are distributed in the central nervous, cardiopulmonary, and gastrointestinal systems. Yet, each subtype of the histamine receptor is a target of a different medicine. Drugs selective for histamine GPCRs subtypes include Tagamet®, Zantac®, Seldane®, and Dramamine®. Each of these drugs is subtly different from the others, and each has a different target site and therapeutic effect.

Of all currently marketed drugs, greater than 30% are modulators of specific GPCRs. However, only 10% of GPCRs (excluding csGPCRs) are targeted by these drugs, emphasizing the potential of the remaining 90% of the gene family for the treatment of human disease. Clearly, there is a need in the art to identify GPCRs associated with specific diseases, including neurological or metabolic diseases and disorders, as well as modulators thereof, for use in the diagnosis and treatment of associated diseases and disorders.

### BRIEF SUMMARY OF THE INVENTION

The invention is defined in the appended claims.

In one embodiment, the present invention relates to a non-human mammal comprising a mutation in a gene encoding a polypeptide having the amino acid sequence set forth in SEQ ID NO: 1, wherein the mutation leads to a reduced or disrupted expression or activity of the gene, and wherein said non-human mammal exhibits one or more symptoms of a metabolic disease or disorder.

In another embodiment of the present invention, the non-human mammal further comprises a mutation in a second gene encoding a polypeptide having the amino acid sequence set forth in SEQ ID NO: 5, wherein the mutation of the second gene leads to a reduced or disrupted expression or activity of said second gene.

In another embodiment, the present invention relates to an animal model of a metabolic disease or disorder that comprises the non-human mammal of the present invention.

In a further embodiment, the invention relates to a method for identifying a compound for the treatment of a metabolic disease or disorder, said method comprising administering a modulator to an animal or an animal model according to the invention, and determining whether said modulator reduces said one or more symptoms, thereby indicating that said modulator is useful for the treatment of a metabolic disease or disorder.

In another embodiment, the present invention includes a cell isolated from the non-human mammal of the present invention.

In certain embodiments, the metabolic disease or disorder is obesity, diabetes, metabolic syndrome, or anorexia.

### BRIEF DESCRIPTION OF THE SEVERAL VIEWS OF THE DRAWING(S)

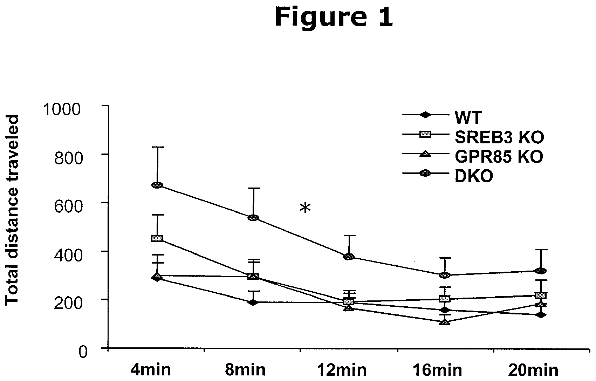

Figure 1 is a graph demonstrating that mice in which both GPR85 and SREB3 have been knocked out (DKO) are hyperactive as assessed in the open field activity test. Data plotted are in 4-min bins. Compared to wild type (WT) mice, the DKO mice exhibit increased locomotor activity as measured by the total distance traveled in the chamber (p<0.05, repeated measures ANOVA, n = 12-14 per group), whereas the SREB3 and GPR85 single knock-outs (Kos) do not behave differently from WT.

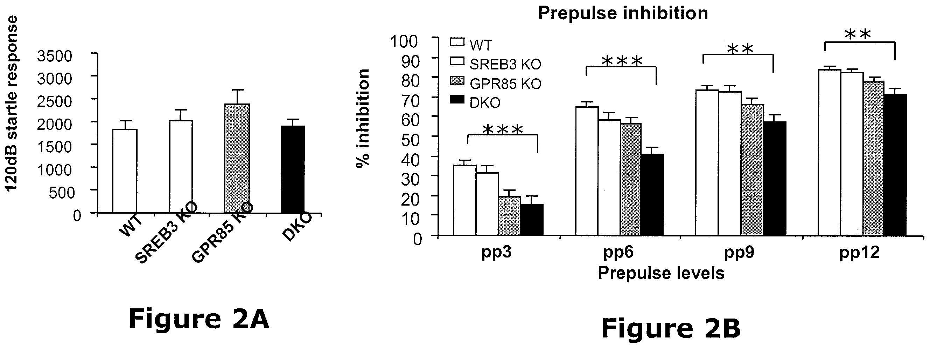

Figures 2A and 2B are graphs demonstrating that DKO mice have reduced prepulse inhibition (PPI). The startle responses to 120dB noise alone are not significantly altered in any of the KO strains (Figure 2A). There is significant reduction of PPI (Figure 2B) in DKO mice at all prepulse levels (**p<0.01, ***p<0.001, n = 19-22 per group, Student's t-test). There is also a partial reduction of PPI in GPR85 KO but not SREB3 KO.

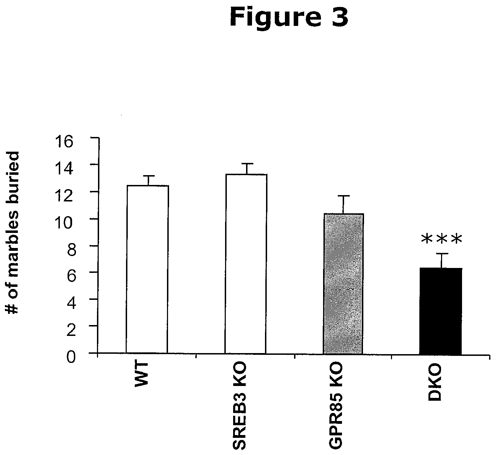

Figure 3 is a graph demonstrating that DKO mice are less anxious or have less obsessive behavior in the marble burying test. There is a significant reduction of number of marbles buried by DKO mice (***p<0.001, n = 19-22 per group, Student's t-test) but not GPR85 KO or SREB3 KO.

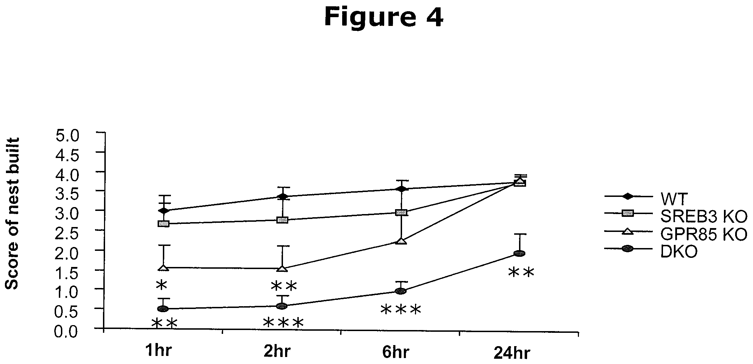

Figure 4 is a graph showing that DKO mice are impaired in nest building. At all time points examined after a piece of nesting material is given, the DKO mice show significant impairment in building the nest (**p<0.01, ***p<0.001, n = 7-10 per group, Mann-Whitney U test). GPR85 KO mice also show significant impairment at 1h and 2h time points (*p<0.05, **p<0.01), while SREB3 KO mice have no significant impairment.

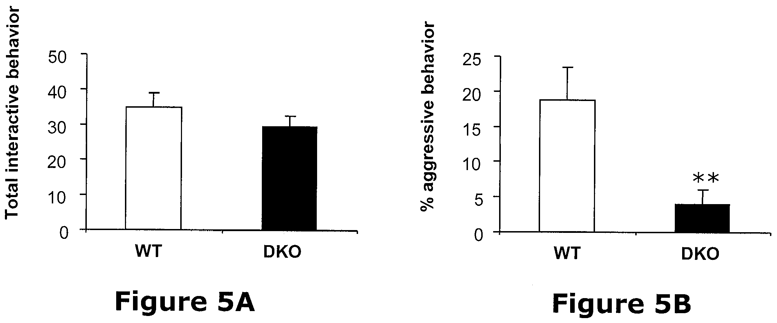

Figures 5A and 5B are graphs demonstrating that DKO mice have reduced aggressive behavior in the resident-intruder test. Only WT and DKO, but not GPR85 KO or SREB3 KO, were tested. The total numbers of all social interactive behavior exhibited by the WT or DKO resident mice, including sniffing, tail chasing and attacking, do not differ significantly by genotype (Figure 5A). There is a significant reduction of aggressive behavior, *i.e.* attacking the intruder mice, in the DKO mice (**p<0.01, n = 16DKO, 21WT, Student's t-test, Figure 5B).

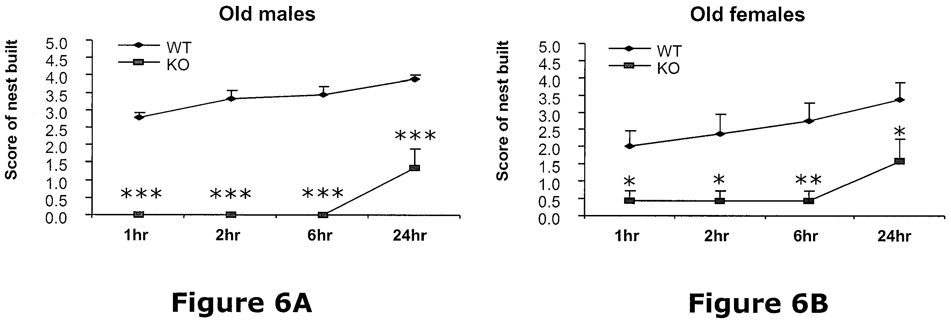

Figures 6A and 6B are graphs demonstrating that aged GPR85 KO mice are also impaired in nest building. When tested at 16-18 months of age, both male (Figure 6A) and female (Figure 6B) GPR85 KO mice show significant impairment in building the nest at all time points examined (*p<0.05, **p<0.01, ***p<0.001, n = 6-9 per group, Mann-Whitney U test).

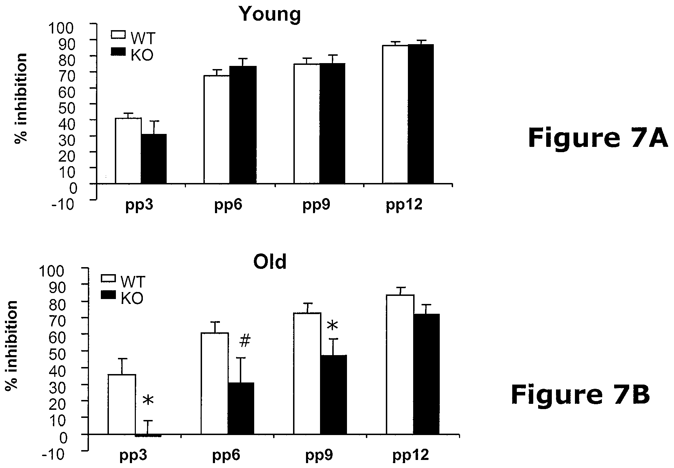

Figures 7A and 7B are graphs that demonstrate that older GPR85 KO mice also have reduced prepulse inhibition (PPI). The same animals are tested at young (3 months, Figure 7A) and old (17 months, Figure 7B) ages. There is a significant reduction of PPI in old GPR85 KO mice (*p<0.05, # p=0.066, n = 6-9 per group, Student's t-test).

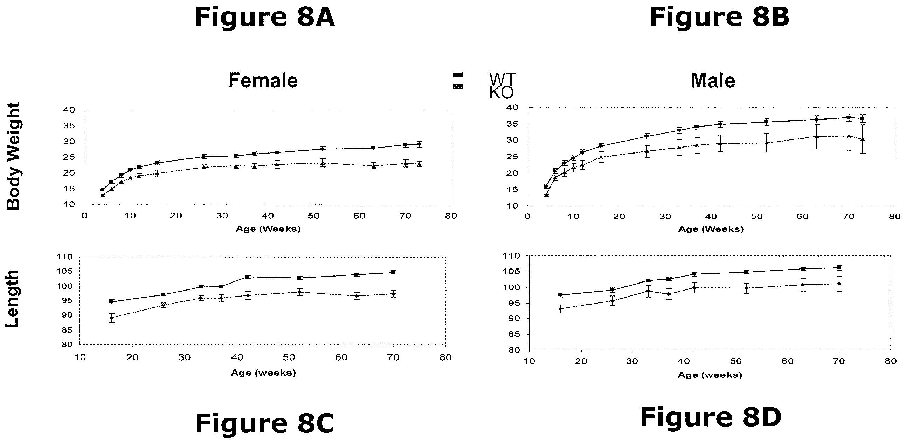

Figures 8A-8D are graphs depicting GPR85 KO mice body weight and body length. Growth curves were generated by measuring the animal's weight and length every one or two weeks. Both male and female KO mice have lower body weight and length than WT, with more significant genotype difference in female mice. P values at 70 weeks are: female weight p<0.0001 (Figure 8A), male weight p=0.12 (Figure 8B), female length p<0.0001 (Figure 8C), male length p<0.05 (Figure 8D)(n = 8-10 per group, Student's t-test).

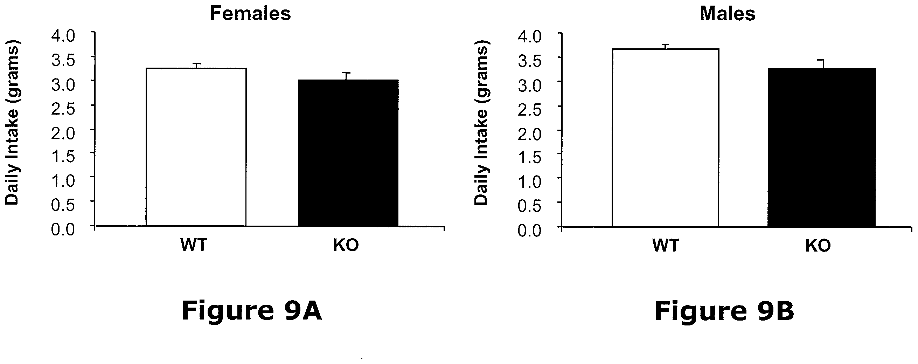

Figures 9A and 9B are graphs demonstrating that GPR85 KO mice have normal baseline food intake. There is no significant difference in the amount of daily food intake between genotypes in either male (Figure 9B) or female (Figure 9A) mice (n = 8-10 per group, Student's t-test).

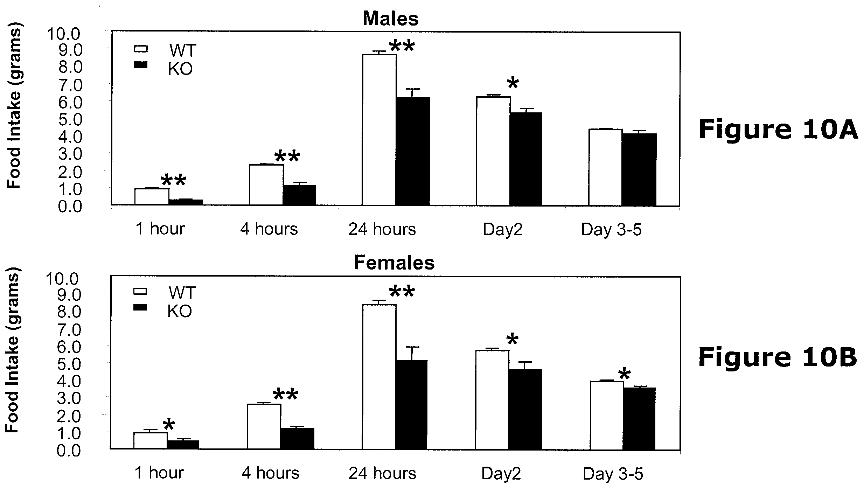

Figure 10A and 10B are graphs demonstrating that GPR85 KO mice eat less after a fasting challenge. Mice are subjected to a 24-hr fasting and then re-feeding. The amount of food eaten and the animal's body weight are measured at 1h, 4h and 24h after re-feeding. Daily food intake is also measured for Days 2-5. Both male (Figure 10A) and female (Figure 10B) KO mice have significantly less cumulative food intake during re-feeding than WT mice (*p<0.05, **p<0.01, n = 8-10 per group, Student's t-test).

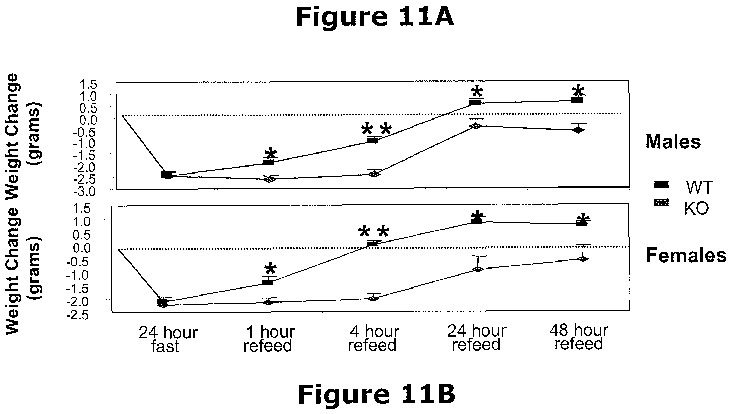

Figures 11A and 11B are graphs showing that GPR85 KO mice are slow to recover body weight after a fasting challenge. Body weight changes are monitored in the same experiment as in Fig. 10. Both male and female KO mice lost the same amount of weight as the WT mice after the fasting (Figures 11A and 11B, respectively). However, during re-feeding, both male and female KO mice are significantly slower in regaining their body weight than WT mice (*p<0.05, **p<0.01, n = 8-10 per group, Student's t-test).

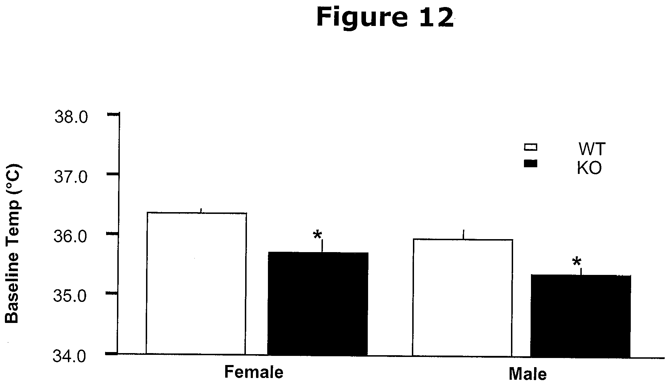

Figure 12 is a graph showing that GPR85 KO mice have lower baseline body temperature. Baseline body temperature is measured by a rectal probe on singly housed, resting mice. Both male and female KO mice have significantly lower body temperature than WT mice (p<0.05, n = 9-11 per group, Student's t-test).

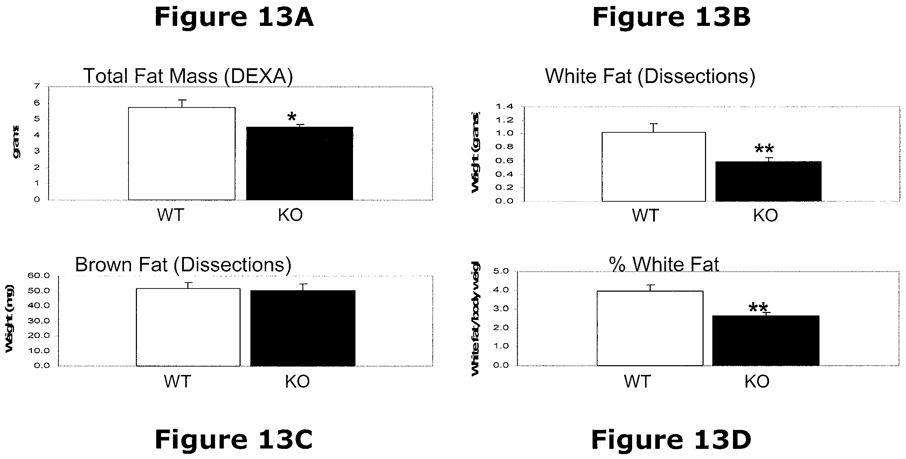

Figures 13A-13D are graphs demonstrating that female GPR85 KO mice have lower percentage of white fat. DEXA analysis shows that KO mice have significantly lower total fat mass than WT mice (p<0.05, n = 10 per group, Student's t-test, Figure 13A). KO mice have significantly lower amount of white fat (p<0.01, Figure 13B) but normal amount of brown fat (Figure 13C). The percentage of white fat versus the whole body weight is also significantly lower in KO mice (p<0.01) by ∼33% (Figure 13D).

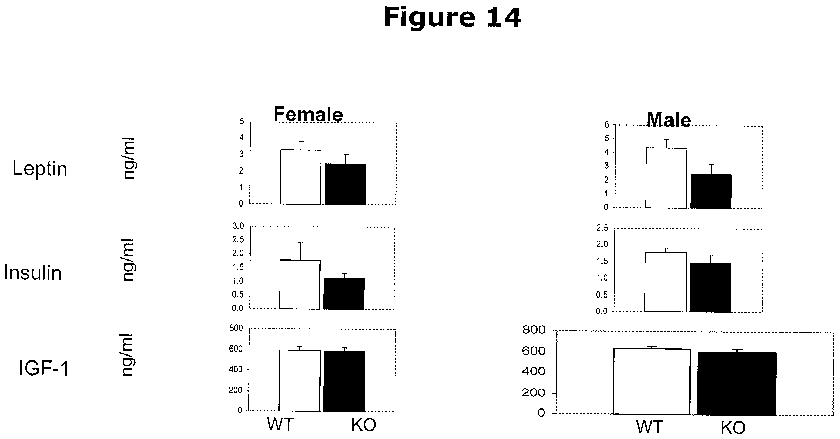

Figure 14 provides graphs showing that GPR85 KO mice have slightly reduced leptin and insulin levels. There is a trend of reduction of baseline plasma leptin and insulin levels in both male and female KO mice (n = 7-10 per group, top four panels), but these differences are not statistically significant. There is no difference in IGF-1 level between genotypes (bottom two panels).

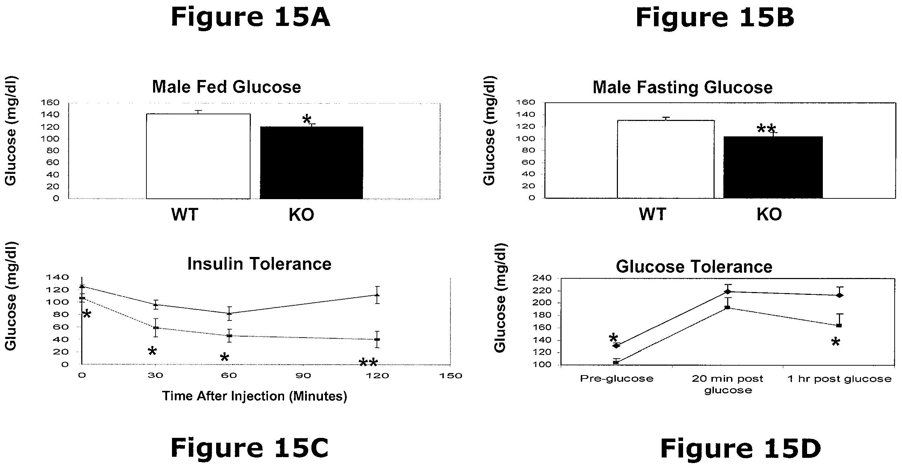

Figures 15A-15D demonstrate that aged male GPR85 KO mice have improved glucose tolerance. Old (16 months) male GPR85 KO mice have significantly lower fed and fasting glucose levels compared to WT mice (Figures 15A and 15B, *p<0.05, **p<0.01, n = 7-10 per group, Student's t-test). These mice are given an insulin challenge or a glucose challenge. In the insulin challenge test (Figure 15C), mice are fasted for 4 hours and then injected with 0.75 U/kg insulin via i.p., and blood glucose levels are measured at different time points afterwards. KO mice have significantly lower glucose levels and are slower in recovery (*p<0.05, **p<0.01). In the glucose challenge test (Figure 15D), mice are injected with 2 g/kg glucose via i.p., and blood glucose levels are measured at different time points afterwards. KO mice are faster in clearing glucose than WT at 1hr post injection (p<0.05).

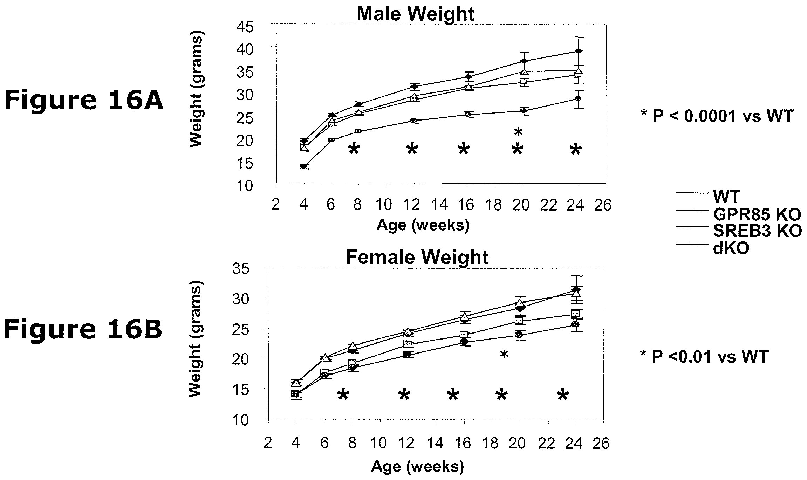

Figures 16A and 16B are graphs showing that DKO mice have greatly reduced body weight and body length. Growth curves are generated by measuring the animal's weight and length every two or four weeks. Both male and female DKO mice have significantly lower body weight and length than WT, with more significant genotype difference in male mice (n = 8-10 per group, Student's t-test). Male DKO mice weigh 27% less than WT (p<0.0001, Figure 16A). Female DKO mice weigh 18% less than WT (p<0.01, Figure 16B).

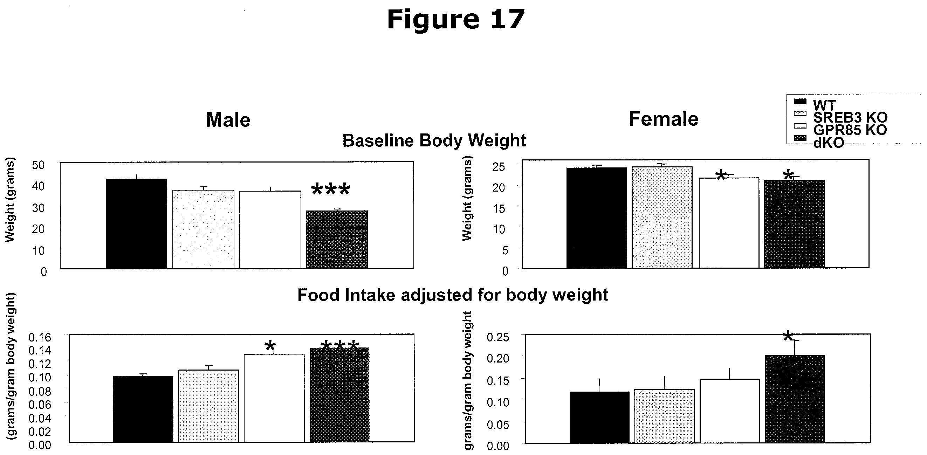

Figure 17 provides graphs showing that DKO mice eat more food per gram body weight. Both male and female DKO mice have lower baseline body weight than WT (upper panels, *p<0.05, ***p<0.001, Student's t-test, n = 6-10 per group, ages 3-5 months). When normalized to their body weights, both male and female DKO mice eat more food per gram body weight (lower panels, *p<0.05, ***p<0.001).

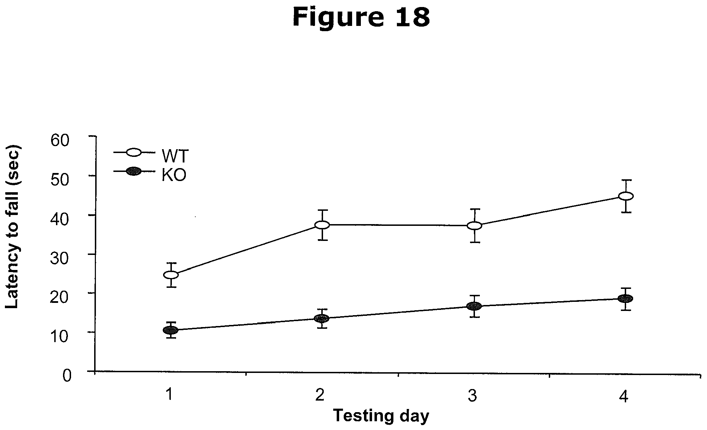

Figure 18 is a graph demonstrating that GPR88 knock-out (KO) mice are impaired in motor coordination as assessed by the rotarod test. Each mouse was tested for 4 trials per day, and the latency to fall from the rotating rod with accelerating rotating speed was averaged across the 4 trials. The KO mice (n=16) had significantly shorter latency compared to wild type (WT) mice (n=18) in each of the four testing days (p<0.01 for day 1, p<0.001 for days 2-4, Student's t-test).

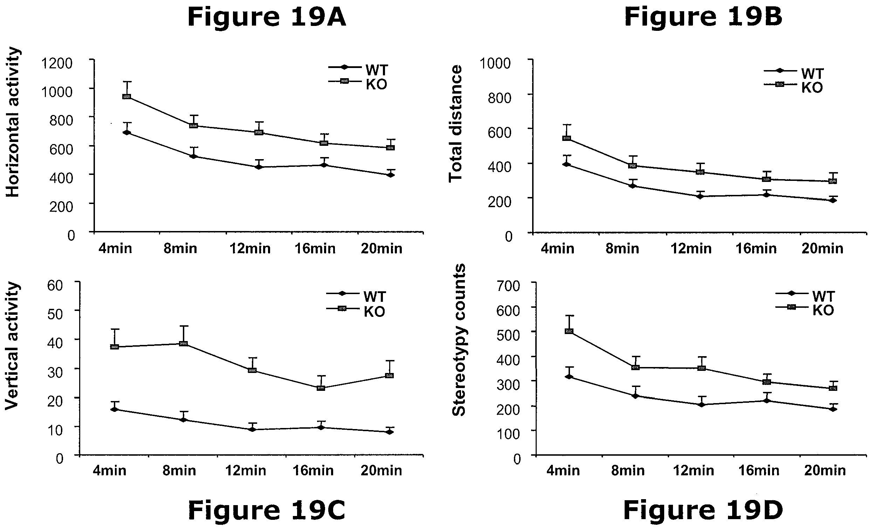

Figures 19A-19D are graphs demonstrating that GPR88 KO mice are hyperactive in the open field activity test. Mice were placed in the novel chamber and their locomotor activities were monitored for 20 min. Data plotted are in 4-min bins. Compared to WT mice (n=18), the KO mice (n=18) had increased horizontal activity (p<0.05, repeated measures ANOVA, Figure 19A), total distance traveled in the chamber (p<0.05, Figure 19B), vertical/rearing activity (p<0.001, Figure 19C) and stereotypy behavior (p<0.05, Figure 19D).

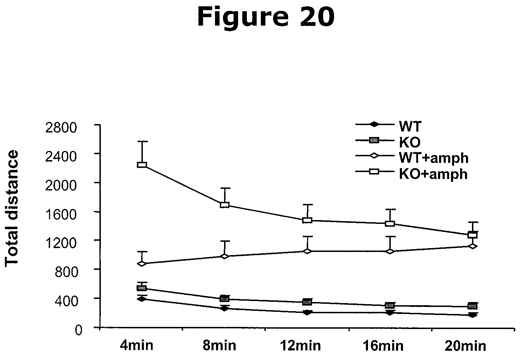

Figure 20 is a graph demonstrating that GPR88 KO mice are hypersensitive to amphetamine-induced hyperactivity. Mice were injected with amphetamine (2.5 mg/kg) via i.p. The mice were placed in the activity chambers 30 minutes after amphetamine injection, and their locomotor activities were monitored for 20 min. Compared to baseline locomotor activity in the absence of drugs, both WT and KO mice had significantly higher activity after amphetamine administration (p<0.001 respectively). However, the activity level of KO mice (n=18) was much higher than that of the WT mice (n=18) (p<0.001 for the first 4-min block, p<0.05 for the second 4-min block, p<0.05 during the whole 20-min period). There was a strong genotype x treatment interaction for the first 4-min block (F(3,68)=10.5, p<0.01, two-way ANOVA).

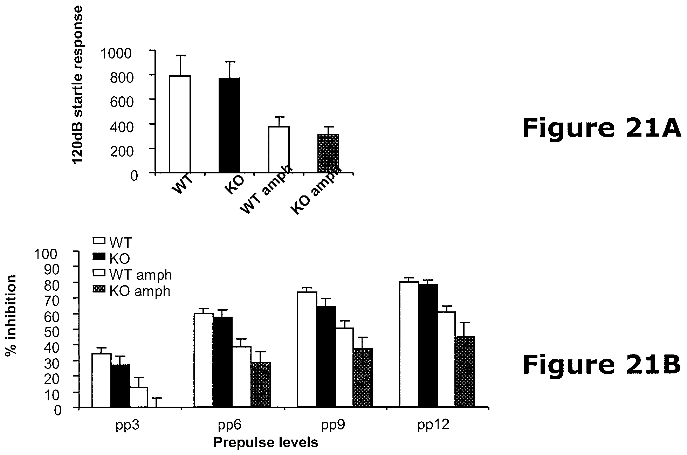

Figures 21A and 21B are graph demonstrating that GPR88 KO mice tend to be more sensitive to amphetamine-induced reduction of prepulse inhibition (PPI). GPR88 KO (n=20) and WT (n=21) mice in the inbred 129sv background were tested for baseline PPI, and there was no significant difference between genotypes (p>0.3, repeated measures ANOVA). Several weeks later, the mice were injected with amphetamine (2.5 mg/kg) via i.p. and tested for PPI 30 minutes later. The startle responses to the 120 dB stimulus of both WT and KO were significantly decreased after amphetamine treatment, and there was no significant difference between genotypes (p>0.5, Figure 4A). There was significant reduction of PPI in both WT and KO after amphetamine treatment (p<0.001 for both, Figure 21B). There was a trend of more reduction of PPI in KO than WT (p=0.091, repeated measures ANOVA).

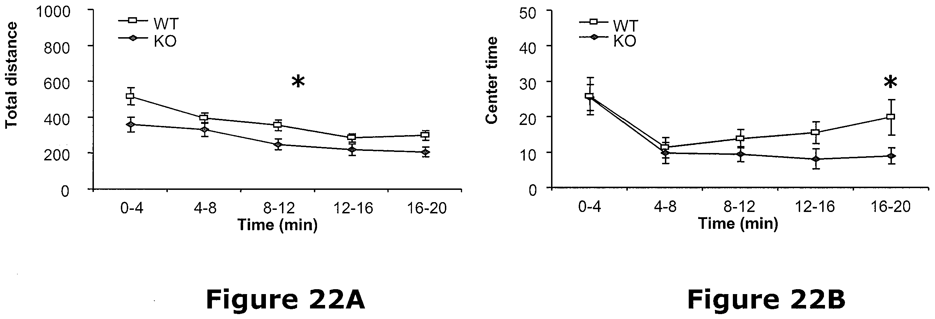

Figures 22A and 22B are graphs demonstrating that GPR22 knock-out (KO) mice are hypoactive and more anxious in the open field activity test. Mice were placed in the novel chamber and their locomotor activities were monitored for 20 min. Data plotted are in 4-min bins. Compared to wild type (WT) mice (n=23), the KO mice (n=27) traveled less distance during the testing period (p<0.05, repeated measures ANOVA) (Figure 22A), and spent less time in the center arena of the chamber (p<0.05, Student's t-test)(Figure 22B).

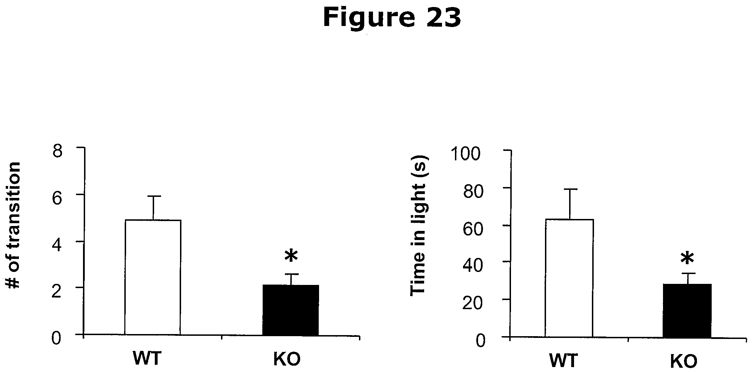

Figure 23 is a graph demonstrating that GPR22 KO mice are more anxious than WT controls in the light-dark box test. In this test, less number of transitions between the light and dark compartments and more time spent in the dark compartment are two indicators for increased anxiety in animals. Compared to WT mice (n=21), the KO mice (n=26) had less number of transitions between the light and dark compartments (p<0.05, Student's t-test), and spent less time in the light compartment (p<0.05).

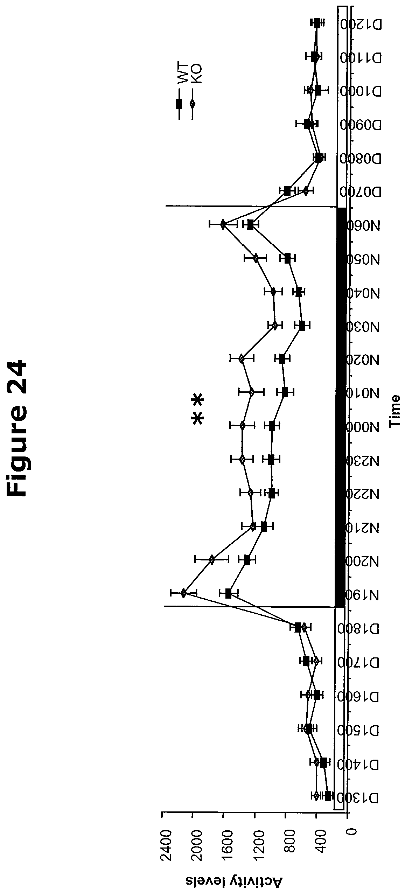

Figure 24 is a graph demonstrating that GPR22 KO mice are hyperactive at night in their home cages. Data plotted are in 1-hr bins. Compared to WT mice (n=23), the KO mice (n=23) had significantly higher locomotor activity levels during the night time (p<0.01, repeated measures ANOVA). During the daytime, locomotor activity levels were not significantly different between genotypes.

### DETAILED DESCRIPTION OF THE INVENTION

Although the size of the GPCR superfamily is still uncertain, recent studies have identified over 300 GPCRs common to humans and mice (US Patent Application Publication No. US2006/0134109). Expression analysis revealed that these GPCRs are expressed in a wide range of tissues, suggesting their importance in numerous physiological processes and associated diseases.

The present invention is based, in part, on the identification of GPCRs that are expressed in the brain and associated with neurological and/or metabolic diseases and disorders, wherein said GPCRs are GPR88, GPR22, or belong to a family of polypeptides called SREBs (Super-Conserved Receptors Expressed in Brain), and have a polypeptide sequence set forth in any one of SEQ ID NOs: 1, 3, 5, 13, and 17. These sequences correspond to SREBs termed GPR85 (SREB2), GPR27 (SREB1), and GPR173 (SREB3), GPR88, and GPR22, respectively. The polynucleotide sequences of GPR85, SREB1, SREB3, GPR88, and GPR22 are provided in SEQ ID NOs: 2, 4, 6, 14, and 18, respectively. The sequences of the mouse polypeptide and polynucleotide homologs are provided in SEQ ID NOs:7 and 8 (mouse GPR85 polypeptide and polynucleotide), SEQ ID NOs:9 and 10 (mouse SREB1 polypeptide and polynucleotide), SEQ ID NOs:11 and 12 (mouse SREB3 polypeptide and polynucleotide), SEQ ID NOs:15 and 16 (mouse GPR88 polypeptide and polynucleotide), and SEQ ID NOs:19 and 20 (mouse GPR22 polypeptide and polynucleotide.

The SREB family of GPCRs is extraordinarily conserved among vertebrate species. The SREB family consists of at least three members, termed SREB1, SREB2, and SREB3. SREB members share 52-63% amino acid identity with each other and show some similarity to previously known rhodopsin-like GPCRs. Amino acid sequence identity between human and rat orthologues is 97% for SREB1 and 99% for SREB3, while the SREB2 sequence is surprisingly completely identical between the species. Furthermore, amino acid sequence of zebrafish SREB2 and SREB3 are 94 and 78% identical to mammal orthologues, respectively. Northern blot analysis revealed that SREB members are predominantly expressed in the brain regions and genital organs. Radiation hybrid analysis localized SREB1, SREB2, and SREB3 genes to different human chromosomes, namely 3p21-p14, 7q31 and Xp11, respectively. The high sequence conservation and abundant expression in the central nervous system suggest that SREBs play fundamental roles in the nervous system.

As described in US Patent Application Publication No. US 2006/0134109, SREB polynucleotides and/or polypeptides are expressed in a variety of neurological tissues, including, hypothalamus, amygdalae, pituitary, female brain, male brain, brainstem, cerebellum, cortex, frontal cortex, hippocampus, striatum, and thalamus. According to the present invention, it has now been found that disruption of two or more SREBs, *e.g.*, GPR85 and SREB3, in mice leads to increased neurological abnormalities (see Examples), thereby demonstrating that SREBs play a fundamental role in these biological processes underlying these neurological conditions. In addition, disruption of GPR85, alone or in combination with a disruption in a second SREB, resulted in lower body weight and improved glucose tolerance, suggesting that SREBs also play a role in metabolic diseases and disorder, such as obesity and diabetes. These results further suggest that the SREBs may play overlapping or redundant roles in the nervous and metabolic systems and that modulating the activity of expression of two or more SREBs may be used to treat associated neurological and metabolic diseases and disorders.

Accordingly, disclosed herein are methods of modulating the biological activity or expression of one or more SREBs, which are useful in treating neurological and metabolic diseases and disorders. In various embodiments of the methods, animals, cells, and kits disclosed herein, the neurological or metabolic disease or disorder is psychosis, schizophrenia, mania, bipolar disorder, obsessive compulsive disorders, autism spectrum disorders, attention-deficit hyperactivity disorders, dementia, mental retardation, other abnormal social behaviors, or other neurodevelopmental disorders. In certain embodiments, the metabolic disease or disorder is obesity, diabetes, metabolic syndrome, or anorexia. Disclosed herein are modulators of one or more SREBs, pharmaceutical compositions comprising modulators of one or more SREBs, and methods of use thereof to treat or prevent neurological and metabolic diseases. In addition, given the association between SREBs and neurological and metabolic diseases and disorders, further disclosed herein are methods of diagnosing or detecting neurological and metabolic diseases or disorders based upon detecting mutations in one or more SREB genes, or alterations in the biological activity or expression of one or more SREBs as compared to normal.

In various embodiments, methods and compounds of disclosed herein may be used to modulate one of the three SREBs described herein, while in other embodiments, methods and compounds of disclosed herein are used to modulate two more more, e.g., two or three, of the SREBs is also contemplated. These methods may employ two or more different modulators, each targeting one or more different SREBs. However, given the high degree of sequence similarity between the SREBs, using one modulator having the ability to modulate two or more SREBs. For example, such a modulator may be an antibody that binds to a conserved extracellular region of SREB1 and SREB2, SREB1 and SREB3, SREB2 and SREB3, or SREB1, SREB2, and SREB3.

The present disclosure is based, in part, on the identification that GPR88 is expressed in the brain and associated with neurological diseases and disorders. As described in US Patent Application Publication No. US 2006/0134109, GPR88 polynucleotides and/or polypeptides are expressed in a variety of neurological tissues, including, hypothalamus, amygdale, female brain, male brain, brainstem, cortex, frontal cortex, hippocampus, striatum, and thalamus. According to the present disclosure, it has now been found that GPR88 is anatomically and functionally involved with the dopamine system and striatal function, thereby suggesting that deregulation of GPR88 expression or activity is associated with a variety of dopamine system- or striatal function-related motor function disease or disorder or other neurological disease or disorder, such as, *e.g.,* Parkinson's disease, Huntington's disease, motor skills disorder, restless legs syndrome, other movement disorders, psychosis, schizophrenia, mania, bipolar disorder, obsessive compulsive disorder, autism spectrum disorders, attention-deficit hyperactivity disorders, dementia, mental retardation, drug abuse and addiction, other abnormal social behaviors, or other neurodevelopmental disease or disorder.

Accordingly, disclosed herein are methods of modulating the biological activity or expression of GPR88, which are useful in treating neurological diseases and disorders, including dopamine system or striatal function-related motor function disease or disorder or other neurological disease or disorder, such as, *e.g.*, Parkinson's disease, Huntington's disease, motor skills disorder, restless legs syndrome, other movement disorders, psychosis, schizophrenia, mania, bipolar disorder, obsessive compulsive disorder, autism spectrum disorders, attention-deficit hyperactivity disorders, dementia, mental retardation, drug abuse and addiction, other abnormal social behaviors, or other neurodevelopmental disease or disorder. Disclosed herein are modulators of GPR88, pharmaceutical compositions comprising modulators of GPR88, and methods of use thereof to treat or prevent neurological diseases. In addition, given the association between GPR88 and neurological diseases and disorders, further disclosed herein are methods of diagnosing or detecting neurological diseases or disorders based upon detecting mutations in the GPR88 gene, or alterations in the biological activity or expression of GPR88 as compared to normal.

The present disclosure is based, in part, on the identification that GPR22 is expressed in the brain and associated with neurological diseases and disorders, including stress, post-traumatic stress disorder (PTSD), anxiety, panic attacks, or a mood or sleep disorder. As described in US Patent Application Publication No. US 2006/0134109, GPR22 polynucleotides and/or polypeptides are expressed in a variety of neurological tissues, including, hypothalamus, amygdala, pituitary, female brain, male brain, brainstem, cerebellum, cortex, frontal cortex, hippocampus, striatum, and thalamus. According to the present invention, it has now been found that disruption of GPR22 in mice leads to increased anxiety and stress, thereby demonstrating that GPR22 plays a fundamental role in the biological processes underlying these neurological conditions.

Accordingly, disclosed herein are methods of modulating the biological activity or expression of GPR22, which are useful in treating neurological diseases and disorders, including stress, post-traumatic stress disorder (PTSD), anxiety, panic attacks, and mood or sleep disorders. Disclosed herein are modulators of GPR22, pharmaceutical compositions comprising modulators of GPR22, and methods of use thereof to treat or prevent neurological diseases. In addition, given the association between GPR22 and neurological diseases and disorders, further disclosed herein are methods of diagnosing or detecting neurological diseases or disorders based upon detecting mutations in the GPR22 gene, or alterations in the biological activity or expression of GPR22 as compared to normal.

The following definitions are provided:
By "polypeptide" is meant any chain of more than two amino acids, regardless of post-translational modification such as glycosylation or phosphorylation. By "substantially identical" is meant a polypeptide or nucleic acid exhibiting at least 50%, preferably 85%, more preferably 90%, and most preferably 95% identity to a reference amino acid or nucleic acid sequence. "Substantially identical" encompasses identical polypeptides. For polypeptides, the length of comparison sequences will generally be at least 16 amino acids, preferably at least 20 amino acids, more preferably at least 25 amino acids, and most preferably 35 amino acids or the full-length polypeptide. For nucleic acids, the length of comparison sequences will generally be at least 50 nucleotides, preferably at least 60 nucleotides, more preferably at least 75 nucleotides, and most preferably 110 nucleotides or the full-length polynucleotide.

Sequence identity is typically measured using a sequence analysis program (*e.g*., BLAST 2; Tatusova et al., FEMS Microbiol Lett. 174:247-250, 1999) with the default parameters specified therein.

By "high stringency conditions" is meant hybridization in 2XSSC at 40° C. with a DNA probe length of at least 40 nucleotides. For other definitions of high stringency conditions, see F. Ausubel et al., Current Protocols in Molecular Biology, pp. 6.3.1-6.3.6, John Wiley & Sons, New York, N.Y., 1994, hereby incorporated by reference. "Substantially identical" polynucleotides also include those that hybridize under high stringency conditions. "Substantially identical" polypeptides include those encoded by polynucleotides that hybridize under high stringency conditions.

By "substantially pure polypeptide" is meant a polypeptide that has been separated from the components that naturally accompany it. Typically, the polypeptide is substantially pure when it is at least 60%, by weight, free from the proteins and naturally-occurring organic molecules with which it is naturally associated. Preferably, the polypeptide is a GPCR polypeptide that is at least 75%, more preferably at least 90%, and most preferably at least 99%, by weight, pure. A substantially pure GPCR polypeptide may be obtained, for example, by extraction from a natural source (*e.g*., a pancreatic cell), by expression of a recombinant nucleic acid encoding a GPCR polypeptide, or by chemically synthesizing the polypeptide. Purity can be measured by any appropriate method, *e.g.*, by column chromatography, polyacrylamide gel electrophoresis, or HPLC analysis.

A polypeptide is substantially free of naturally associated components when it is separated from those contaminants that accompany it in its natural state. Thus, a polypeptide that is chemically synthesized or produced in a cellular system different from the cell from which it naturally originates will be substantially free from its naturally associated components. Accordingly, substantially pure polypeptides include those that naturally occur in eukaryotic organisms but are synthesized in E. coli, yeast or other microbial system.

By "purified antibody" is meant antibody that is at least 60%, by weight, free from proteins and naturally occurring organic molecules with which it is naturally associated. Preferably, the preparation is at least 75%, more preferably 90%, and most preferably at least 99%, by weight, antibody. A purified antibody may be obtained, for example, by affinity chromatography using recombinantly-produced protein or conserved motif peptides and standard techniques.

By "specifically binds" is meant any small molecule, peptide, antibody, or polypeptide that recognizes and binds, for example, a human GPCR polypeptide but does not substantially recognize and bind other molecules in a sample, *e.g.*, a biological sample that naturally includes the protein.

By "polymorphism" is meant that a nucleotide or nucleotide region is characterized as occurring in several different sequence forms. A "mutation" is a form of a polymorphism in which the expression level, stability, function, or biological activity of the encoded protein is substantially altered.

By "GPCR related polypeptide" is meant a polypeptide having substantial identity to a known GPCR polypeptide, including polymorphic forms (*e.g*., sequences having one or more SNPs) and splice variants. GPCR polypeptides include human GPCRs and GPCRs from other animals, including, but not limited to, those GPCRs described in U.S. Patent Application Publication No. US2006/0134109.

By "GPCR biological activity" is meant measurable effect or change in an organism or a cell resulting from the modulation of a GPCR at the molecular, cellular, physiological or behavioral levels or alteration in the extent of activation or deactivation that can be elicited by an agonist or antagonist.

"Dominant negative" means an effect of a mutant form of a gene product that dominately interferes with the function of the normal gene product.

"Reporter system" means any gene, compound or polypeptide whose product can be assayed, measured or monitored. Examples include, but are not limited to neomycin (Kang et al., Mol. Cells; 7:502-508, 1997), luciferase (Welsh et al., Curr. Opin. Biotechnol. 8:617-622, 1997), lacZ (Spergel et al., Prog. Neurobiol. 63:673-686, 2001), aequorin (Deo et al., J. Anal. Chem. 369:258-266, 2001) and green fluorescent protein (Tsien, Annu. Rev. Biochem. 67:509-544, 1998).

"Conditional mutant" is any gene, cell or organism for which the expression of the mutant phenotype can be controlled through alteration in the temperature, diet or other external conditions.

"Overexpression" means level of expression higher than the physiological level of expression.

"Isolated" or "purified" means altered from its natural state, *i.e*., if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated," as the term is employed herein. Moreover, a polynucleotide or polypeptide that is introduced into an organism by transformation, genetic manipulation, or by any other recombinant method is "isolated" even if it is still present in the organism.

"Polynucleotide" generally refers to any polyribonucleotide (RNA) or polydeoxribonucleotide (DNA), which may be unmodified or modified RNA or DNA. Polynucleotides include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. Polynucleotide can also refer to triple helix nucleic acids.

"Variant" refers to a polynucleotide or polypeptide that differs from a reference polynucleotide or polypeptide, but retains the essential properties thereof. A typical variant of a polynucleotide differs in nucleotide sequence from the reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from the reference polypeptide. Generally, alterations are limited so that the sequences of the reference polypeptide and the variant are closely similar overall and, in many regions, identical. A variant and reference polypeptide may differ in amino acid sequence by one or more substitutions, insertions, or deletions in any combination. A substituted or inserted amino acid residue may or may not be one encoded by the genetic code. Typical conservative substitutions include Gly, Ala; Val, lie, Leu; Asp, Glu; Asn, Gln; Ser, Thr; Lys, Arg; and Phe and Tyr. A variant of a polynucleotide or polypeptide may be naturally occurring such as an allele, or it may be a variant that is not known to occur naturally. Non-naturally occurring variants of polynucleotides and polypeptides may be made by mutagenesis techniques or by direct synthesis. Also included as variants are polypeptides having one or more post-translational modifications, for instance glycosylation, phosphorylation, methylation, ADP ribosylation and the like. Embodiments include methylation of the N-terminal amino acid, phosphorylations of serines and threonines and modification of C-terminal glycines. In particular embodiments, variants have at least 75%, at least 80%, at least 85%, at least 90%, at least 95%, or at least 99% identity to a reference polypeptide or polynucleotide.

"Allele" refers to one of two or more alternative forms of a gene occurring at a given locus in the genome.

A "transgenic organism," as used herein, is any organism, including but not limited to animals and plants, in which one or more of the cells of the organism contains heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection, transfection or by infection with a recombinant virus. The transgenic organisms contemplated in accordance with the present invention include mice, bacteria, cyanobacteria, fungi, plants and animals. The isolated DNA of the present invention can be introduced into the host by methods known in the art, for example infection, transfection, transformation or transconjugation.

A "transgenic mouse," as used herein, is a mouse, in which one or more of the cells of the organism contains nucleic acid introduced by way of human intervention, such as by transgenic techniques well known in the art. The nucleic acid is introduced into the cell, directly or indirectly by introduction into a precursor of the cell, by way of deliberate genetic manipulation, by methods known in the art, for example microinjection, infection, transfection, or transformation.

"Transgene" is any exogenously added nucleic acid.

"Antisense" or "Reverse complement" means a nucleic acid sequence complementary to the messenger RNA.

"Single nucleotide polymorphism" or "SNP" refers to the occurrence of nucleotide variability at a single nucleotide position in the genome, within a population. An SNP may occur within a gene or within intergenic regions of the genome. SNPs can be assayed using Allele Specific Amplification (ASA). For this process, at least three primers are required. A common primer is used in reverse complement to the polymorphism being assayed. This common primer can be between 50 and 1500 bps from the polymorphic base. The other two (or more) primers are identical to each other except that the final 3' base wobbles to match one of the two (or more) alleles that make up the polymorphism. Two (or more) PCR reactions are then conducted on sample DNA, each using the common primer and one of the Allele Specific Primers.

"Splice variant" as used herein refers to cDNA molecules produced from RNA molecules initially transcribed from the same genomic DNA sequence but which have undergone alternative RNA splicing. Alternative RNA splicing occurs when a primary RNA transcript undergoes splicing, generally for the removal of introns, which results in the production of more than one distinct mRNA molecules each of which may encode different amino acid sequences. The term splice variant also refers to the polypeptides encoded by the above mRNA molecules.

"Fusion protein" refers to a polypeptide encoded by two, often unrelated, fused genes or fragments thereof.

By "candidate compound" or "test compound" is meant a chemical, be it naturally-occurring or artificially-derived, that is assayed for its ability to modulate gene activity or protein stability or binding, expression levels, or activity, by employing any standard assay method. Test compounds may include, for example, peptides, polypeptides, synthesized organic molecules, naturally occurring organic molecules, polynucleotide molecules, and components thereof.

By "promoter" is meant a minimal sequence sufficient to direct transcription. Also included in the invention are those promoter elements which are sufficient to render promoter-dependent gene expression controllable for cell type-specific, tissue-specific, temporal-specific, or inducible by external signals or agents; such elements may be located in the 5' or 3' or intron sequence regions of the native gene.

By "operably linked" is meant that a gene and one or more regulatory sequences are connected in such a way as to permit gene expression.

Other features and advantages of the invention will be apparent from the following description of embodiments thereof and from the claims.

### A. Methods of Treating and Preventing Neurological and Metabolic Diseases and Disorders

Disclosed herein are methods of modulating the biological activity or expression of one or more SREBs, which methods may be used to treat or prevent a neurological or metabolic disease or disorder, including any one of those described in U.S. Patent Application Publication No. US2006/0134109. In particular embodiments, the neurological disease or disorder is psychosis, schizophrenia, mania, bipolar disorder, obsessive compulsive disorders, autism spectrum disorders, attention-deficit hyperactivity disorders, dementia, mental retardation, other abnormal social behaviors, or other neurodevelopmental disorders. In certain embodiments, the metabolic disease is obesity, diabetes, metabolic syndrome, or anorexia. In particular embodiments, these methods comprise modulating the biological activity or expression of two or more SREBs. In one embodiment, the methods comprise modulating the biological activity of GRCR85 and SREB1, or GPCR85 and SREB3.

Disclosed herein are also methods of modulating the biological activity or expression of GPR88, which methods may be used to treat or prevent a neurological condition, including any of those described in U.S. Patent Application Publication No. US2006/0134109. In particular embodiments, the neurological disease or disorder is a dopamine system- or striatal function-related motor function disease or disorder or other neurological disease or disorder, such as, *e.g*., Parkinson's disease, Huntington's disease, motor skills disorder, restless legs syndrome, other movement disorders, psychosis, schizophrenia, mania, bipolar disorder, obsessive compulsive disorder, autism spectrum disorders, attention-deficit hyperactivity disorders, dementia, mental retardation, drug abuse and addiction, other abnormal social behaviors, or other neurodevelopmental disease or disorder.

Disclosed herein are methods of modulating the biological activity or expression of GPR22, which methods may be used to treat or prevent a neurological condition. In particular embodiments, the neurological disease or disorder is stress, post-traumatic stress disorder (PTSD), anxiety, panic attacks, or a mood or sleep disorder.

In various embodiments, methods or modulating the biological activity or expression of a GPCR, e.g., one or more SREBs, GPR88, or GPR22, include either increasing the activity or expression of a GPCR or decreasing the activity or expression of a GPCR. This may be accomplished genetically, *e.g*., by introducing a GPCR transgene into a cell or animal or by mutating or disrupting genes encoding A GPCR in a cell or animal. This may also be accomplished using one or more compounds that bind to GPCR polypeptides or polynucleotides, or bind to another molecule that regulates the expression or activity or a GPCR, *e.g*., compounds that bind to a GPCR ligand or a transcriptional repressor that binds the promoter of a GPCR gene. Accordingly, the methods disclosed herein may directly affect the activity or expression of a GPCR, or they may indirectly affect the activity or expression of a GPCR. In particular embodiments, wherein the activity of more than one SREB is modulated, these methods are practiced using more than one agent, each which modulates a particular SEB being modulated.

Methods that involve increasing the expression or activity of one or more GPCRs are particularly useful in treating or preventing disease states or conditions characterized by insufficient GPCR signaling (*e*.*g*., as a result of insufficient activity of one or more GPCR ligands). Methods that involve decreasing the expression or activity of one or more GPCRs are particularly useful in treating or preventing disease states or conditions characterized by excessive GPCR signaling. These methods may involve administering a compound that modulates one or more GPCRs, wherein the compound is either an agonist or antagonist of one or more GPCRs to a cell or patient.

In one embodiment, disclosed herein is a method of treating or preventing a neurological or metabolic disease or disorder in a mammal, comprising administering one or more polynucleotides that encode a GPCR, or a functional variant or fragment thereof, to said mammal, wherein said polynucleotides are expressed in said mammal. In certain embodiments, the polynucleotide is an expression vector, and in other embodiments, the polynucleotide is a transgene. In particular embodiments, a neurological or metabolic disease is treated or prevented by administering one or more polynucleotides that encode a SREB. In particular embodiments, a neurological or metabolic disease is treated or prevented by administering two or more polynucleotides that encode polypeptides selected from SREB1, SREB2, and SREB3. In other embodiments, a neurological disease is treated or prevented by administered a polynucleotide that encodes GPR88 or GPR22.

In another embodiment, disclosed herein is a method of treating or preventing a neurological or metabolic disease or disorder in a mammal, comprising administering one or more GPCR polypeptides, or a functional variant or fragment thereof, to said mammal. In particular embodiments, a neurological or metabolic disease is treated or prevented by administering one or more SREB polypeptides. In particular embodiments, a neurological or metabolic disease is treated or prevented by administering two or more polypeptides selected from SREB1, SREB2, and SREB3. In other embodiments, a neurological disease is treated or prevented by administered a GPR88 or GPR22 polypeptide.

In one embodiment, disclosed herein is a method of treating or preventing a neurological or metabolic disease or disorder in a mammal, comprising administering one or more polynucleotides comprising a sequence identical to a region of a SREB polynucleotide sequence set forth in any one of SEQ ID NOs:2, 4, or 6 or a complement thereof, to said mammal, wherein said polynucleotide inhibits expression of one or more SREBs. In certain embodiments, the polynucleotide is an expression vector, and in particular embodiments, the polynucleotide is an antisense RNA, an RNAi molecule, or a ribozyme.

In one embodiment, disclosed herein is a method of treating or preventing a neurological disease or disorder in a mammal, comprising administering a polynucleotide comprising a sequence identical to a region of the GPR88 polynucleotide sequence set forth in SEQ ID NO:2 or a complement thereof, to said mammal, wherein said polynucleotide inhibits expression of GPR88. In certain embodiments, the polynucleotide is an expression vector, and in particular embodiments, the polynucleotide is an antisense RNA, an RNAi molecule, or a ribozyme.

In one embodiment, disclosed herein is a method of treating or preventing a neurological disease or disorder in a mammal, comprising administering a polynucleotide comprising a sequence identical to a region of the GPR22 polynucleotide sequence set forth in SEQ ID NO:2 or a complement thereof, to said mammal, wherein said polynucleotide inhibits expression of GPR22. In certain embodiments, the polynucleotide is an expression vector, and in particular embodiments, the polynucleotide is an antisense RNA, an RNAi molecule, or a ribozyme.

In another embodiment, disclosed herein is a method of treating or preventing a neurological or metabolic disease or disorder in a mammal, comprising administering one or more antibodies that specifically bind to a SREB polypeptide having the sequence set forth in any one of SEQ ID NOs: 1, 3, or 5 to said mammal. In one embodiment, the antibodies inhibit a biological activity of a SREB, while in another embodiment, the antibodies increase a biological activity of a SREB.

In another embodiment, disclosed herein is a method of treating or preventing a neurological disease or disorder in a mammal, comprising administering an antibody that specifically binds to the GPR88 polypeptide having the sequence set forth in SEQ ID NO:1 to said mammal. In one embodiment, the antibody inhibits a biological activity of GPR88, while in another embodiment, the antibody increases a biological activity of GPR88.

In another embodiment, disclosed herein is a method of treating or preventing a neurological disease or disorder in a mammal, comprising administering an antibody that specifically binds to the GPR22 polypeptide having the sequence set forth in SEQ ID NO:1 to said mammal. In one embodiment, the antibody inhibits a biological activity of GPR22, while in another embodiment, the antibody increases a biological activity of GPR22.

Further disclosed herein is a method of treating or preventing a neurological or metabolic disease or disorder in a mammal, comprising administering one or more small molecules that specifically binds to a SREB polypeptide having the sequence set forth in any one of SEQ ID NOs: 1, 3, or 5 to said mammal. In one embodiment, the small molecule inhibits a biological activity of a SREB, while in another embodiment, the small molecule increases a biological activity of a SREB.

Further disclosed herein is a method of treating or preventing a neurological disease or disorder in a mammal, comprising administering a small molecule that specifically binds to the GPR88 polypeptide having the sequence set forth in SEQ ID NO:1 to said mammal. In one embodiment, the small molecule inhibits a biological activity of GPR88, while in another embodiment, the small molecule increases a biological activity of GPR88.

Further disclosed herein is a method of treating or preventing a neurological disease or disorder in a mammal, comprising administering a small molecule that specifically binds to the GPR22 polypeptide having the sequence set forth in SEQ ID NO:1 to said mammal. In one embodiment, the small molecule inhibits a biological activity of GPR22, while in another embodiment, the small molecule increases a biological activity of GPR22.

These methods may be readily adapted for use of any compound that modulates a biological activity or expression of a GPCR, including those specifically described herein. In certain embodiments, two or more compounds that modulate a GPCR, e.g., an SREB, are administered simultaneously or sequentially. These two or more compounds may modulate the same of different GPCRs, e.g., SREBs.

The methods disclosed herein may be used to treat or prevent a variety of neurological or metabolic diseases and disorders in an animal. Thus, disclosed herein are methods of treating or preventing a neurological or metabolic disease or disorder in an animal, comprising administering to the animal one or more compounds that modulate one or more GPCRs' biological activity or expression. In various embodiments, the compounds increase the expression or activity of one or more GPCRs, while in other embodiments, they decrease the expression or activity of one or more GPCRs. In particular embodiments, the animal is a mammal, and in one embodiment, the animal is a human. The animal may be diagnosed with or considered at risk of developing a neurological or metabolic disease or disorder. Neurological or metabolic diseases and disorders that may be treated or prevented include, but are not limited to, those described in US Patent Application Publication No. US 2006/0134109.

Compounds disclosed herein may be administered in a pharmaceutical composition. Pharmaceutical compositions disclosed herein comprise one or more compounds that modulate the expression or activity of one or more GPCrs and a pharmaceutically-acceptable diluent, carrier, or excipient. Pharmaceutical compositions may be administered in unit dosage form.

Any appropriate route of administration may be employed, for example, parenteral, subcutaneous, intramuscular, intracranial, intraorbital, ophthalmic, intraventricular, intracapsular, intraarticular, intraspinal, intracistemal, intraperitoneal, intranasal, aerosol, or oral administration. Examples of specific routes of administration include parenteral, *e.g*., intravenous, intradermal, subcutaneous, oral (*e.g*., inhalation), transdermal (topical), transmucosal, and rectal administration.

Conventional pharmaceutical practice may be employed to provide suitable formulations or compositions to administer such compositions to patients. Methods well known in the art for making pharmaceutical compositions and formulations are found in, for example, Remington: The Science and Practice of Pharmacy, (20th ed.) ed. A. R. Gennaro A R., 2000, Lippincott: Philadelphia. Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for compounds of the invention include ethylenevinyl acetate copolymer particles, osmotic pumps, implantable infusion systems, and liposomes. Formulations for inhalation may contain excipients, or example, lactose, or may be aqueous solutions containing, for example, polyoxyethylene-9-lauryl ether, glycocholate and deoxycholate, or may be oily solutions for administration in the form of nasal drops, or as a gel.

Therapeutic formulations may be in the form of liquid solutions or suspension; for oral administration, formulations may be in the form of tablets or capsules; and for intranasal formulations, in the form of powders, nasal drops, or aerosols. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor ELTM (BASF, Parsippany, N.J.) or phosphate buffered saline (PBS).

The composition should be sterile and should be fluid to the extent that easy syringability exists. It should be stable under the conditions of manufacture and storage and should be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds may be delivered in the form of an aerosol spray from pressured container or dispenser which contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.*, with conventional suppository bases such as cocoa butter and other glycerides) or retention enemas for rectal delivery. In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid.

Liposomal suspensions (including liposomes targeted to specific cell types) can also be used as pharmaceutically acceptable carriers. A variety of liposomal formulations suitable for delivering a compound to an animal have been described and demonstrated to be effective in delivering a variety of compound, including, *e.g*., small molecules, nucleic acids, and polypeptides.

It may be advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. Depending on the type and severity of the disease, about 1 ug/kg to 15 mg/kg (*e.g.,* 0.1 to 20 mg/kg) of antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy can be monitored by standard techniques and assays. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, *e.g*., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

Data obtained from cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (*i.e.*, the concentration of the test compound which achieves a half-maximal inhibition of symptoms) as determined in cell culture. Such information can be used to more accurately determine useful doses in humans. Levels in plasma may be measured, for example, by high performance liquid chromatography.

The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

Disclosed herein are compounds that modulate expression or activity. A compound may, for example, be a small molecule. For example, such small molecules include, but are not limited to, peptides, peptidomimetics, amino acids, amino acid analogs, polynucleotides, polynucleotide analogs, nucleotides, nucleotide analogs, organic or inorganic compounds (i.e. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

It is understood that appropriate doses of small molecule agents depends upon a number of factors within the ken of the ordinarily skilled physician, veterinarian, or researcher. The dose(s) of the small molecule will vary, for example, depending upon the identity, size, and condition of the subject or sample being treated, further depending upon the route by which the composition is to be administered, if applicable, and the effect which the practitioner desires the small molecule to have upon the nucleic acid or polypeptide of the invention.

Appropriate doses of a small molecule also depend upon the potency of the small molecule with respect to the expression or activity to be modulated. Such appropriate doses may be determined using the assays described herein. When one or more of these small molecules is to be administered to an animal (*e.g*., a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

The present invention also encompasses a method of agonizing (stimulating) or antagonizing a GPCR's natural binding partner's associated activity in a mammal, comprising administering to said mammal one or more agonists or antagonists to a GPCR in an amount sufficient to effect said agonism or antagonism. One embodiment of disclosed herein then, is a method of treating diseases in a mammal with an agonist or antagonist of a GPCR comprising administering the agonist or antagonist to a mammal in an amount sufficient to agonize or antagonize one or more GPCR-associated functions.

In one embodiment, disclosed herein is a method for monitoring the effectiveness of treatment of a subject with a compound (*e.g*., an agonist, antagonist, peptidomimetic, protein, peptide, nucleic acid, small molecule, or other drug candidate identified by the screening assays described herein) including the steps of (i) obtaining a pre-administration biological sample from a subject prior to administration of the agent; (ii) detecting the level of expression of a GPCR polypeptide, mRNA, or genomic DNA in the preadministration sample; (iii) obtaining one or more post-administration biological samples from the subject; (iv) detecting the level of expression or activity of a GPCR polypeptide, mRNA, or genomic DNA in the post-administration samples; (v) comparing the level of expression or activity of a GPCR polypeptide, mRNA, or genomic DNA in the pre-administration sample with the polypeptide, mRNA, or genomic DNA in the post administration sample or samples; and (vi) altering the administration of the compound to the subject accordingly. For example, increased administration of the compound may be desirable to increase the expression or activity of a GPCR polypeptide to higher levels than detected, *i.e.*, to increase the effectiveness of the agent. Alternatively, decreased administration of the compound may be desirable to decrease expression or activity of a GPCR polypeptide to lower levels than detected.

### B. Modulators of SREBS for Use in the Treatment or Prevention of Neurological and Metabolic Diseases and Disorders

The methods of the present invention may be practiced using a variety of different compounds that modulate the expression or activity of one or more GPCRs ("modulators"), which are described herein below with reference to certain US patents, published International PCT Patent Applications, texts and scientific journals. In particular embodiments, a GPCR modulator modulates the expression or activity of GPR88, GPR22, or one or more SREBs.

In particular embodiments, modulators are polynucleotides, polypeptides, peptides, peptide nucleic acids, antibodies and fragments thereof, viruses, small molecules, inorganic compounds and organic compounds. Examples of modulators that increase GPCR expression include GPCR polypeptides and polynucleotides, such as transgenes and expression vectors. Examples of modulators that decrease GPCR expression include, *e.g*., knockout constructs, GPCR antisense RNA, GPCR-directed microRNA, and GPCR RNAi molecules. Examples of modulators that decrease GPCR activity include compounds that interfere with GPCR binding to a ligand or inhibit downstream signaling events, such as antagonist antibodies to a GPCR. Examples of modulators that increase GPCR activity include compounds that enhance GPCR binding to a ligand or promote downstream signaling events, such as agonist antibodies to a GPCR.

### 1. Polypeptides and Polynucleotides

In certain embodiments, methods of the invention are practiced using peptide or polypeptide modulators of a GPCR. In certain embodiments, the modulator is a peptide or polypeptide comprising an amino acid sequence identical to or substantially identical to a portion of a GPCR, including human and other GPCRs. The amino acid sequences of human SREBs is provided in SEQ ID NOs:1 (SREB2), 3 (SREB1), and 5 (SREB3). The amino acid sequences of human GPR88 and human GPR22 are provides in SEQ ID NOs:13 and 17, respectively. Thus, in certain embodiments, the polypeptide modulator may be a fragment of a GPCR, and preferably a functional fragment, which has one or more biological activities in common with a full length GPCR, e.g., binding to a ligand or stimulation of a downstream signaling pathway.

Peptides and polypeptides may be readily synthesized or produced recombinantly using routine methods known and available in the art. For example, GPCR polynucleotides can be used as a tool to express a GPCR polypeptide in an appropriate cell *in vitro* or *in vivo* (gene therapy). Methods well known to those skilled in the art may be used to construct expression vectors containing sequences encoding a polynucleotide or polypeptide of interest and appropriate transcriptional and translational control elements. These methods include *in vitro* recombinant DNA techniques, synthetic techniques, and *in vivo* genetic recombination. Such techniques are described, for example, in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York. N.Y.

In one embodiment, GPCR expression is increased using an expression construct that expresses a full length GPCR, or a functional variant or fragment thereof. In various embodiments, the expression construct is adapted for transient expression in a cell, while in other embodiments, the expression construct is adapted for stable expression in a cell. Accordingly, in certain embodiments, the expression construct may be a plasmid or virus.

In another embodiment, GPCR expression is increased by inserting a transgene into an animal. A variety of vectors and constructs suitable for introducing a transgene into an animal's genome have been described and demonstrated to successfully deliver therapeutic levels of a polypeptide to a cell.

In one embodiment, expression constructs of the invention comprise polynucleotide sequences encoding all or a region of a modulator, in addition to regulatory sequences that govern expression of coding sequences. Regulatory sequences present in an expression vector include those non-translated regions of the vector, *e.g.,* enhancers, promoters, 5' and 3' untranslated regions, which interact with host cellular proteins to carry out transcription and translation. Such elements may vary in their strength and specificity. Depending on the vector system and cell utilized, any number of suitable transcription and translation elements, including constitutive and inducible promoters, may be used. A variety of expression systems may be employed for the recombinant production of polypeptides, including, *e.g*., baculovirus, herpes virus, adenovirus, adeno-associated virus, bacterial systems, and eukaryotic systems such as CHO cells. Naked DNA and DNA-liposome complexes can also be used. In particular embodiments, polynucleotides and polypeptides may be introduced into cells in plasmid DNA vectors, viral DNA vectors (such as adenovirus or adeno-associated vectors), or viral RNA vectors (such as retroviral, semliki forest virus, sindbis virus vectors).

In another embodiment, the activity of a GPCR is altered by over expression of a dominant negative inhibitor of a GPCR. Dominant negative inhibitors of GPCR s are typically mutant forms of a GPCR, which reduce or block the activity of wild type GPCR, *e.g.*, by competing for binding to a ligand but failing to fully activate the GPCR signaling pathway. In one embodiment, the dominant negative is a soluble ligand binding domain of a GPCR.

Polypeptide inhibitors also include other variants and fragments of GPCR s having reduced biological activity as compared to wild type GPCRs. One example of a mutant GPCR inhibitor is a GPCR in which a region that binds a ligand is mutated, so that it has reduced binding ability. One example of an inhibitor is a soluble fragment of a GPCR that includes a GPCR extracellular domain and is capable of binding to ligand. Extracellular regions of GPCRs can be predicted based upon the conserved structure of GPCRs.

Examples of peptide modulators of GPCR receptors exhibit the following primary structures: GLGPRPLRFamide, GNSFLRFamide, GGPQGPLRFamide, GPSGPLRFamide, PDVDHVFLRFamide, and pyro-EDVDHVFLRFamide.

Various polynucleotides are contemplated for use as modulators of GPCR expression and/or activity. In one embodiment, a polynucleotide encoding a GPCR or a functional variant or fragment thereof is used to increase GPCR expression, essentially as described above. The polynucleotide sequence of human SREBs is provided in SEQ ID NOs:2 (SREB2), 4 (SREB1), and 6 (SREB3). The polynucleotide sequence of human GPR88 and human GPR22 are provided in SEQ ID NOs:14 and 18, respectively. In certain embodiments, polynucleotides include expression vectors and replacement or insertion vectors designed for integration into the genome of a cell, and suitable for gene therapy, *e.g.*, transgenes, or disruption of allele, *e.g.*, knockout constructs.

In certain embodiments, polynucleotide inhibitors of GPCR s are double-stranded or single-stranded DNA or RNA, including, *e.g*., antisense RNA, ribozymes, or RNA interference reagents designed to specifically target a GPCR polynucleotide, according to methods known and available in the art. Polynucleotide inhibitors may also be DNA-RNA hybrids. Other polynucleotide inhibitors include, *e.*g., targeting vectors designed for integration into the genome and suitable for deleting all or a portion of a GPCR allele or mutating a GPCR allele, *e.g*., through insertional mutagenesis.

In one embodiment, a GPCR inhibitor is an antisense RNA directed to a GPCR polynucleotide, or other components of the signaling cascade. Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal GABA_{A} receptor and human EGF (Jaskulski et al., Science. 1988 Jun 10;240(4858):1544-6; Vasanthakumar and Ahmed, Cancer Commun. 1989;1(4):225-32; Peris et al., Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Furthermore, antisense constructs have also been described that inhibit and can be used to treat a variety of abnormal cellular proliferations, e.g. cancer (U. S. Patent 5,747,470; U. S. Patent 5,591,317 and U. S. Patent 5,783,683).

Therefore, in certain embodiments, disclosed herein are te methods of providing oligonucleotide sequences that comprise all, or a portion of, any sequence that is capable of specifically binding to a GPCR target polynucleotide sequence, or a complement thereof. In another embodiment, the oligonucleotide sequence comprises all, or a portion of a GPCR polynucleotide sequence set forth in any one of SEQ ID NOs:2, 4, 6, 14, or 18, or a complement thereof. In one embodiment, the antisense oligonucleotide comprises DNA or derivatives thereof. In another embodiment, the oligonucleotide comprises RNA or derivatives thereof. The antisense oligonucleotides may be modified DNAs comprising a phosphorothioated modified backbone. Also, the oligonucleotide sequences may comprise peptide nucleic acids or derivatives thereof. In each case, preferred compositions comprise a sequence region that is complementary, and more preferably, completely complementary to one or more portions of a GPCR target gene or polynucleotide sequence.

Methods of producing antisense molecules are known in the art and can be readily adapted to produce an antisense molecule that targets a GPCR. Selection of antisense compositions specific for a given sequence is based upon analysis of the chosen target sequence and determination of secondary structure, Tₘ, binding energy, and relative stability. Antisense compositions may be selected based upon their relative inability to form dimers, hairpins, or other secondary structures that would reduce or prohibit specific binding to the target mRNA in a host cell. Highly preferred target regions of the mRNA include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et al., Nucleic Acids Res. 1997, 25(17):3389-402).

According to another embodiment of methods of the invention, ribozyme molecules are used to inhibit expression of a GPCR target gene or polynucleotide sequence. Ribozymes are RNA-protein complexes having specific catalytic domains that possess endonuclease activity (Kim and Cech, Proc Natl Acad Sci U S A. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et a/., Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction.

At least six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, a hepatitis δ virus, group I intron or RNaseP RNA (in association with an RNA guide sequence) or Neurospora VS RNA motif, for example. Specific examples of hammerhead motifs are described by Rossi et al. Nucleic Acids Res. 1992 Sep 11;20(17):4559-65. Examples of hairpin motifs are described by Hampel *et al.* (Eur. Pat. Appl. Publ. No. EP 0360257), Hampel and Tritz, Biochemistry 1989 Jun 13;28(12):4929-33; Hampel et a/., Nucleic Acids Res. 1990 Jan 25;18(2):299-304 and U. S. Patent 5,631,359. An example of the hepatitis δ virus motif is described by Perrotta and Been, Biochemistry. 1992 Dec 1;31(47):11843-52; an example of the RNaseP motif is described by Guerrier-Takada et al., Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci U S A. 1991 Oct 1;88(19):8826-30; Collins and Olive, Biochemistry. 1993 Mar 23;32(11):2795-9); and an example of the Group I intron is described in U. S. Patent 4,987,071. Important characteristics of enzymatic nucleic acid molecules used according to the invention are that they have a specific substrate binding site which is complementary to one or more of the target gene DNA or RNA regions, and that they have nucleotide sequences within or surrounding that substrate binding site which impart an RNA cleaving activity to the molecule. Thus the ribozyme constructs need not be limited to specific motifs mentioned herein.

Methods of producing a ribozyme targeted to a GPCR are known in the art. Ribozymes may be designed as described in Int. Pat. Appl. Publ. No. WO 93/23569 and Int. Pat. Appl. Publ. No. WO 94/02595, and synthesized to be tested *in vitro* and *in vivo*, as described therein.

Ribozyme activity can be optimized by altering the length of the ribozyme binding arms or chemically synthesizing ribozymes with modifications that prevent their degradation by serum ribonucleases (see e.g., Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298.4; U. S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

RNA interference methods using RNAi molecules also may be used to disrupt the expression of a gene or polynucleotide of interest, including a GPCR gene or another gene associated with a GPCR signaling cascade. While the first described RNAi molecules were RNA:RNA hybrids comprising both an RNA sense and an RNA antisense strand, it has now been demonstrated that DNA sense:RNA antisense hybrids, RNA sense:DNA antisense hybrids, and DNA:DNA hybrids are capable of mediating RNAi (Lamberton, J.S. and Christian, A.T., (2003) Molecular Biotechnology 24:111-119). Accordingly, the invention includes the use of RNAi reagents comprising any of these different types of double-stranded molecules. In addition, it is understood that RNAi reagents may be used and introduced to cells in a variety of forms. Accordingly, as used herein, RNAi reagents encompasses any and all reagents capable of inducing an RNAi response in cells, including, but not limited to, double-stranded polynucleotides comprising two separate strands, *i.e.* a sense strand and an antisense strand, polynucleotides comprising a hairpin loop of complementary sequences, which forms a double-stranded region, e.g., shRNAi molecules, and expression vectors that express one or more polynucleotides capable of forming a double-stranded polynucleotide alone or in combination with another polynucleotide.

In one particular embodiment, a dsRNA molecule that targets and induces degradation of a GPCR polynucleotide is introduced to a cell. While the exact mechanism is not essential to the invention, it is believed the association of the dsRNA to the target gene is defined by the homology between the dsRNA and the actual and/or predicted mRNA transcript. It is believed that this association will affect the ability of the dsRNA to disrupt the target gene. DsRNA methods and reagents are described in PCT applications WO 99/32619, WO 01/68836, WO 01/29058, WO 02/44321, WO 01/92513, WO 01/96584, and WO 01/75164.

In one embodiment disclosed herein RNA interference (RNAi) may be used to specifically inhibit expression of a GPCR. Double-stranded RNA-mediated suppression of gene and nucleic acid expression may be accomplished according to the invention by introducing dsRNA, siRNA or shRNA into cells or organisms. SiRNA may be double-stranded RNA, or a hybrid molecule comprising both RNA and DNA, *e.g*., one RNA strand and one DNA strand. It has been demonstrated that the direct introduction of siRNAs to a cell can trigger RNAi in mammalian cells (Elshabir, S.M., et al. Nature 411:494-498 (2001)). Furthermore, suppression in mammalian cells occurred at the RNA level and was specific for the targeted genes, with a strong correlation between RNA and protein suppression (Caplen, N. et al., Proc. Natl. Acad. Sci. USA 98:9746-9747 (2001)). In addition, it was shown that a wide variety of cell lines, including HeLa S3, COS7, 293, NIH/3T3, A549, HT-29, CHO-KI and MCF-7 cells, are susceptible to some level of siRNA silencing (Brown, D. et al. TechNotes 9(1):1-7, available at http://www.dot.ambion.dot.com/techlib/tn/91/912.html (9/1/02)).

RNAi reagents targeting a GPCR can be readily prepared according to procedures known in the art. Structural characteristics of effective siRNA molecules have been identified. Elshabir, S.M. et al. (2001) Nature 411:494-498 and Elshabir, S.M. et al. (2001), EMBO 20:6877-6888. Accordingly, one of skill in the art would understand that a wide variety of different siRNA molecules may be used to target a specific gene or transcript. In certain embodiments, siRNA molecules according to the invention are 16 - 30 or 18 - 25 nucleotides in length, including each integer in between. In one embodiment, an siRNA is 21 nucleotides in length. In certain embodiments, siRNAs have 0-7 nucleotide 3' overhangs or 0-4 nucleotide 5' overhangs. In one embodiment, an siRNA molecule has a two nucleotide 3' overhang. In one embodiment, an siRNA is 21 nucleotides in length with two nucleotide 3' overhangs (*i.e.* they contain a 19 nucleotide complementary region between the sense and antisense strands). In certain embodiments, the overhangs are UU or dTdT 3' overhangs. Generally, siRNA molecules are completely complementary to one strand of a target DNA molecule, since even single base pair mismatches have been shown to reduce silencing. In other embodiments, siRNAs may have a modified backbone composition, such as, for example, 2'-deoxy- or 2'-O-methyl modifications. However, in preferred embodiments, the entire strand of the siRNA is not made with either 2' deoxy or 2'-O-modified bases.

In one embodiment, siRNA target sites are selected by scanning the target mRNA transcript sequence for the occurrence of AA dinucleotide sequences. Each AA dinucleotide sequence in combination with the 3' adjacent approximately 19 nucleotides are potential siRNA target sites. In one embodiment, siRNA target sites are preferentially not located within the 5' and 3' untranslated regions (UTRs) or regions near the start codon (within approximately 75 bases), since proteins that bind regulatory regions may interfere with the binding of the siRNP endonuclease complex (Elshabir, S. et al. Nature 411:494-498 (2001); Elshabir, S. et al. EMBO J. 20:6877-6888 (2001)). In addition, potential target sites may be compared to an appropriate genome database, such as BLASTN 2.0.5, available on the NCBI server at www.ncbi.nlm, and potential target sequences with significant homology to other coding sequences eliminated.

Short hairpin RNAs may also be used to inhibit or knockdown gene or nucleic acid expression according to the invention. Short Hairpin RNA (shRNA) is a form of hairpin RNA capable of sequence-specifically reducing expression of a target gene. Short hairpin RNAs may offer an advantage over siRNAs in suppressing gene expression, as they are generally more stable and less susceptible to degradation in the cellular environment. It has been established that such short hairpin RNA-mediated gene silencing (also termed SHAGging) works in a variety of normal and cancer cell lines, and in mammalian cells, including mouse and human cells. Paddison, P. et al., Genes Dev. 16(8):948-58 (2002). Furthermore, transgenic cell lines bearing chromosomal genes that code for engineered shRNAs have been generated. These cells are able to constitutively synthesize shRNAs, thereby facilitating long-lasting or constitutive gene silencing that may be passed on to progeny cells. Paddison, P. et al., Proc. Natl. Acad. Sci. USA 99(3):1443-1448 (2002).

ShRNAs contain a stem loop structure. In certain embodiments, they may contain variable stem lengths, typically from 19 to 29 nucleotides in length, or any number in between. In certain embodiments, hairpins contain 19 to 21 nucleotide stems, while in other embodiments, hairpins contain 27 to 29 nucleotide stems. In certain embodiments, loop size is between 4 to 23 nucleotides in length, although the loop size may be larger than 23 nucleotides without significantly affecting silencing activity. ShRNA molecules may contain mismatches, for example G-U mismatches between the two strands of the shRNA stem without decreasing potency. In fact, in certain embodiments, shRNAs are designed to include one or several G-U pairings in the hairpin stem to stabilize hairpins during propagation in bacteria, for example. However, complementarity between the portion of the stem that binds to the target mRNA (antisense strand) and the mRNA is typically required, and even a single base pair mismatch is this region may abolish silencing. 5' and 3' overhangs are not required, since they do not appear to be critical for shRNA function, although they may be present (Paddison et al. (2002) Genes & Dev. 16(8):948-58).

In certain embodiments, the activity of a GPCR is altered is by mutating a gene encoding the GPCR molecule or a gene encoding another component of the GPCR biological pathway. A variety of methods of mutating an endogenous gene are known and available in the art, including, *e.g*., insertional mutagenesis and knockout methods, such as those described herein. Accordingly, disclosed herein are methods of knocking out one or more alleles of a GPCR gene. It is understood that knockout vectors according to the invention include any vector capable of disrupting expression or activity of a GPCR gene, including, in certain embodiments, both gene trap and targeting vectors.

In preferred methods, targeting vectors are used to selectively disrupt one or more GPCR genes. Knockout vectors disclosed herein include those that alter gene expression, for example, by disrupting a regulatory element of GPCR gene, including, *e.g*., inserting a regulatory element that reduces gene expression or deleting or otherwise reducing the activity of an endogenous element that positively affects transcription of the target gene. In other embodiments, knockout vectors of the invention disrupt, *e.g*., delete or mutate, the 5' region, 3' region or coding region of a GPCR gene. In some embodiments, knockout vectors delete a region or the entirety of the coding region of a GPCR gene. In certain embodiments, knockout vectors delete a region of a GPCR gene, while in other embodiments, they insert exogenous sequences into a GPCR gene. In addition, in certain embodiments, including those using replacement vectors, knockout vectors both remove a region of a gene and introduce an exogenous sequence.

Targeting vectors disclosed herein include all vectors capable of undergoing homologous recombination with an endogenous GPCR gene, including replacement vectors. Targeting vectors include all those used in methods of positive selection, negative selection, positive-negative selection, and positive switch selection. Targeting vectors employing positive, negative, and positive-negative selection are well known in the art and representative examples are described in Joyner, A.L., Gene Targeting: A practical Approach, 2nd ed. (2000) and references cited therein.

### 2. Antibodies

Antibodies, or antigen-binding fragments thereof, that specifically bind a GPCR are also modulators, *i.e*., activators or inhibitors, of GPCR s according to the methods described herein. An antibody, or antigen-binding fragment thereof, is said to "specifically bind," "immunogically bind," and/or is "immunologically reactive" to a polypeptide of the invention if it reacts at a detectable level (within, for example, an ELISA assay) with the polypeptide, and does not react detectably with unrelated polypeptides under similar conditions. Antibodies are considered to specifically bind to a target polypeptide when the binding affinity is at least 1x10⁻⁷ M or, preferably, at least 1x10⁻⁸ M. In one embodiment, a modulator is an antibody that specifically binds the extracellular domain of a GPCR.

Antibodies used in the methods of the invention include, but are not limited to, polyclonal antibodies, monoclonal antibodies, chimeric antibodies, humanized antibodies, fully human antibodies, Primatized® antibodies, single chains, Fab fragments and scFv fragments.

Antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies via conventional techniques known in the art, or via transfection of antibody genes into suitable bacterial or mammalian cell hosts, in order to allow for the production of recombinant antibodies. Monoclonal antibodies specific for an antigenic polypeptide of interest may be prepared, for example, using the technique of Kohler and Milstein, Eur. J. Immunol. 6:511-519, 1976, and improvements thereto. Methods of making chimeric and humanized antibodies are well known in the art, (*see, e.g.*, U.S. Pat. No. 4,816,567, International Application No. WO84/03712, respectively).

In certain embodiment, methods of preparing monoclonal antibodies involve the preparation of immortal cell lines capable of producing antibodies having the desired specificity (*i.e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides disclosed herein may be used in the purification process in, for example, an affinity chromatography step.

A number of therapeutically useful molecules are known in the art, which comprise antigen-binding sites that are capable of exhibiting immunological binding properties of an antibody molecule. The proteolytic enzyme papain preferentially cleaves IgG molecules to yield several fragments, two of which (the "F(ab)" fragments) each comprise a covalent heterodimer that includes an intact antigen-binding site. The enzyme pepsin is able to cleave IgG molecules to provide several fragments, including the "F(ab')₂" fragment which comprises both antigen-binding sites. An "Fv" fragment can be produced by preferential proteolytic cleavage of an IgM, and on rare occasions IgG or IgA immunoglobulin molecule. Fv fragments are, however, more commonly derived using recombinant techniques known in the art. The Fv fragment includes a non-covalent V_{H}::V_{L} heterodimer including an antigen-binding site which retains much of the antigen recognition and binding capabilities of the native antibody molecule. Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

A single chain Fv ("sFv") polypeptide is a covalently linked V_{H}::V_{L} heterodimer which is expressed from a gene fusion including V_{H}- and V_{L}-encoding genes linked by a peptide-encoding linker. Huston et al. (1988) Proc. Nat. Acad. Sci. USA 85(16):5879-5883. A number of methods have been described to discern chemical structures for converting the naturally aggregated--but chemically separated--light and heavy polypeptide chains from an antibody V region into an sFv molecule which will fold into a three dimensional structure substantially similar to the structure of an antigen-binding site. See, *e.g*., U.S. Pat. Nos. 5,091,513 and 5,132,405, to Huston et al.; and U.S. Pat. No. 4,946,778, to Ladner et al.

In certain embodiments, each of the above-described molecules includes a heavy chain and a light chain CDR set, respectively interposed between a heavy chain and a light chain FR set which provide support to the CDRS and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (*e.g*., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRS. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structuresregardless of the precise CDR amino acid sequence. Further, certain FR residues are known to participate in non-covalent interdomain contacts which stabilize the interaction of the antibody heavy and light chains.

The invention further includes veneered framework (FR) antibodies. As used herein, the terms "veneered FRs" and "recombinantly veneered FRs" refer to the selective replacement of FR residues from, *e.g*., a rodent heavy or light chain V region, with human FR residues in order to provide a xenogeneic molecule comprising an antigen-binding site which retains substantially all of the native FR polypeptide folding structure. Veneering techniques are based on the understanding that the ligand binding characteristics of an antigen-binding site are determined primarily by the structure and relative disposition of the heavy and light chain CDR sets within the antigen-binding surface. Davies et al. (1990) Ann. Rev. Biochem. 59:439-473. Thus, antigen binding specificity can be preserved in a humanized antibody only wherein the CDR structures, their interaction with each other, and their interaction with the rest of the V region domains are carefully maintained. By using veneering techniques, exterior (*e.g*., solvent-accessible) FR residues which are readily encountered by the immune system are selectively replaced with human residues to provide a hybrid molecule that comprises either a weakly immunogenic, or substantially non-immunogenic veneered surface.

The process of veneering makes use of the available sequence data for human antibody variable domains compiled by Kabat et al., in Sequences of Proteins of Immunological Interest, 4th ed., (U.S. Dept. of Health and Human Services, U.S. Government Printing Office, 1987), updates to the Kabat database, and other accessible U.S. and foreign databases (both nucleic acid and protein). Solvent accessibilities of V region amino acids can be deduced from the known three-dimensional structure for human and murine antibody fragments. There are two general steps in veneering a murine antigen-binding site. Initially, the FRs of the variable domains of an antibody molecule of interest are compared with corresponding FR sequences of human variable domains obtained from the above-identified sources. The most homologous human V regions are then compared residue by residue to corresponding murine amino acids. The residues in the murine FR which differ from the human counterpart are replaced by the residues present in the human moiety using recombinant techniques well known in the art. Residue switching is only carried out with moieties which are at least partially exposed (solvent accessible), and care is exercised in the replacement of amino acid residues which may have a significant effect on the tertiary structure of V region domains, such as proline, glycine and charged amino acids.

In this manner, the resultant "veneered" murine antigen-binding sites are thus designed to retain the murine CDR residues, the residues substantially adjacent to the CDRs, the residues identified as buried or mostly buried (solvent inaccessible), the residues believed to participate in non-covalent (*e.g*., electrostatic and hydrophobic) contacts between heavy and light chain domains, and the residues from conserved structural regions of the FRs which are believed to influence the "canonical" tertiary structures of the CDR loops. These design criteria are then used to prepare recombinant nucleotide sequences which combine the CDRs of both the heavy and light chain of a murine antigen-binding site into human-appearing FRs that can be used to transfect mammalian cells for the expression of recombinant human antibodies which exhibit the antigen specificity of the murine antibody molecule.

Fab or F(ab')₂ fragments may be wholly animal or human derived, or they may be in chimeric form, such that the constant domains are derived from the constant regions of human immunoglobulins and the variable regions are derived from the parent murine MAb. Alternatively, the Fv, Fab, or F(ab')₂ may be humanized, so that only the complementarity determining regions (CDR) are derived from an animal MAb, and the constant domains and the framework regions of the variable regions are of human origin. These chimeric and humanized fragments are less immunogenic than their wholly animal counterparts, and thus more suitable for in vivo use, especially over prolonged periods.

A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al. (1989) Proc. Nat. Acad. Sci. USA 86:4220-4224; Shaw et al. (1987) J Immunol. 138:4534-4538; and Brown et al. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients.

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.,* Jakobovits et al., Proc. Natl. Acad. Sci. USA, 90:2551 (1993); Jakobovits et al., Nature, 362:255-258 (1993); Bruggermann et al., Year in Immuno., 7:33 (1993); and U.S. Pat. Nos. 5,591,669, 5,589,369 and 5,545,807.

In one embodiment, humanized or fully human antibodies of the present invention are prepared according to the methods described in U.S. Patent Nos. 5,770,429, 5,833,985, 5,837,243, 5,922,845, 6,071,517, 6,096,311, 6,111,166, 6,270,765, 6,303,755, 6,365,116, 6,410,690, 6,682,928, and 6,984,720, all assigned to Medarex, Inc.

Alternatively, phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, *e.g.,* Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Pat. Nos. 5,565,332 and 5,573,905.

Human antibodies may also be generated by *in vitro* activated B cells (see U.S. Pat. Nos. 5,567,610 and 5,229,275).

Antibodies to GPCR s may be agonists that increase an activity of a GPCR, or they may be antagonists that decrease an activity of a GPCR. In one embodiment, an antibody serves as an inhibitor of GPCR signaling by binding to a GPCR, *e.g.,* the extracellular domain, and thereby inhibiting binding of a ligand to the GPCR.

### 3. Small Molecules

Modulators (inhibitors or activators) disclosed herein further include large or small inorganic or organic molecules. In certain embodiments, modulators are small organic molecules, or derivatives or analogs thereof. Such small molecules preferably have a molecular weight below 2,000 daltons, more preferably between 300 and 1,000 daltons, and most preferably between 400 and 700 daltons. It is preferred that these small molecules be organic molecules.

In certain embodiments, a modulator includes a protecting group. The term "protecting group" refers to chemical moieties that block at least some reactive moieties and prevent such groups from participating in chemical reactions until the protective group is removed (or "cleaved"). Examples of blocking/protecting groups are described, *e.g.,* in Greene and Wuts, Protective Groups in Organic Synthesis, 3rd Ed., John Wiley & Sons, New York, NY, 1999.

Any of the modulators may possess one or more chiral centers and each center may exist in the R or S configuration. Modulators of the present invention include all diastereomeric, enantiomeric, and epimeric forms as well as mixtures thereof. Stereoisomers may be obtained, if desired, by methods known in the art as, for example, the separation of stereoisomers by chiral chromatographic columns. Modulators further include of N-oxides, crystalline forms (also known as polymorphs), and pharmaceutically acceptable salts, as well as active metabolites of any inhibitor. All tautomers are included within the scope of the modulators presented herein. In addition, the modulators described herein can exist in unsolvated as well as solvated forms with pharmaceutically acceptable solvents such as water, ethanol, and the like. The solvated forms of the modulators presented herein are also included within the present invention.

In a particular embodiment, a small molecule modulator binds to a GPCR, such as SREB1, SREB2, SREB3, GPR88, or GPR22. In one embodiment, a small molecule binds to the extracellular region of a GPCR and interferes or reduces ligand binding to the GPCR. In another embodiment, it binds to the extracellular region of a GPCR and acts as an agonist to stimulate GPCR activity.

Modulators of a GPCR, including small organic compounds, may be identified according to routine screening procedures available in the art, *e.g.,* using commercially available libraries of such compounds.

### 4. Characteristic of GPCR Modulators

The ability of a compound of the present invention to modulate GPCR biological activity or expression may be readily confirmed using routine assays known and available in the art. For expression based assays, a cell or animal is contacted with a compound that modulates GPCR expression, and the expression levels of a GPCR in the cell or animal contacted with the compound are compared to the expression level of the GPCR in cells or an animal not contacted with the compound. Any increase or decrease in GPCR expression levels associated with contact with the compound indicates that the compound modulates GPCR expression. Expression levels of GPCR polypeptides and polynucleotides may be measured by routine procedures in the art, including, *e.g.,* reverse transcriptase-polymerase chain reaction (RT-PCR) or immunological-based assays using an antibody that binds a GPCR. In preferred embodiments, a compound that increases GPCR expression results in an at least two-fold, at least three-fold, at least five-fold, or at least ten-fold increase in GPCR expression, and a compound that decreases GPCR expression results in GPCR expression levels less than 75%, less than 50%, less than 20%, or less than 10% those determined in control cells or animals.

For activity based assays, a cell or animal is contacted with a compound that modulates GPCR expression, and the level of GPCR biological activity in the cell or animal contacted with the compound is compared to the level in cells or an animal not contacted with the compound. Any increase or decrease in GPCR activity associated with contact with the compound indicates that the compound modulates GPCR activity. In preferred embodiments, a compound that increases GPCR activity results in an at least two-fold, at least three-fold, at least five-fold, or at least ten-fold increase in GPCR biological activity, and a compound that decreases GPCR activity results in GPCR activity level less than 75%, less than 50%, less than 20%, or less than 10% that determined in control cells or animals.

Biological activities of GPCR s that may be altered by a modulator include, but are not limited to, the binding of a natural or an unnatural ligand, as well as any one of the functional activities of GPCRs known in the art. Nonlimiting examples of GPCR activities include transmembrane signaling of various forms, which may involve G protein association and/or the exertion of an influence over G protein binding of various guanidylate nucleotides; another exemplary activity of GPCRs is the binding of accessory proteins or polypeptides that differ from known G proteins.

The activity of GPCRs can be determined by, for example, by examining the ability to bind or be activated by chemically synthesized peptide ligands. Alternatively, the activity of GPCR polypeptides can be assayed by examining their ability to bind calcium ions, hormones, chemokines, neuropeptides, neurotransmitters, nucleotides, lipids, odorants, and photons. Alternatively, the activity of the GPCR polypeptides can be determined by examining the activity of effector molecules including, but not limited to, adenylate cyclase, phospholipases and ion channels. Thus, modulators of GPCR polypeptide activity may alter a GPCR receptor function, such as a binding property of a receptor or an activity such as G protein-mediated signal transduction or membrane localization.

In various embodiments of the method, the assay may take the form of an ion flux assay, a yeast growth assay, a non-hydrolyzable GTP assay such as a [35S]-GTPγS assay, a cAMP assay, an inositol triphosphate assay, a diacylglycerol assay, an Aequorin assay, a Luciferase assay, a FLIPR assay for intracellular Ca²⁺ concentration, a mitogenesis assay, a MAP Kinase activity assay, an arachidonic acid release assay (*e.g.,* using [³H]-arachidonic acid), and an assay for extracellular acidification rates, as well as other binding or function-based assays of GPCR activity that are generally known in the art. In several of these embodiments, the invention comprehends the inclusion of any of the G proteins known in the art, such as G 16, G 15, Gs, Gi, Gz, Gq or chimeric G proteins, and the like. SREB activity can be determined by methodologies that are used to assay-for FARP activity, which is well known to those skilled in the art. The assay may take the form of following a change in intracellular localization of the receptor upon binding of a natural or artificial agonist or antagonist. Such cellular trafficking may represent a normal component of a signal transduction pathway, as in "high content screening" (*see, e.g.,* Comley, J.C.W. and Fox, S (2004), Growing market for high content analysis tools, DDW, 5(2):25-34) or may be caused by a receptor modification, as described (O'Dowd, B.F. et al., Dopamine receptor oligomerization visualized in living cells, J. Biol. Chem. (2005), 4:280(44):37225-35).

### C. Identification of GPCR Modulators Useful in the Treatment or Prevention of Neurological and Metabolic Diseases and Disorders

Disclosed herein are methods of identifying and producing compounds useful in the treatment or prevention of neurological or metabolic diseases and disorders, including compounds that modulate an SREB for the treatment or prevention of a neurological or metabolic disease or disorder, and compounds that modulate GPR88 or GPR22 for the treatment or prevention of a neurological disease or disorder. These compounds include modulators (*i.e*., inhibitors and inducers, including antagonists and agonists) of GPCR, e.g., SREB1, SREB2, SREB3, GPR22, and/or GPR88, expression and/or activity.

In general, modulators of GPCR s are identified by screening candidate molecules, including, *e.g.,* all of the different types of molecules described above. Any assay suitable for determining GPCR expression or activity may be utilized, including, but not limited to, binding assays and biological functional assays described herein. A variety of such assays are also described in US Patent Application Publication No. US 2006/0134109. cDNAs encoding GPCRs have been used to successfully screen for new drugs, and assays capable of testing thousands of unknown compounds per day in high-throughput screens (HTSs) are thoroughly documented (*see,* Williams, Medicinal Research Reviews, 1991, 11, 147-184; Sweetnam, etal., J Natural Products, 1993, 56, 441-455 for review).

The invention contemplates at least two different types of inhibitors of GPCRs, including (1) compounds that decrease a functional activity of a GPCR; and (2) compounds that decrease expression levels of a GPCR. An inhibitor of a GPCR is identified as a compound that reduces GPCR activity or expression by at least 10%, at least 25%, at least 50%, at least 75% or 100%.

In general, the invention contemplates two different types of inducers, including (1) compounds that increase the functional activity of a GPCR; and (2) compounds that increase expression levels of a GPCR, including, *e.g.,* a GPCR expression construct. An inducer of a GPCR is identified as a molecule or compound that increases a SREB activity by at least two-fold, at least five-fold, at least ten-fold or more. In the context of overexpression of GPCR s, an inducer is a molecule or compound that increases expression of a GPCR at least two-fold, at least five-fold, at least ten-fold or more.

Modulators may be identified from a variety of sources, for example, cells, cell-free preparations, chemical libraries, collections of chemical compounds, and natural product mixtures. They may be identified using any of the numerous approaches in combinatorial library methods known in the art, including, *e.g.,* screening of biological libraries, screening of spatially addressable parallel solid phase or solution phase libraries, synthetic library methods requiring deconvolution, the 'one-bead one-compound' library method, and synthetic library methods using affinity chromatography selection.

Candidate modulators may be screened individually, *e.g.,* when a specific molecule is predicted to function as an inhibitor or inducer/activator. Candidate modulators may be purified (or substantially purified) molecules or may be one component of a mixture of compounds (*e.g.,* an extract or supernatant obtained from cells). In certain embodiments of a mixed compound assay, GPCR expression or activity is tested against progressively smaller subsets of the candidate compound pool until a single compound or minimal compound mixture is demonstrated to modulate GPCR expression or activity. Alternatively, diverse mixtures (*i.e.,* libraries) of test compounds may be assayed in such a way that the pattern of response indicates which compounds in the various mixtures are responsible for the effect (deconvolution). Libraries of compounds are commercially available and may be synthesized accordingly to methods known in the art.

In one embodiment, disclosed herein is a method of identifying a compound that modulates the expression or activity of a GPCR, e.g., SREB1, SREB2, SREB3, GPR88, or GPR22, comprising contacting a cell comprising the GPCR polypeptide or polynucleotide with a compound and determining whether said contacting results in increased expression or activity of a GPCR polypeptide or polynucleotide. The skilled artisan would understand that a variety of different GPCR polypeptides and polynucleotides may be used in such assays, including full length cDNAs and polypeptides, as well as fragments or variants thereof. Accordingly, it is understood that the methods described herein may be adapted to utilize GPCR polypeptide or polynucleotide fragments or variants. The selectivity of a compound that modulates the activity of GPCR can be evaluated by comparing its effects on a particular GPCR to its effect on other GPCRs.

In certain embodiments, the GPCR polypeptide or polynucleotide employed in these various assays may either be free in solution, attached to a solid support, borne on a cell surface or located intracellularly or associated with a portion of a cell. One skilled in the art can, for example, measure the formation of complexes between the GPCR polypeptide and the compound being tested. Alternatively, one skilled in the art can examine the diminution in complex formation between a GPCR polypeptide and its substrate caused by the compound being tested.

In one embodiment, modulators are identified by their ability to bind to a GPCR or a functional fragment thereof. Binding molecules are useful in modulating (*i.e.,* blocking, inhibiting or stimulating) biological activities of a GPCR polypeptide, especially those activities involved in signal transduction. Accordingly, the present invention provides a method of identifying a modulator of a GPCR comprising contacting one or more GPCR polypeptides and determining whether the compound specifically binds to the GPCR as compared to a control polypeptide. In particular embodiments, at least two-fold, three-fold, five-fold, or ten-fold more modulator will be bound to the GPCR as to an equivalent amount of an unrelated polypeptide.

Routine binding assays suitable for screening candidate molecules and compounds are well known in the art and include, *e.g.,* GST pulldown assays using recombinantly-produced GST-SREB fusion polypeptides, affinity chromatography, phage display, immunoprecipitation assays under low stringency conditions suitable for precipitating GPCR complexes using antibodies to GPCRs, ELISA assays, and radioimmunoassays. In other embodiments, modulators are identified by other means of identifying a GPCR binding partner, such as phage display techniques and yeast two-hybrid screening. Such interactions can be further assayed by means including but not limited to fluorescence polarization or scintillation proximity methods.

In certain embodiments, binding molecules are identified or developed using isolated or recombinant GPCRs, GPCR variants, or cells expressing a GPCR. The use of recombinant GPCR s may allow for better specificity (higher relative purity), provide the ability to generate large amounts of receptor material, and can be used in a broad variety of formats (see Hodgson, Bio/Technology, 1992, 10, 973-980).

In certain embodiment, methods of identifying a modulator of a GPCR comprise identifying a compound that modulates (*i.e.,* increases or decreases) an activity of a GPCR, e.g., SREB1, SREB2, SREB3, GRP88, or GRP22. These methods comprise contacting the GPCR polypeptide with a compound, and determining whether the compound modifies activity of the GPCR polypeptide. Typically, the GPCR polypeptide will be present in a cell or animal. In this approach, the degree of GPCR biological activity in the presence of a candidate compound is compared to the degree of activity in its absence, under equivalent conditions. Where the activity of the sample containing the test compound is higher than the activity in the sample lacking the test compound, the compound will have increased GPCR activity. Similarly, where the activity of the sample containing the test compound is lower than the activity in the sample lacking the test compound, the compound will have inhibited GPCR activity. GPCR biological activity may be measured by any standard assay, for example, those described herein.

In one example, the phosphorylation state or other post-translational modification is monitored as a measure of GPCR biological activity. In addition, activity assays may be based upon the ability of the protein to transduce a signal across a membrane or upon the ability to activate a G protein or another molecule. For example, the ability of a G protein to bind GTP may be assayed. Alternatively, a target of the G protein can be used as a measure GPCR biological activity.

It is well known that activation of heterologous receptors expressed in recombinant systems results in a variety of biological responses, which are mediated by G proteins expressed in the host cells. Occupation of a GPCR by an agonist results in exchange of bound GDP for GTP at a binding site on the G alpha subunit; one can use a radioactive, non-hydrolyzable derivative of GTP, GTPγ[³⁵S], to measure binding of an agonist to the receptor (Sim et al., Neuroreport, 1996, 7, 729-733). One can also use this binding to measure the ability of antagonists to bind to the receptor by decreasing binding of GTPγ[³⁵S] in the presence of a known agonist.

The G proteins can be intact or chimeric. Often, a nearly universally competent G protein (*e.g.,* G16) is used to couple any given receptor to a detectable response pathway. G protein activation results in the stimulation or inhibition of other native proteins, events that can be linked to a measurable response. Examples of such biological responses include, but are not limited to, the following: the ability to survive in the absence of a limiting nutrient in specifically engineered yeast cells (Pausch, Trends in Biotechnology, 1997, 15, 487-494); changes in intracellular Ca2+ concentration as measured by fluorescent dyes (Murphy et al., Cur. Opinion Drug Disc. Dev., 1998, 1, 192-199). Fluorescence changes can also be used to monitor ligand-induced changes in membrane potential or intracellular pH; an automated system suitable for HTS has been described for these purposes (Schroeder et al., J Biomolecular Screening, 1996, 1, 75-80).

Melanophores prepared from Xenopus laevis show a ligand-dependent change in pigment organization in response to heterologous GPCR activation; this response is adaptable to high throughput screening (HTS) formats (Jayawickreme et al., Cur. Opinion Biotechnology, 1997, 8, 629-634). Assays are also available for the measurement of common second messengers, including cAMP, phosphoinositides and arachidonic acid, but these are not generally preferred for HTS.

In particular embodiments, methods utilize permanently transfected CHO cells expressing a recombinant GPCR (or a fragment or variant thereof), in which agonists and antagonists are identified by the ability to specifically alter the binding of GTPγ[³⁵S] in membranes prepared from these cells. In another embodiment of the invention, permanently transfected CHO cells are used for the preparation of membranes which contain significant amounts of the recombinant receptor proteins; these membrane preparations would then be used in receptor binding assays, employing the radiolabelled ligand specific for the particular receptor. Alternatively, a functional assay, such as fluorescent monitoring of ligand-induced changes in internal calcium concentration or membrane potential in permanently transfected CHO cells containing each of these receptors individually or in combination is used for HTS. Such assays may also be performed using other mammalian cells, such as HEK293 or COS cells, permanently transfected insect cell lines, such as Drosophila S2 cells, or recombinant yeast cells expressing the Drosophila melanogaster receptors in high throughput system formats, including those well known in the art (*e.g.,* Pausch, Trends in Biotechnology, 1997, 15, 487-494).

Modulators of GPCR s may be manufactured, *e.g.,* by identifying such a molecule as described above and producing said identified modulator. In addition, identified modulators may be derivatized using standard procedures available in the art and further screened or tested to identify a compound having improved function as an inhibitor or inducer of GPCR expression or activity. When an organic compound is designed, a molecule according to the invention is used as a "lead" compound. The design of mimetics to known pharmaceutically active compounds is a well-known approach in the development of pharmaceuticals based on such "lead" compounds. Mimetic design, synthesis and testing are generally used to avoid randomly screening a large number of molecules for a target property.

### D. Assays of GPCR Expression and Biological Activity

The ability of compounds to alter GPCR expression or biological activity can be confirmed using any of a variety of assays, including those described below. For example, GPCR expression levels may be readily determined at the polynucleotide or polypeptide level using polymerase chain reaction, *e.g.,* RT-PCR, or immunological assays, *e.g.,* cell sorting using antibodies specific for a GPCR. Representative biological assays useful in confirming the biological effect of GPCR modulators are described below.

Agonist and antagonist modulators are useful for up-regulating and down-regulating GPCR activity, respectively, to treat disease states characterized by abnormal levels of GPCR activity, such as neurological or metabolic diseases and disorders. The assays may be performed using single putative modulators, and/or may be performed using a known agonist in combination with candidate antagonists (or visa versa).

### 1. cAMP Assays

In one type of assay, levels of cyclic adenosine monophosphate (cAMP) are measured in GPCR -transfected cells that have been exposed to candidate modulator compounds. Protocols for cAMP assays have been described in the literature (*see, e.g.,* Sutherland et al., Circulation 37: 279 (1968); Frandsen et al., Life Sciences 18: 529-541 (1976); Dooley et al., Journal of Pharmacology and Experimental Therapeutics 283 (2): 735-41 (1997); and George et al., Journal of Biomolecular Screening 2 (4): 235-40 (1997)). An exemplary protocol for such an assay, using an Adenylyl Cyclase Activation FlashPlate Assay from NEN.TM. Life Science Products, is set forth below.

Briefly, the GPCR coding sequence (*e.g.,* a cDNA or intronless genomic DNA) is subcloned into a commercial expression vector, such as pzeoSV2 (Invitrogen), and transiently transfected into Chinese Hamster Ovary (CHO) cells using known methods, such as the transfection protocol provided by Boehringer-Mannheim when supplying the FuGENE 6 transfection reagent. Transfected CHO cells are seeded into 96-well microplates from the FlashPlate (which are coated with solid scintillant to which antisera to cAMP has been bound). For a control, some wells are seeded with wild type (untransfected) CHO cells. Other wells in the plate receive various amounts of a cAMP standard solution for use in creating a standard curve.

One or more compounds (*i.e.,* candidate modulators) are added to the cells in each well, with water and/or compound-free medium/diluent serving as a control or controls. After treatment, cAMP is allowed to accumulate in the cells for exactly 15 minutes at room temperature. The assay is terminated by the addition of lysis buffer containing labeled cAMP, and the plate is counted using a Packard Topcount.TM. 96-well microplate scintillation counter. Unlabeled cAMP from the lysed cells (or from standards) and fixed amounts of cAMP compete for antibody bound to the plate. A standard curve is constructed, and cAMP values for the unknowns are obtained by interpolation. Changes in intracellular cAMP levels of cells in response to exposure to a test compound are indicative of SREB modulating activity

Modulators that act as agonists of receptors which couple to certain G proteins will stimulate production of cAMP, leading to a measurable 2-10 fold increase in cAMP levels. Agonists of receptors which couple to the Gi/z subtype of G proteins will inhibit forskolin stimulated cAMP production, leading to a measurable decrease in cAMP levels of 50-100%. Modulators that act as inverse agonists will reverse these effects at receptors that are either constitutively active or activated by known agonists.

GPCR modulators that act as agonists at receptors that couple to the Gs subtype of G proteins will activate adenyly cyclase leading to a 3-10 fold increase in cyclic adenosine monophosphate (cAMP). Compounds to be tested for the ability to activate GPCR are assayed for cAMP using an Adenylyl Cyclase Activation FlashPlate@ Assay from NEN™ Life Science Products.

In a similar assay to measure cAMP release, a GPCR cDNA is subcloned into the commercial expression vector pCMVSport (Gibco/Life Technologies) and transiently transfected into CHO or COS 7 cells using the transfection reagent FuGENE 6 (Boehringer-Mannheim) and the transfection protocol provided in the product insert. 24 hours post transfection the cells are harvested by dislodging from the culture flask using Versene (Gibco/BRL). The cells are counted and prepared as a suspension in a buffer included in the assay kit that contains the phophodiesterase inhibitor isobutyhnethylxanthine. The assay is conducted in a special 96 well microplate included in the kit which is coated with solid scintillant to which antisera to cAMP has been bound. Dilutions of test compounds to be tested for activation of a GPCR are added to assay wells. Several wells on the plate receive various amounts of cAMP standard solution. After the addition of cells transiently expressing a GPCR, cAMP is allowed to accumulate for exactly 15 minutes at room temperature. The assay is terminated by the addition of lysis buffer containing labelled cAMP, and the plate is covered and allowed to incubate at room temperature for 2-24 hours. The plate is then counted using a Packard Topcount™ 96-well microplate scintillation counter.

Unlabelled cAMP from cells (or standards) competes with fixed amounts of labelled cAMP for antibody bound to the plate. A standard curve is constructed and CAMP values for the unknowns are obtained by interpolation. Data are analyzed using GraphPad Prism (San Diego, Calif.).

### 2. Aequorin Assays

In another assay, cells (*e.g.,* CHO cells) are transiently co-transfected with both a GPCR expression construct and a construct that encodes the photoprotein apoaquorin. In the presence of the cofactor coelenterazine, apoaquorin will emit a measurable luminescence that is proportional to the amount of intracellular (cytoplasmic) free calcium (Cobbold, et al. "Aequorin measurements of cytoplasmic free calcium," In: McCormack J. G. and Cobbold P. H., eds., Cellular Calcium: A Practical Approach. Oxford: IRL Press (199 1); Stables et al., Analytical Biochemistry 252: 115-26 (1997); and Haugland, Handbook of Fluorescent Probes and Research Chemicals, Sixth edition. Eugene Oreg.: Molecular Probes (1996)). In one exemplary assay, a GPCR is subcloned into the commercial expression vector pzeoSV2 (Invitrogen) and transiently co-transfected along with a construct that encodes the photoprotein apoaquorin (Molecular Probes, Eugene, Oreg.) into CHO cells using the transfection reagent FuGENE 6 (Boehringer-Mannheim) and the transfection protocol provided in the product insert.

The cells are cultured for 24 hours at 37 C in MEM (Gibco/BRL, Gaithersburg, Md.) supplemented with 10% fetal bovine serum, 2 mM glutainine, 10 U/ml penicillin and 10 µg/ml streptomycin, at which time the medium is changed to serum-free MEM containing coelenterazine (Molecular Probes, Eugene, Oreg.). Culturing is then continued for two additional hours at 37 C. Subsequently, cells are detached from the plate using VERSEN (Gibco/BRL), washed, and resuspended at 200,000 cells/ml in serum free MEM.

Dilutions of candidate GPCR modulator compounds are prepared in serum free MEM and dispensed into wells of an opaque 96-well assay plate. Plates are then loaded onto an MLX microtiter plate luminometer (Dynex Technologies, Inc., Chantilly, Va.). The instrument is programmed to dispense cell suspensions into each well, one well at a time, and immediately read luminescence for 15 seconds. Dose-response curves for the candidate modulators are constructed using the area under the curve for each light signal peak. Data are analyzed with SlideWrite, using the equation for a one-site ligand, and EC50 values are obtained. Changes in luminescence caused by the compounds are considered indicative of modulatory activity. Modulators that act as agonists at receptors which couple to the Gq subtype of G proteins give an increase in luminescence of up to 100 fold. Modulators that act as inverse agonists will reverse this effect at receptors that are either constitutively active or activated by known agonists. GPCR agonist activation of receptors that couple to the Gq subtype of G proteins will lead to the release of intracellular calcium. The photoprotein aequorin emits a characteristic luminescence in the presence of calcium and may be expressed in cells along with the receptor of interest in order to report agonist signalling.

Briefly, a GPCR cDNA is subcloned into the commercial expression vector pCMVSport (Gibco/Life Technologies) and transiently transfected along with an Aequorin expression construct (Molecular Probes, Eugene, Oreg.) into COS 7 cells using the transfection reagent FuGENE 6 (Boehringer-Mannheim) and the transfection protocol provided in the product insert. 24 hours post transfection the cells are harvested by dislodging from the culture flask using Versene (Gibco/BRL) and prepared as a suspension in assay buffer (Dulbecco's Modified Eagle's Medium with high glucose, pyridoxine HCl, L-glutamine, sodium pyruvate, and 0.1% fetal bovine serum (Gibco/BRL)) and containing the cofactor coelenterazine (Molecular Probes). The cell suspension is incubated for 4 hours at room temperature with gentle stirring. After the coelenterazine loading incubation, the cells are counted and diluted to 1,000,000 cells/ml in assay buffer. Dilutions of test compound are prepared in assay buffer and pipetted into wells of an opaque 96-well assay plate. Plates are loaded onto an MLX microtiter plate luminometer (Dynex Technologies, Chantilly, Va.). The instrument is programmed to dispense cell suspension into each well, one well at a time, and immediately read luminescence for 20 seconds. Dose response curves are constructed using the area under the curve for each light signal peak.

### 3. Luciferase Reporter Gene Assay

The photoprotein luciferase provides another useful tool for assaying for modulators of GPCR activity. Cells (*e.g*., CHO cells or COS 7 cells) are transiently co-transfected with both a GPCR expression construct (e.g., GPCR in pzeoSV2) and a reporter construct which includes a gene for the luciferase protein downstream from a transcription factor binding site, such as the cAMP-response element (CRE), AP-1, or NF-kappa B. Agonist binding to receptors coupled to the G, subtype of G proteins leads to increases in cAMP, thereby activating the CRE transcription factor and resulting in expression of the luciferase gene. Agonist binding to receptors coupled to the Gq subtype of G protein leads to production of diacylglycerol that activates protein kinase C, which activates the AP-1 or NF-kappa B transcription factors, in turn resulting in expression of the luciferase gene. Expression levels of luciferase reflect the activation status of the signaling events (George et al., Journal of Biomolecular Screening, 2(4): 235-240 (1997); and Stratowa et al., Current Opinion in Biotechnology 6: 574-581 (1995)). Luciferase activity may be quantitatively measured using, *e.g.,* luciferase assay reagents that are commercially available from Promega (Madison, Wis.).

In one exemplary assay, CHO cells are plated in 24-well culture dishes at a density of 100,000 cells/well one day prior to transfection and cultured at 37° C in MEM (Gibco/13RL) supplemented with 10% fetal bovine serum, 2 mM glutamine, 10 U/ml penicillin and 10 µg/ml streptomycin. Cells are transiently co-transfected with both a GPCR expression construct and a reporter construct containing the luciferase gene. The reporter plasmids CRE-luciferase, AP1-luciferase and NF-kappaB-luciferase may be purchased from Stratagene (LaJolla, Calif.).

Transfections are performed using the FuGENE 6 transfection reagent (Boehringer-Mannheim) according to the supplier's instructions. Cells transfected with the reporter construct alone are used as a control. Twenty-four hours after transfection, cells are washed once with PBS pre-warmed to 37° C Serum-free MEM is then added to the cells either alone (control) or with one or more candidate modulators and the cells are incubated at 37° C for five hours. Thereafter, cells are washed once with ice-cold PBS and lysed by the addition of lysis buffer from the luciferase assay kit supplied by Promega. After incubation for 15 minutes at room temperature, lysate is mixed with substrate solution (Promega) in an opaque-white, 96-well plate, and the luminescence is read immediately on a Wallace model 1450 MicroBeta scintillation and luminescence counter (Wallace Instruments, Gaithersburg, Md.). Differences in luminescence in the presence versus the absence of a candidate modulator compound are indicative of modulatory activity. Receptors that are either constitutively active or activated by agonists typically give a 3 to 20-fold stimulation of luminescence compared to cells transfected with the reporter gene alone. Modulators that act as inverse agonists will reverse this effect.

### 4. Intracellular Calcium Measurement Using FLIPR

Changes in intracellular calcium levels are another recognized indicator of G protein-coupled receptor activity, and such assays can be employed to screen for modulators of GPCR activity. For example, CHO cells stably transfected with a GPCR expression vector are plated at a density of 40,000 cells/well in 96-well plates specially designed to discriminate fluorescence signals emanating from the various wells on the plate. The cells are incubated for 60 minutes at 37° C in modified Dulbecco's PBS containing pyruvate and 1 g/L glucose with the addition of 1% fetal bovine serum and one of four calcium indicator dyes (Fluo-3™ AM, Fluo-4™ AM, Calcium Green™-1 AM, or Oregon Green™ BAPTA-1 AM). Plates are washed once with modified Dulbecco's PBS without 1% fetal bovine serum and incubated for 10 minutes at 37° C to remove residual dye from the cellular membrane. In addition, a series of washes with modified Dulbecco's PBS without fetal bovine serum is performed immediately prior to activation of the calcium response. A calcium response is initiated by the addition of one or more candidate receptor agonist compounds, calcium ionophore A23187 (positive control), or ATP (positive control). Fluorescence is measured by Molecular Device's FLIPR with an argon laser (excitation 144 at 488 mn) (Kuntzweiler et al., Drug Development Research, 44(1):14-20 (1998)).

Basal fluorescence of cells was measured for 20 seconds prior to addition of candidate agonist, ATP, or A23187, and the basal fluorescence level was subtracted from the response signal. The calcium signal is measured for approximately 200 seconds, taking readings every two seconds. Calcium ionophore A23187 and ATP increase the calcium signal 200% above baseline levels. In general, activated GPCRs increase the calcium signal to approximately 10-15% above baseline signal.

In one embodiment, GPCR HEK293 cells are transiently transfected with an expression vector containing the nucleic acid of a GPCR and empty vector using Lipofectamine plus (Gibco) according to the manufacturer's instructions. The next day, the cells are seeded into 96-well plates at 25,000 cells per well. The following day, cells are loaded with 1 uM Fluo-4-acetoxymethyl fluorescent indicator dye (Molecular Probes) in MEM (minimal essential media) containing 0.1% bovine serum albumin, 0.04% pluronic acid and 2.5 mM probenecid for 30 minutes at 37° C. The cells are washed with pre-warmed (37° C) assay buffer (Hanks buffer containing 15 mM HEPES, 2.5 mM probenecid and 0.1% bovine serum albumin). Assay buffer (100 ul) is added to each well and plates are incubated at 37° C for 15 minutes. Various concentrations (0.03 pM-10 nM) of human Peptide A or salmon Peptide B are added and fluorescence produced by fluo-4 (a calcium sensitive dye) was measured every second for 150 seconds on a fluorometric imaging plate reader (FLIPR; Molecular Devices).

### 5. Mitogenesis

In a mitogenesis assay, the ability of candidate modulators to induce or inhibit a GPCR mediated cell division is determined (*see, e.g.,* Lajiness et al., Journal of Phannacology and Experimental Therapeutics 267(3): 1573-1581-(1-993)). For example, CHO cells stably expressing GPCR are seeded into 96-well plates at a density of 5000 cells/well and grown in MEM with 10% fetal calf serum for 48 hours, at which time the cells are rinsed twice with serum-free MEM. After rinsing, fresh MEM, or MEM containing a known mitogen, is added along with MEM containing varying concentrations of one or more candidate modulators or test compounds diluted in serum-free medium. As controls, some wells on each plate receive serum-free medium alone, and some receive medium containing 10% fetal bovine serum. Untransfected cells or cells transfected with vector alone also may serve as controls. After culture for 16-18 hours, [³H]-thymidine is added to the wells and cells are incubated for an additional 2 hours at 37° C. The cells are trypsinized and collected on filter mats with a cell harvester, the filters are then counted in a Betaplate counter. The incorporation of [³H]-thymidine in serum-free test wells is compared to the results achieved in cells stimulated with serum (positive control). Use of multiple concentrations of test compounds permits creation and analysis of dose-response curves using the non-linear, least squares fit equation: A=BxC/(D+Q+G where A is the percent of serum stimulation; B is the maximal effect minus baseline; C is the EC50; D is the concentration of the compound; and G is the maximal effect. Parameters B, C and G are determined by Simplex optimization. Agonists that bind to the receptor are expected to increase [³H]-thymidine incorporation into cells, showing up to 80% of the response to serum. Antagonists that bind to the receptor will inhibit the stimulation seen with a known agonist by up to 100%.

### 6. GTPyS Binding Assay

Because G protein-coupled receptors signal through intracellular G proteins whose activity involves GTP binding and hydrolysis to yield bound GDP, measurement of binding of the non-hydrolyzable GTP analog [³⁵S]-GTPyS in the presence and absence of candidate modulators provides another assay for modulator activity (*see, e.g.,* Kowal et al., Neuropharinacology 37:179-187 (1998)). In one exemplary assay, cells stably transfected with a GPCR expression vector are grown in 10 cm tissue culture dishes to subconfluence, rinsed once with 5 ml of ice-cold Ca2+/Mg2+-free phosphate-buffered saline, and scraped into 5 ml of the same buffer. Cells are pelleted by centrifugation (500.times.g, 5 minutes), resuspended in TEE buffer (25 mM Tris, pH 7.5, 5 mM EDTA, 5 mM EGTA), and frozen in liquid nitrogen. After thawing, the cells are homogenized using a Dounce homogenizer (one ml TEE per plate of cells), and centrifuged at 1,000xg for 5 minutes to remove nuclei and unbroken cells

The homogenate supernatant is centrifuged at 20,000xg for 20 minutes to isolate the membrane fraction, and the membrane pellet is washed once with TEE and resuspended in binding buffer (20 mM HEPES, pH 7.5, 150 mM NaCl, 10 mm MgCI2, 1 mM EDTA). The resuspended membranes can be frozen in liquid nitrogen and stored at -70° C until use. Aliquots of cell membranes prepared as described above and stored at -70° C are thawed, homogenized, and diluted. Final homogenates are incubated with varying concentrations of candidate modulator compounds or GTP for 30 minutes at 30° C and then placed on ice. To each sample, guanosine 5'-0-(3 [³⁵S thio)triphosphate (NEN, 1200 Ci/mmol; [³⁵S]-GTPγS), was added to a final concentration of 100-200 pM. Samples are incubated at 30° C for an additional 30 minutes, 1 ml of 10 mM HEPES, pH 7.4, 10 mM MgCI2, at 4° C is added and the reaction is stopped by filtration.

Samples are filtered over Whatman GF/B filters and the filters are washed with 20 ml ice-cold 10 mM HEPES, pH 7.4, 10 mM MgCI2. Filters are counted by liquid scintillation spectroscopy. Nonspecific binding of [³⁵S]-GTPγS is measured in the presence of GTP and subtracted from the total. Compounds are selected that modulate the amount of [³⁵S]-GTPγS binding in the cells, compared to untransfected control cells. Activation of receptors by agonists gives up to a five-fold increase in [³⁵S] GTPγS binding. This response is blocked by antagonists.

### 7. MAP Kinase Activity Assay

Evaluation of MAP kinase activity in cells expressing a GPCR provides another assay to identify modulators of SREB activity (Lajiness et al., Journal of Pharmacology and Experimental Therapeutics 267(3):1573-1581 (1993) and Boulton etal., Cell 65: 663-675 (1991)). In one embodiment, CHO cells stably transfected with a GPCR are seeded into 6-well plates at a density of 70,000 cells/well 48 hours prior to the assay. During this 48-hour period, the cells are cultured at 37° C in MEM medium supplemented with 10% fetal bovine serum, 2 mM glutamine, 10 U/ml penicillin and streptomycin. The cells are serum-starved for 1-2 hours prior to the addition of stimulants.

For the assay, the cells are treated with medium alone or medium containing either a candidate agonist or 200 nM Phorbol ester-myristoyl acetate (*i.e.,* PMA, a positive control), and the cells are incubated at 37° C for varying times. To stop the reaction, the plates are placed on ice, the medium is aspirated, and the cells are rinsed with 1 ml of ice-cold PBS containing EDTA. Thereafter, cell lysis buffer is added to the cells. The cells are scraped from the plates and homogenized by 10 passages through a 23 G needle, and the cytosol fraction is prepared by centrifugation at 20,000xg for 15 minutes. Aliquots of cytosol are mixed with MAPK Substrate Peptide (APRTPGGRR), Upstate Biotechnology, Inc., N.Y.) and [γ-³²P]-ATP (NEN, 3000 Ci/mmol), diluted to a final specific activity of 2000 cpm/pmol. The samples are incubated for 5 minutes at 30° C, and reactions are stopped by spotting on Whatman P81 phosphocellulose paper. The filter squares are washed and are subjected to liquid scintillation spectroscopy to quantitate bound label. Equivalent cytosolic extracts are incubated without MAPK substrate peptide, and the bound label from these samples are subtracted from the matched samples with the substrate peptide. The cytosolic extract from each well is used as a separate point. Protein concentrations are determined by a dye binding protein assay (Bio-Rad Laboratories). Agonist activation of the receptor is expected to result in up to a five-fold increase in MAPK enzyme activity. This increase is blocked by antagonists.

### 8. Arachidonic Acid Release

The activation of GPCRs also has been observed to potentiate arachidonic acid release in cells, providing yet another useful assay for modulators of GPCR activity (Kanterman et al., Molecular Pharmacology 3 9:3 64-3 69 (199 1)). For example, CHO cells that are stably transfected with a GPCR expression vector are plated in 24 well plates at a density of 15,000 cells/well and grown in MEM medium supplemented with 10% fetal bovine serum, 2 mM glutamine, 10 U/ml penicillin and streptomycin for 48 hours at 37° C before use. Cells of each well are labeled by incubation with [3H]-arachidonic acid (Amersham Corp., 210 Ci/mmol) for 2 hours at 37° C. The cells are then washed twice with 1 ml of buffer. Candidate modulator compounds are added in 1 ml of the same buffer, either alone or with ATP and the cells are incubated at 37° C for 30 minutes. Buffer alone and mock transfected cells are used as controls. Samples (0.5 ml) from each well are counted by liquid scintillation spectroscopy. Agonists that activate the receptor will lead to potentiation of the ATP-stimulated release of [³H]-arachidonic acid. This potentiation is blocked by antagonists.

### 9. Extracellular Acidification Rate

In yet another assay, the effects of candidate modulators of GPCR activity are assayed by monitoring extracellular changes in pH induced by the test compounds (*see, e.g.,* Dunlop et al., Journal of Pharmacological and Toxicological Methods 40(1):47-55 (1998)). In one embodiment, CHO cells transfected with one or more GPCR expression vector are seeded into 12 min capsule cups (Molecular Devices Corp.) at 400,000 cells/cup in MEM supplemented with 10% fetal bovine serum, 2 mM L-glutamine, 10 U/ml penicillin, and 10 µg/ml streptomycin. The cells are incubated in this medium at 37° C in 5% CO₂ for 24 hours. Extracellular acidification rates are measured using a Cytosensor microphysiometer (Molecular Devices Corp.). Candidate agonists or other agents are diluted into the running buffer and perfused through a second fluid path. The pH of the running buffer in the sensor chamber is recorded during the cycle from 43-58 seconds, and the pump is re-started at 60 seconds to start the next cycle. The rate of acidification of the running buffer during the recording time is calculated by the Cytosoft program. Changes in the rate of acidification are calculated by subtracting the baseline value (the average of 4 rate measurements immediately before addition of a modulator candidate) from the highest rate measurement obtained after addition of a modulator candidate. Modulators that act as agonists of the receptor result in an increase in the rate of extracellular acidification compared to the rate in the absence of agonist. This response is blocked by modulators that act as antagonists of the receptor.

### 10. High content screening

In another assay, confocal microscopy and a fluorescent cellular component, for example a protein are used to follow either changes in receptor intracellular localization caused by its activation, or receptor activation-induced protein-protein interaction, such as interaction of the receptor with β-arrestin, or a change in cell shape, or any change in the cellular distribution of a fluorescently labeled component (*see* Comley, JCW and Fox, S (2004), Growing market for high content analysis tools. DDW, 5 (2): 25-34 for a review). A number of instruments are available to conduct this type of analysis in a high throughput manner, including, *e.g.,* Cellomics, Inc. ArrayScan® VTI HCS Reader.

### 11. Nuclear translocation technology

Another assay uses incorporation of a nuclear localization sequence (NLS, a 5 amino acid substitution) in a location in helix 8 of the GPCR structure, resulting in ligand independent receptor translocation from the cell surface to the nucleus. Blockade of the GPCR-NLS translocation from the cell surface may be achieved by either antagonist or agonist treatments (O'Dowd, B.F. et al. (2005) Dopamine receptor oligomerization visualized in living cells. J. Biol. Chem. 280(44):37225-35.). The GPCR-NLS trafficking may by visualized by fusion to optically detectable molecules. Alternatively, it can be quantified by determining the density of cell surface receptors using enzyme fragment complementation in a manner suitable for high throughput screening.

### E. Animal Model Systems

Compounds that modulate GPCR expression or activity, including compounds identified as having activity in any of the above-described assays, may be subsequently screened in any available animal model system, including, but not limited to, mice, rats, pigs, and dogs, to determine their *in vivo* effect on GPCR expression or activity, as well as their effect on neurological or metabolic characteristics of the animal. Modulators are administered to these animals according to standard methods. In particular embodiments, modulators are administered to animal models of human neurological or metabolic disease. In particular embodiments, modulators are tested in animals bearing a mutation in a gene encoding a GPCR polypeptide, such as an animal having a mutation in one or more GPCR genes, e.g., SREB1, SREB2, SREB3, GPR22, or GRP88.

As demonstrated in the Examples, GPR88, GPR22, and SREB knockout mice display altered neurological behaviors s. In addition, SREB knockout mice display altered metabolic characteristics. Accordingly, GPCR knockout animals may be used to confirm the therapeutic activity of modulators of GPCRs. Accordingly, the present invention provides methods of identifying a compound for the treatment of a metabolic disease or disorder, comprising administering a modulator of a GPCR to an animal having one or both SREB2, gene alleles inactivated. In certain embodiments, such screens are performed in animals having double knockouts of various SREB genes.

### 1. GPCR Knock-out and Knock-in Animals

In one embodiment, the present invention provides an animal model of metabolic disease, wherein said animal model is an animal that has one or both alleles of one or more SREB genes inactivated. An animal, such as a mouse, that has had one or both alleles of one or more SREB genes inactivated (*e.g.,* by homologous recombination or by insertional mutagenesis) is a preferred animal model for testing for compounds that alleviate symptoms of neurological or metabolic diseases and disorders associated with loss of one or more SREBs. In related embodiments, the present invention includes animal models of neurological disease, wherein said animal model in an animal that has one or both alleles of GPR88 or GPR22 inactivated.

In one embodiment, mice having mutations in a gene encoding a GPCR are made using homologous recombination. Suitable methods and reagents are described, for example, in U.S. Pat. Nos. 5,464,764, 5,487,992, 5,612,205, 5,627,059, 5,789,215, 6,204,061, and 6,228,639. In other embodiments, such mice are produced using the methods described in U.S. Patent Application Publication Nos. US 2005/0059078, US 2002/0115210, US 2002/0083481, US 2002/0012996, and US 2001/0056581.

In some instances, it may be desirable to "knock in" a polynucleotide encoding a human GPCR polypeptide of the invention to replace the polynucleotide encoding the orthologous mouse GPCR polypeptide. This can be accomplished using many of the methods described for the production of knock-out mice. The knocked-in polynucleotide may be expressed under the control of the endogenous mouse regulatory sequence, or may have exogenous regulatory sequences.

Knock-out and knock-in mice may be produced according to methods well known in the art (*see, e.g.,* Manipulating the Mouse Embryo. A Laboratory Manual, 2nd ed. B. Hogan, R. Beddington, F. Constantini, E. Lacy, Cold Spring Harbor Laboratory Press, 1984). In certain embodiments, embryonic stem (ES) cells containing a disrupted one or more SREB gene(s) are injected into mice blastocysts. These blastocysts are then transferred into uteri of pseudopregnant female mice. Pups born are scored for fur color, and chimeric mice (black and agouti color) with high contribution of agouti fur (50% or more) are tested for germ line transmission by breeding with C57B6/J mice. Presence of agouti progeny indicates germ line transmission, and the same chimera mice are then bred to generate knock-out mice on an inbred background. Alternatively, the chimeric mice are bred directly to 129 mice, and germ line transmission determined by PCR, Southern blotting, or other methods known in the art. The resulting heterozygous mice are then bred to generate knock-out mice on an inbred background.

The methods used to generate embryonic stem cells containing one or more disrupted SREB genes are known in the art. For example, a library of ES cells with random gene disruptions and the screening and isolation of ES clones containing a SREB disruption may be carried out essentially as described in U.S. Pat. No. 6,228,639. In brief, to generate a library of ES cells with random gene disruptions, ES cells are transfected with a retroviral vector. The vector is designed to inactivate genes in which it gets inserted. The ES cell insertional library is organized in a 3-D matrix of tubes. One copy of the library is stored as viable cells, and the other copy is used to isolate DNA. DNA from the library pools is screened by PCR for the insertions in the genes of interest. The same insertion found by PCR in pools corresponding to the other dimensions of the library matrix determines the 3-D address of the ES clone containing the disrupted gene.

Other methods are known in the art to generate gene disruptions in animals, including homologous recombination, chemical, radiation, and other mutational methods (Shastry, Mol. Cell Biochem. 181:163-179,1998; Shastry, Experientia 51:1028-1039, 1995; Zheng et al., Nucleic Acids Res. 27:2354-2360, 1999; Hokkaido Igaku Zasshi 77:151-156, 2002; Babinet et al., Ann. Acad. Bras. Cienc. 73:577-580, 2001; Williams, J. Appl. Physiol. 88:1119-1126, 2000). Any of these methods may be used to produce GPCR knock-out or knock-in animals, *e.g.,* a mouse, including double knockout animals, using methods described therein and polynucleotide sequences of the GPCR genes, such as those set forth in SEQ ID NOs:2, 4, 6, 8, 10, 12, 14, 16, 18, or 20.

### 2. Assays of SREB Modulator Activity in Animals

The effect of modulators of GPCR s can be assayed in animals using any of a wide variety of measurements or tests; Barbee et al., Am. J. Physiol. 263:R728-733, 1992; Berul et al., Circulation 94:2641-2648, 1996; Butz et al., Physiol. Genomics 5:89-97, 2001; Coatney, liar J. 42:233-247, 2001; Crawley et al., Horm. Behav. 31:197-211, 1997; Crawley et al., Psychopharmacol. (Berl) 132:107-124, 1997; Crawley et al. (eds.) Current Protocols in Neuroscience (John Wiley and Sons, 2001); Furukawa et al., Lab. Anim. Sci. 48:357-363, 1998; Hartley et al., Ilar J. 43:147-158, 2002; Krege et al., Hypertension 25:1111-1115, 1995; Kurien et al., Lab. Anim. 33:83-86, 1999; Lorenz et al., Am. J. Physiol. 272:H1137-H1146, 1997; Mattson, Am. J. Physiol. 274:R564-R570, 1998; Mitchell et al., Am. J. Physiol. 274:H747-H751, 1998; Pollick et al., J. Am. Soc. Echocardiogr. 8:602-610, 1995; Rogers et al., Mamm. Genome 8:711-713, 1997; Rogers et al., Neurosci. Lett. 306:89-92, 2001; Shih et al., Nat. Med. 6:711-714, 2000; Wiesmann et al., Magma 6:186-188, 1998; Irwin, Psychopharmacologia 13:222-257, 1968; Brayton etal., Vet. Pathol. 38:1-19, 2001; Ward etal., Pathology of Genetically Engineered Mice (Iowa State University Press, Ames, Iowa, 2000).

General physiological tests and measurements include, for example, measurement of body temperature, body length and proportions, body mass index, general health appearance, vocalization during handling, lacrimation and salivation, visual tests (*e.g.,* visual cliff, reaching response, visual menace), auditory tests (*e.g.,* click test, acoustic startle, acoustic threshold), olfactory tests (*e.g.,* sniffing and habituation to a novel odor, finding buried food), reflex tests (*e.g.,* righting reflex, eye blink, whisker twitch), measurement of metabolic hormones (*e.g.,* leptin, IGF-1, insulin, metabolites), whole body densitometry by dual energy x-ray absorptometry DEXA or high resolution radiography (Faxitron), and necropsy examination of organ systems.

Behavioral tests may be used to determine the behavioral phenotype of animals (*e.g.,* mice in which one or more GPCR genes of the present invention has been deleted or otherwise modified, and mice overexpressing one or more GPCR polypeptides of the present invention). Suitable tests include, but are not limited to, those that measure behaviors related to anxiety, hyperactivity, hypoactivity, appetite, eating habits, attention, drug abuse, drug addiction, learning and memory, mood, depression, schizophrenia, pain, sleep, arousal, sexuality, and social dominance.

The functional observational battery (FOB) is a series of tests applied to an animal to determine gross sensory and motor deficits. In general, short-duration, non-harmful tactile, olfactory, and visual stimuli are applied to the animal to determine its ability to detect and respond normally to the stimuli. The FOB also provides an opportunity for an investigator to closely observe each animal for skeletal and spontaneous neurological deficits (Crawley et al., Hormones Behav. 31:197-211, 1997).

General observational tests include, for example, swim tests, the auditory click test, measurement of body temperature or body weight, the Irwin Observational Test Battery, the olfactory acuity test, and the visual cliff test.

One means for measuring animal activity is the home cage activity test. Infrared photobeams provide information of when an animal is moving in its home cage. Animals in their home cages are placed in the photobeam boxes, and data are generated that provide insight into the animal's circadian rhythms activity, as well as general traits of activity (*e.g.,* hypoactivity or hyperactivity) during the testing period.

Another test assays open field activity. Locomotor activity is detected by photobeam breaks as the animal crosses each beam. Measurements used to assess locomotor activity include, for example, total distance traveled, total number of rearing events (animal raises up on hindlimbs), and distance traveled in the center compared to total distance traveled (center: total distance ratio). Typically, mice are placed in the center of the field. Mice will normally explore the edges/walls first and then, over time, spend more time in the center as they become familiar with the environment. Open field activity determination provides data on the general activity level of mice (*i.e.*, hypo- or hyper-active), as well as an indication of the animal's anxiety-related behaviors in an open-space.

Other means for measuring animal activity include measurement of circadian activity, elctroencephalography, electromyography, locomotor activity, novel object exploration, sleep deprivation and sleep rebound after deprivation, susceptibility to acute administration of phannacological agents in activity and sleep-related tests, susceptibility to chronic administration of pharmacological agents in activity and sleep-related tests, and wheel running activity.

The study of sleep is carried out with the use of the electroencephalograph (EEG) and/or electromyography (EMG). Stereotaxic placement of electrodes onto the cortex for EEG recording and bilateral placement of electrodes into the trapezius muscle in the neck (EMG) allow the different stages of wake and sleep to be analyzed. Animals that display disrupted or altered sleep pattern may serve as models for screening for drugs that treat sleep disorders such as dysomnias and parasomnias.

Tests for determining whether a mouse has altered coordination or movement include the Balance Beam test, Bilateral Tactile Stimulation test, Circling Behavior test, Disengage test, Grip Strength test, Holeboard test, Paw Reaching test, Parallel Bar Walking test, Ring Catalepsy test, Rotorod test, Sterotypy Behavior test, or Vertical Pole test. Coordination and movement can also be assessed by assessment of exercise capacity, footprint pattern, forelimb asymmetry, posture, and gait.

In one example, motor coordination and skill learning is assessed using the rotarod assay, which measures the ability of an animal to maintain balance on an accelerating rotating rod. The mice must walk continuously to avoid falling off (*see* Crawley et al., Hormones Behav. 31:197-211, 1997). Animals are generally given multiple trials spaced at least 20 minutes apart to allow for recovery from any fatigue testing may cause. In general, the time the animal spends walking on top of the rotating rod increases over the trials, thus demonstrating motor coordination and the ability to learn a rudimentary skill. This test relates to coordination and balance deficiencies.

Feeding and ingestive behaviors can be examined, for example, by monitoring 24 hour food consumption, 24 hour water consumption, body weight during development, circadian feeding patterns, conditioned taste aversion, conditioned taste preference, fasting studies (*e.g.,* weight loss during fasting, weight gain after fasting, feeding response after fasting), liquid intake, macronutrient choice, novel food preference, rebound food consumption response after restricted daily access to food, response to specialized diets (*e.g.,* cafeteria diet, high or low protein diet, high or low fat diet, and high or low carbohydrate diet), susceptibility to acute administration of pharmacological agents in feeding paradigms, and susceptibility to chronic administration of pharmacological agents in feeding paradigms. Food consumption over consecutive days may be determined, *e.g.,* during the monitoring of home cage activity. The amount of consumed food and the body weight of the mouse are determined at various timepoints. If desired, the frequency and duration of eating may also be determined. This assay provides insight into the appetite and eating habits that might relate to eating conditions or disorders.

Sexual responsiveness can be tested, *e.g.,* in a clear chamber with video recording. Male mice are tested to determine if they respond normally to a female mouse. Measurements used to assess normal male responsiveness include, but not limited to, mount latency, mount frequency, pelvic thrusts, intromissions, and ejaculation. Female mice are also tested to determine their sexual receptivity to a male. Measurements used to assess normal female receptivity involve assessing the degree and frequency of lordosis behavior. Sexual behaviors can also be measured by examining sexual motivation, ethologically relevant behaviors (*e.g.,* anogenital investigation) as part of normal social interactions, susceptibility to acute administration of pharmacological agents in sexual responsiveness assays, and susceptibility to chronic administration of pharmacological agents in sexual responsiveness assays. These assays can be used to determine sexual activity in general and to detect any abnormal sexual behavior that might relate to sexual conditions or disorders.

Nociceptive behaviors can be assessed using a test that measures, for example, allodynia as a model for chronic pain, inflammatory pain, pain threshold, sensitivity to drug-induced analgesia, thermal pain, mechanical pain, chemical pain, hyperalgesia, or shock sensitivity. Particular tests include the allodynia/place avoidance, calibrated von Frey hairs for mechanical pain, cold plate test, cold water tail immersion test, conditioned suppression, formalin paw assay, Hargreaves test, hot plate test, hot water tail immersion test, paw pressure test, paw withdrawal, plantar test, tail flick test, tail pressure test, and the writhing test, susceptibility to actue administration of pharmacological agents in nociception tests, and susceptibility to chronic administration of pharmacological agents in nociception tests. In one example, a mouse's nociception is assessed by placing the mouse on a 55° C hot plate. The latency to a hind limb response (shake or lick) is measured. This assay provides data on the animal's general analgesic response to a thermal stimulus, and is used to detect a nociceptive condition or disorder. The formalin paw assay measures the response to a noxious chemical injected into the hindpaw. Licking and biting of the hindpaw is quantitated as the amount of time engaged in these activities. Two phases of responses are demonstrated with the first phase representing an acute pain response and the second phase representing a hyperalgesic response. Alterations in this normal biphasic display may serve as a model of various forms of pain and chronic pain disorders (Abbott et al., Pain 60:91-102,1995).

Tests that measure or detect anxiety-related behaviors include acoustic startle habituation, acoustic startle reactivity, active avoidance, the canopy test, conditioned emotional response, conditioned suppression of drinking, conditioned ultrasonic vocalization, dark light emergence task, defensive burying, dPAG-induced flight, elevated plus maze, elevated zero maze, exploration tests in a novel environment, fear-potentiated startle, food exploration test, four plate test, Gellar-Seifter conflict test, light-dark box, light-enhanced startle, marble burying test, mirror chamber, novelty supressed feeding, pain-induced ultrasonic vocalizations, petition test, passive avoidance, probe burying test, punished locomotion test, separation-induced ultrasonic vocalizations, shock sensitization of startle response, social competition, social interation, staircase test, susceptibility to acute administration of pharmacological agents in anxiety-related assays, and susceptibility to chronic administration of pharmacological agents in anxiety-related assays. One such test is the light-dark exploration test, which measures the conflict between the natural tendencies of mice to explore novel environments but to avoid the aversive properties of brightly lit (anxiety-provoking) open areas. In this test, the brightly lit compartment encompasses about two-thirds of the surface area, while the dark compartment encompasses the remaining one-third of the area. An opening is designed to allow the mouse access to both compartments. The mouse is placed at the one end of the brightly lit compartment. The latency to enter the dark compartment, total time spent in the dark compartment, and the number of transitions between the two compartments is measured to give a sense of an anxiety-related response that might be related to an anxiety condition or disorder.

Tests for identifying stress-related behaviors include electric footshock stress tests, handling stress test, maternal separation stress test, restraint induced stress test, sleep deprivation stress test, social isolation stress test, swim stress test, stress-induced hyperthermia, and susceptibility to acute or chronic administration of pharmacological agents in stress-related tasks. These assays provide the ability to study stress and to provide insight into behaviors that may be related to stress conditions or disorders.

Tests for identifying fear-related behaviors in rodents include conditioned fear, fear potentiated startle, fear-response behavior, mouse defense test battery, ultrasonic vocalization test, and susceptibility to acute or chronic administration of pharmacological agnets in fear-related tests. These assays provide the ability to study emotional based behaviors that may be related to fear-based conditions or disorders.

Depression-related tests include acute restraint, chronic restraint, circadian activity, conditioned defensive burying, differential reinforcement to low rate of responding, learned helplessness, Porsolt forced swim test, tail suspension test, sucrose preference test, and susceptibility to acute or chronic administration of pharmacological agents in depression-related tests. Another is the tail suspension test, which includes suspending a mouse by its tail and measuring the duration of time it continues to struggle to escape from an inescapable situation. The time spent struggling is considered a measure of learned helplessness behavior or behavioral despair. The latency to the onset of the end of the struggling can be increased by clinically effective antidepressants. This assay therefore can be used to identify mice that may serve as models for depressive disorders.

Mood related behavioral assays include latent inhibition, marble burying, prepulse inhibition of the acoustic startle response, and susceptibility to acute and chronic administration in mood-related tests. Prepulse inhibition of the acoustic startle response occurs when a loud (120 dB) startle stimulus is preceded by a softer tone that does not elicit a startle response (the prepulse). It is believed that this is a measure of a filtering mechanism in the nervous system that allows an individual to focus on important incoming information and to ignore unimportant information. Schizophrenic patients have been documented to have impaired prepulse inhibition; therefore this test can be used employing mice to identify those having a response that may be indicative of schizophrenia or another psychotic disorder.

Suitable tests for assessing a mouse's learning and memory capacity include, for example, those that measure active avoidance, autoshaping, Barnes maze, conditioned taste aversion, conditional spatial alternation, context and auditory cued conditioned fear, contextual discrimination, delayed matching to position, delayed matching/non-matching to position, eyeblink conditioning, fear potentiated startle, FIG. 8 maze, holeboard test, motor leamingusing an accelerated rotarod, place aversion test, novel object recognition, olfactory discrimination, passive-avoidance, position/response learning, schedule-induced operant behaviors, radial arm maze, social recognition, social transmission of food preference, step down avoidance, taste learning, temporal processing using the Peak procedure, trace conditioning, T maze avoidance, transverse patterning, visual discrimination, wate maze place memory test, vigilence test, Y maze, and Y maze avoidance.

The Morris water maze test is an assay that measures spatial learning and memory. An animal is trained in a pool of opaque water to locate a platform hidden under the water's surface using spatial cues external cues in the room. Measurements of spatial learning require analysis of spatial selectivity on a probe trial, in which the platform has been removed and the pattern in which the animal searches is examined. An animal that has learned the position of the platform using spatial cues will spend more time in the quadrant where the platform was located, and will also cross the precise location of the platform more often versus other possible sites. This complex learning task provides a way to determine learning and memory deficits and enhancements, and offers insight into the neural mechanisms of learning and memory (Crawley et al., Psychopharmacol. 132:107-124, 1997).

Context and auditory cue fear conditioning (*i.e*., conditioned fear) is determined by placing a mouse in an enclosed chamber in which the floor is equipped to deliver a mild electrical shock to the mouse's feet. The training day consists of placing the mouse in the chamber and allowing it to explore the environment. At the end of the exploration period, a white noise is turned on (i.e., the conditioning stimulus, CS). A footshock is paired with the white noise turning off. This training trial is then repeated again. At the end of the second trial, the mouse is returned to its home cage. The mouse is tested 24 hours later by separately assaying the amount of freezing exhibited in the context in which it was shocked (Context Test) and the amount of freezing exhibited to the white noise (CS Test). As the mouse conditions to the pairing of the tone and shock, it may exhibit a freezing behavior due to the fear that the mild foot shock imparts to the mouse. Freezing behavior on the test day suggests that the mouse has learned that it received a shock in this particular context when the white noise is turned off. This test is considered to provide data about emotional-based learning and memory.

Aggression and other social behaviors can be monitored by observation or quantification of behaviors such as grooming, home cage behaviors (e.g., nesting, huddling, playing, and barbering) isolation-induced fighting, maternal behavior, parental behavior, social interaction, social investigation. Particular tests include the Partition test, the social defeat test, the Resident versus Intruder test, and the Social Place Preference test. Any of the foregoing can be used to determine a mouse's susceptibility to acute or chronic administration of pharmacological agents. The resident-intruder paradigm is an assay that demonstrates species-specific aggressive behavior. This test is conducted by individually housing an animal (the resident) and introducing a new animal of the same gender (the intruder) into the cage. The new animal is viewed by the resident animal as an intruder and displays aggressive behaviors toward the intruder (Crusio, Behav. Genet. 26:459-533, 1996). The normal display of aggression towards an intruder may serve as a model for examining increased or decreased aggression to a normal environmental situation.

One test for social dominance can be carried out to assay social interactions and social behaviors. In the so-called "tube test," a mouse is placed into the end of a plexiglass cylinder and another mouse (called a social cohort) is placed at the other end of the tube. The animal that backs out of the tube first is considered the loser and the mouse that remains in the tube is considered the winner. In general, an animal that backs out of the tube during the first round generally backs out of the tube in subsequent rounds. A ranking can then be given to each animal, thus identifying the dominance or submissive status of an animal within a social context, as well as detecting abnormal social behaviors that can be related to antisocial personality conditions or disorders.

Behaviors relating to reward and addiction are assessed using tests that measure, for example, reward and place preference, self-administration of drugs of abuse (acute and chronic), sensitization and tolerance to drugs of abuse, sensitization to the motor activating properties of drugs, tolerance to repeated analgesic drug administration, or withdrawl symptoms after repeated self-administration of drugs of abuse. The impact on self-administration of drugs of abuse in stress tests can also be used to assess addiction.

Tolerance and sensitivity to ethanol and cocaine can be tested, for example, by examining core body temperature of the mice after an intraperitoneal (i.p.) injection of cocaine or ethanol. Initial sensitivity to cocaine and alcohol can be measured in mice after a single (acute) dose. In rodents, repeated exposure to alcohol or cocaine via repeated injections across days has been shown to produce tolerance. In both the alcohol studies and the cocaine studies, mice are administered an i.p. dose, and core body temperature is measured post injection with a digital thermometer with a rectal probe. On Day 2, mice are administered the same dose using the same route, and temperature again recorded post injection. Tolerance to the drug is indicated by an increase in body temperature on the second day of drug administration compared to the first day of drug administration. These assays detect sensitivity to various drug substances and, thus, are indicators of alcohol or cocaine use disorders.

The rewarding effects of various substances of abuse can be studied using the conditioned place preference paradigm and self-administration tests. The place preference paradigm is a non-invasive method that is amenable to classical Pavlovian conditioining. The rewarding drug serves as an unconditioned stimulus (US) that is paired with an environment that serves as the conditioned stimulus (CS). Given a choice between exploring a novel environment and the drug-paired CS environment, the animals prefer the drug-paired CS environment, thereby demonstrating conditioned place preference (Itzhak and Martin, Neuropsychopharmacol. 26:130-134, 2002). This Pavlovian conditioned response to a drug of abuse has been postulated to be involved in drug-seeking behavior and relapse following exposure to cues that were previously associated with drug use. Self-administration studies, in general, allow the animal to regulate the administration of a drug to its nervous system. With these types of studies, extinction and reinstatement of drug intake behaviors can be examined and may serve as a model for drug-seeking behavior and relapse in humans (Stewart et al., Brain Res. 457:287-294, 1988).

Administration of a drug such as bicuculine can be utilized to study an animal's susceptibility to seizures or seizure-like events. Mice that enter into classical seizure symptoms earliest are considered to be more susceptible to seizures. Likewise, mice that present seizure symptoms later than normal, are considered to be more resistant to seizures. This assay may allow the identification of alterations central to the formation of seizure disorders and related conditions.

Methods for performing many of the foregoing screens are well known in the art (*see, e.g.,* Brunner et al., J. Exp. Psychol. Anim. Behav. Process 20:331-346, 1994; Crawley, What's Wrong With My Mouse? (John Wiley and Sons, Somerset, N.J., 2000). Crawley et al., (eds.); Current Protocols in Neuroscience (John Wiley and Sons, Somerset, N.J., 2001); Crawley et al., Hormones Behav. 31:197-211, 1997; Crawley et al., Psychopharmacol. (Berl) 132:107-124, 1997; Galey et al., Neurosci. Lett. 143:87-90, 1992; Hascoet et al., Pharmacol. Biochem. Behav. 65:339-344, 2000; Martinez-Mota et al., Psychoneuroendocrinol. 25:109-120, 2000; Mogil et al., Pain 80: 67-82, 1999; Toubas et al., Pharmacol. Biochem. Behav. 35:121-126, 1990; Van Der Hyden et al., Physiol. Behav. 62:463-470, 1997, Walker et al., Molec. Med. Today 5:319-321, 1999).

In addition to determining the physiological effects of compounds that modulate GPCR expression or activity, the animals having one or more mutant GPCR genes are useful for further testing of efficacy and safety of such compounds. In addition, cells or tissues isolated from the animal and placed in culture may also be used to determine the effects of compounds that modulate the expression or activity of GPCRs. Compounds that modulate the expression or activity of one or more GPCRs and are useful in the treatment of a neurological or metabolic condition are expected to alleviate one or more symptoms of a neurological or metabolic disease or disorder in an animal.

### F. Methods of Diagnosing Neurological and Metabolic Diseases and Disorders

Further disclosed herein are methods of diagnosing or detecting a risk for developing a neurological or metabolic disease or disorder in a patient. Expression, biological activity, and mutational analysis of SREB polypeptides and polynucleotides can each serve as a diagnostic tool for neurological or metabolic diseases and disorders. Expression, biological activity, and mutational analysis of GPR88 and GPR22 can each serve asa diagnostic tool for neurological diseases and disorders. In addition, determination of the genetic subtyping of one or more GPCR gene sequences can be used to subtype individuals or families to determine their predisposition for developing a particular disease or disorder.

In certain embodiments related to both neurological and metabolic diseases and disorders, these methods comprise measuring a level of activity or expression of one or more SREB polynucleotides or polypeptides, e.g., SREB1, SREB2, and/or SREB3, in a biological sample obtained from a patient, and then comparing the level measured to control values or to levels measured in a control subject that does not have the neurological or metabolic disease or disorder. If the level in the patient is significantly higher or lower than the control level, then the patient may be considered at risk of having the neurological or metabolic disease or disorder. In certain embodiments, the patient is considered to have the neurological or metabolic disorder if the level in the patient is at least two-fold, three-fold, or five-fold different than the level in the control. In one embodiment, a patient is diagnosed with a neurological or metabolic disease or disorder if the level of expression or activity of one or more SREBs is significantly lower than a control value. In certain embodiments, the level of one or more SREBs' expression or activity in the patient is less than 75%, les than 50%, less than 25%, or less than 10% the level measured in the control.

In certain embodiments related to neurological diseases and disorders, these methods comprise measuring a level of activity or expression of GPR88 and/or GPR22 in a biological sample obtained from a patient, and then comparing the level measured to control values or to levels measured in a control subject that does not have the neurological or metabolic disease or disorder. If the level in the patient is significantly higher or lower than the control level, then the patient may be considered at risk of having the neurological disease or disorder. In certain embodiments, the patient is considered to have the neurological disease or disorder if the level in the patient is at least two-fold, three-fold, or five-fold different than the level in the control. In one embodiment, a patient is diagnosed with a neurological disease or disorder if the level of expression or activity of one or more of GPR88 and/or GPR22 is significantly lower than a control value. In certain embodiments, the level of one or more of GPR88 and/or GPR22 expression or activity in the patient is less than 75%, les than 50%, less than 25%, or less than 10% the level measured in the control.

An exemplary method for detecting the presence or absence of a GPCR protein or nucleic acid in a biological sample involves obtaining a biological sample from a test subject and contacting the biological sample with a compound or an agent capable of detecting a GPCR protein or nucleic acid (e.g., mRNA, genomic DNA) that encodes a GPCR protein such that the presence of the GPCR protein or nucleic acid is detected in the biological sample. A preferred agent for detecting GPCR mRNA or genomic DNA is a labeled nucleic acid probe capable of hybridizing to a GPCR mRNA or genomic DNA.

The nucleic acid probe can be, for example, a full-length GPCR nucleic acid, such as the nucleic acid of any of SEQ ID NOs:2, 4, 6, 14, or 18, or a portion thereof, such as an oligonucleotide of at least 15, 30, 50, 100, 250 or 500 nucleotides in length and sufficient to specifically hybridize under stringent conditions to a GPCR mRNA or genomic DNA. Other suitable probes for use in the diagnostic assays of the invention are described herein.

Another method for detecting the presence or absence of a GPCR protein in a biological sample involves obtaining a biological sample from a test subject and contacting the biological sample with an antibody that is capable of detecting a GPCR protein, where said antibody capable of binding to the GPCR protein preferably has a detectable label. Antibodies can be polyclonal, or more preferably, monoclonal. An intact antibody, or a fragment thereof (*e.g.,* Fab or F(ab')2) can be used.

The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

The term "biological sample" is intended to include tissues, cells and biological fluids isolated from a subject, as well as tissues, cells and fluids present within a subject. That is, GPCR mRNA, protein, or genomic DNA in a biological sample *in vitro* as well as *in vivo.* For example, *in vitro* techniques for detection of GPCR mRNA include Northern hybridizations and in situ hybridizations. *In vitro* techniques for detection of GPCR protein include enzyme linked immunosorbent assays (ELISAs), Western blots, immunoprecipitations and immunofluorescence. In vitro techniques for detection of GPCR genomic DNA include Southern hybridizations. Furthermore, *in vivo* techniques for detection of a GPCR protein include introducing into a subject a labeled anti-GPCR antibody specific for the GPCR of interest. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a serum sample isolated by conventional means from a subject.

In another embodiment, the methods further involve obtaining a control biological sample from a control subject, contacting the control sample with a compound or agent capable of detecting a GPCR protein, mRNA, or genomic DNA, such that the presence of the GPCR protein, mRNA or genomic DNA is detected in the biological sample, and comparing the presence of the GPCR protein, mRNA or genomic DNA in the control sample with the presence of the GPCR protein, mRNA or genomic DNA in the test sample.

Also disclosed herein are kits for detecting the presence of a GPCR in a biological sample. For example, the kit can comprise a compound or agent capable of detecting a particular GPCR protein or mRNA in a biological sample; means for determining the amount of the GPCR in the sample; and means for comparing the amount of the GPCR in the sample with a standard. The kit may include two or more compounds capable of detecting a particular GPCR protein or mRNA in a biological sample. In certain embodiments, the two or more compounds detect the same GPCR, while in other embodiments, they detect different GPCRs, e.g., SREBs. The compounds may be labeled. The compound or agent can be packaged in a suitable container. The kit can further comprise instructions for using the kit to detect the GPCR protein or nucleic acid.

The diagnostic methods described herein can furthermore be utilized to identify subjects having or at risk of developing a disease or disorder associated with aberrant GPCR expression or activity. For example, the assays described herein, such as the preceding diagnostic assays or the following assays, can be utilized to identify a subject having or at risk of developing a disorder associated with a misregulation in GPCR protein activity or nucleic acid expression, such as a neurological or metabolic disorder. Alternatively, the prognostic assays can be utilized to identify a subject having or at risk for developing a disorder associated with a misregulation in GPCR protein activity or nucleic acid expression, such as a neurological or metabolic disorder.

Thus, the present invention provides a method for identifying a disease or disorder associated with aberrant GPCR expression or activity in which a test sample is obtained from a subject and GPCR protein or nucleic acid (*e.g.,* mRNA or genomic DNA) is detected, wherein the presence or absence of a GPCR protein or nucleic acid is diagnostic for a subject having or at risk of developing a disease or disorder associated with aberrant GPCR expression or activity. As used herein, a "test sample" refers to a biological sample obtained from a subject of interest. For example, a test sample can be a biological fluid (*e.g.,* serum), cell sample, or tissue.

Furthermore, the prognostic assays described herein can be used to determine whether a subject can be administered an agent (*e.g.,* an agonist, antagonist, peptidomimetic, protein, peptide, nucleic acid, small molecule, or other drug candidate) to treat a disease or disorder associated with aberrant GPCR expression or activity. For example, such methods can be used to determine whether a subject can be effectively treated with an agent for a weight, cardiovascular, neural or endocrine disorder. Thus, the present invention provides methods for determining whether a subject can be effectively treated with an agent for a disorder associated with aberrant GPCR expression or activity in which a test sample is obtained and GPCR protein or nucleic acid expression or activity is detected (*e.g.,* wherein the abundance of the GPCR protein or nucleic acid expression or activity is diagnostic for a subject that can be administered the agent to treat a disorder associated with aberrant GPCR expression or activity).

Further disclosed herein are methods of diagnosing a neurological or metabolic disease or disorder in a patient based upon detecting a mutation in one or more of a patient's GPCR genes, including, e.g., one or more SREB genes. Similarly, in particular embodiments, the method is used to diagnosis a neurological disease or disorder based upon detecting a mutation in one or more of a patients GPR88 or GPR22 genes. A gene encoding a GPCR polypeptide may have a polymorphism that may be, for example, a causative or risk factor of neurological or metabolic disease. Screening methods that identify polymorphisms may be of diagnostic and therapeutic benefit. For example, early detection of a particular polymorphism may enable preventative treatment or prediction of a patient's response (*e.g.,* increased or decreased efficacy or undesirable side effects of treatment). Methods of identifying polymorphisms are well-known in the art and include, *e.g.,* PCR, RT-PCR, northern blot (*e.g.,* using clones encompassing discrete regions of cDNA), Southern blot, polymorphic specific probes, sequencing analysis, hybridization assays, restriction endonuclease analysis, and exon-specific amplification.

Accordingly, methods of the invention can be used to detect genetic alterations in one or more GPCR genes, thereby determining if a subject with the altered gene is at risk for a disorder characterized by misregulation in GPCR protein activity or nucleic acid expression, such as a neurological or metabolic disease or disorder. In preferred embodiments, the methods include detecting, in a sample of cells from the subject, the presence or absence of a genetic alteration characterized by at least one of an alteration affecting the integrity of a gene encoding a GPCR protein, or the mis-expression of the GPCR gene. For example, such genetic alterations can be detected by ascertaining the existence of at least one of 1) a deletion of one or more nucleotides from a GPCR gene; 2) an addition of one or more nucleotides to a GPCR gene; 3) a substitution of one or more nucleotides of a GPCR gene, 4) a chromosomal rearrangement of a GPCR gene; 5) an alteration in the level of a messenger RNA transcript of a GPCR gene, 6) aberrant modification of a GPCR gene, such as of the methylation pattern of the genomic DNA, 7) the presence of a non-wild type splicing pattern of a messenger RNA transcript of a GPCR gene, 8) a non-wild type level of GPCR protein, 9) allelic loss of a GPCR gene, and 10) inappropriate post-translational modification of a GPCR protein. As described herein, there are a large number of assays known in the art that can be used for detecting alterations in GPCR genes. A preferred biological sample is a tissue or serum sample isolated by conventional means from a subject, *i.e.,* patient. In particular embodiments, these methods may be practiced specifically with respect to SREB1, SREB2, SREB3, GPR88 and/or GPR22.

In certain embodiments, detection of the alteration involves the use of a probe/primer in a polymerase chain reaction (PCR) such as anchor PCR or RACE PCR, or, alternatively, in a ligation chain reaction (LCR) (*see, e.g.,* Landegran et al. (1988) Science 241:1077-1080; and Nakazawa et al. (1994) Proc. Natl. Acad Sci. USA 91:360-364), the latter of which can be particularly useful for detecting point mutations in the GPCR-gene (see Abravaya et al. (1995) Nucleic Acids Res. 23:675-682). This method can include the steps of collecting a sample of cells from a subject, isolating nucleic acid (*e.g.,* genomic, mRNA or both) from the cells of the sample, contacting the nucleic acid sample with one or more primers which specifically hybridize to a GPCR gene under conditions such that hybridization and amplification of the GPCR gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J. C. et al., (1990) Proc. Natl. Acad Sci. USA 87:1874-1878), transcriptional amplification system (Kwoh, D. Y. et al., (1989) Proc. Nail. Acad Sci. USA 86:1173-1177), Q-Beta Replicase (Lizardi, P. M. et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a GPCR gene from a sample cell can be identified by alterations in restriction enzyme cleavage patterns. For example, sample and control DNA is isolated, amplified (optionally), digested with one or more restriction endonucleases, and fragment length sizes;are determined by gel electrophoresis and compared. Differences in fragment length sizes between sample and control DNA indicates mutations in the sample DNA. Moreover, the use of sequence specific ribozymes can be used to score for the presence of specific mutations by development or loss of a ribozyme cleavage site.

In other embodiments, genetic mutations in GPCR genes can be identified by hybridizing a sample and control nucleic acids, *e.g.,* DNA or RNA, to high density arrays containing hundreds or thousands of oligonucleotides probes (Cronin, M. T. et al. (1996) Human Mutation 7: 244-255; Kozal, M. J. et al. (1996) Nature Medicine 2: 753-759). For example, genetic mutations in GPCR genes can be identified in two dimensional arrays containing light-generated DNA probes as described in Cronin, M. T. et al. (1996) Human Mutation 7: 244-255

In yet another embodiment, any of a variety of sequencing reactions known in the art can be used to directly sequence a GPCR gene and detect mutations by comparing the sequence of the sample GPCR with the corresponding wild-type (control) sequence. Examples of sequencing reactions include those based on techniques developed by Maxam and Gilbert (1977) Proc. Nati. Acad. Sci. USA 74:560 or Sanger (1977) Proc. Nati. Acad. Sci. USA 74:5463. It is also contemplated that any of a variety of automated sequencing procedures can be utilized when performing the diagnostic assays ((1995) Biotechniques 19:448), including sequencing by mass spectrometry (Cohen et al. (1996) Adv. Chromatogr. 36:127-162; and Griffin et al. (1993) AppL. Biochem. Biotechnol. 38:147-159).

Other methods for detecting mutations in the GPCR gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes of formed by hybridizing (labeled) RNA or DNA containing the wild-type GPCR sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with SI nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. For examples see, Cotton et al. (1988) Proc. Natl Acad Sci USA 85:4397; and Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in a SREB cDNA obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on a wild-type SREB sequence, is hybridized to a cDNA or other DNA product from a test cell(s). The duplex is treated with a DNA mismatch repair enzyme, and the cleavage products, if any, can be detected from electrophoresis protocols or the like.

In other embodiments, alterations in electrophoretic mobility will be used to identify mutations in GPCR genes. For example, single strand conformation polymorphism (SSCP) may be used to detect differences in electrophoretic mobility between mutant and wild type nucleic acids (Orita et al. (1989) Proc Nati. Acad. Sci USA: 86:2766, see also Cotton (1993) Mutat. Res. 285:125-144; and Hayashi (1992) Genet. Anal. Tech. AppL. 9:73-79). Single-stranded DNA fragments of sample and control LGR6 nucleic acids will be denatured and allowed to renature. The secondary structure of single-stranded nucleic acids varies according to sequence, the resulting alteration in electrophoretic mobility enables the detection of even a single base change.

The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) Proc. Natl Acad Sci USA 86:6230). Such allele specific oligonucleotides are hybridized to PCR amplified target DNA or a number of different mutations when the oligonucleotides are attached to the hybridizing membrane and hybridized with labeled target DNA. Alternatively, allele specific amplification technology which depends on selective PCR amplification may be used in conjunction with the instant invention.

The methods described herein may be performed, for example, by utilizing pre-packaged diagnostic kits comprising at least one probe nucleic acid or antibody reagent described herein, which may be conveniently used, e.g., in clinical settings to diagnose patients exhibiting symptoms or family history of a disease or illness involving one or more GPCR genes, including, in particular embodiments, SREB1, SREB2, SREB3, GPR88, and GPR22.

### EXAMPLES

### EXAMPLE 1

### GPR85/SREB3 DKO MICE ARE HYPERACTIVE IN OPEN FIELD

To further examine the role of SREBs in neurological and metabolic diseases and disorders, GPR85 and SREB3 knock-out mice were generated according to the methods described in U.S. Patent No. 6,228,639. GPR85/SREB3 double knock-out (DKO) mice were generated by mating GPR85 knock-out (KO) mice to SREB3 knock-out mice.

Open field activity of GPR85/SREB3 double knock-out mice was examined to determine if loss of GPR85 and SREB3 was associated with neurological defects. This assay provides data on the general activity level of mice (*i.e.,* hypo- or hyper-active), and indication of anxiety towards the aversive properties of the novel, open environment.

Open field activity was monitored in VersaMax chambers (Accuscan Instruments, Columbus, OH) measuring 40 x 40 cm. Locomotor activity was detected by photobeams breaks as the animal crossed each beam. The animal was placed in the center of the field and then left undisturbed for a period of time (20 min to 2 hr) in order to measure its spontaneous activity in a novel environment. Measurements used to assess locomotor activity included: horizontal activity, total distance traveled, vertical activity (rearing events - animal raises up on hindlimbs), rotation, stereotypy, and distance traveled in the center compared to total distance traveled (center: total distance ratio).

As shown in Figure 1, GPR85/SREB3 DKO mice were hyperactive as assessed in the open field activity test. Compared to wild type (WT) mice, the DKO mice exhibited increased locomotor activity as measured by the total distance traveled in the chamber (p<0.05, repeated measures ANOVA, n = 12-14 per group), whereas the SREB3 and GPR85 single KOs did not behave differently from WT.

### EXAMPLE 2

### GPR85/SREB3 DKO MICE HAVE REDUCED PREPULSE INHIBITION (PPI)

Prepulse inhibition (PPI) of the acoustic startle response evaluates the brain's sensorimotor gating function that is often disrupted in schizophrenic and other psychotic conditions. Psychostimulants such as PCP and amphetamine reduce or disrupt PPI, and many antipsychotics increase PPI and/or reverse psychostimulant-induced reduction of PPI. In addition, the startle response to the loud noise itself (in the absense of any prepulse) is a measure of the basic sensorimotor reflex.

The PPI of GPR85/SREB3 DKO mice was determined using the SR-Lab System (San Diego Instruments, San Diego, CA). A test session consisted of six trial types under the background noise of 70 dB. One type used a 40 msec, 120 dB noise as the startle stimulus. Four types contained acoustic startle stimulus preceded by acoustic prepulses of different intensity: the 20-msec prepulse noise of 73, 76, 79, or 82 dB was presented 100 msec before the 120 dB startle stimulus. The last trial type used the 70 dB background noise with no startle stimulus to measure baseline reaction. Six blocks of the six trial types were presented in pseudorandom order. The startle response was recorded for 65 ms starting with the onset of the startle stimulus. Measurements used to assess PPI were the maximum startle amplitude and the percent each of the 4 prepulses inhibited the startle response.

As shown in Figure 2, GPR85/SREB3 DKO mice had reduced prepulse inhibition (PPI). The startle responses to 120dB noise alone were not significantly altered in any of the KO strains (Figure 2A). There was significant reduction of PPI (Figure 2B) in DKO mice at all prepulse levels (**p<0.01, ***p<0.001, n = 19-22 per group, Student's t-test). There was also a partial reduction of PPI in GPR85 KO but not SREB3 KO.

Old GPR85 KO mice also had reduced prepulse inhibition (PPI, Figure 7). The same animals we tested at young (3 months, Figure 7A) and old (17 months, Figure 7B) ages. There was a significant reduction of PPI in old GPR85 KO mice (*p<0.05, # p=0.066, n = 6-9 per group, Student's t-test).

### EXAMPLE 3

### GPR85/SREB3 DKO MICE EXHIBIT BEHAVIORAL DISORDERS

Marble burying is a behavioral test that can demonstrate the anxiolytic-like effect of both known anxiolytics and antidepressants. It also models certain aspects of the obsessive compulsive disorder. This procedure was performed on GPR85/SREB3 DKO mice, as well as GPR85 KO, SREB3 KO and WT mice, as follows. Eighteen clean glass marbles of diameter 1.5 cm were evenly distributed on top of 5-cm deep cobb bedding in a large clean mouse cage. Each mouse was individually placed into the cage, and the cage was covered with a metal grid. The mouse was left in the cage for 30 min without food or water, and then transferred back into its home cage. The number of marbles at least two-thirds buried by the mouse was counted. Anxiolytic-like effect was reflected in the reduction of number of marbles buried.

As depicted in Figure 3, GPR85/SREB3 DKO mice are less anxious or have less compulsive behavior in the marble burying test. There was a significant reduction of number of marbles buried by DKO mice (***p<0.001, n = 19-22 per group, Student's t-test) but not GPR85 KO or SREB3 KO.

Nest building is considered a form of instinct social behavior. Mice of both sexes build a nest when provided with suitable material and are typically found lying in it during the daytime. In the wild, the nest provides shelter, camouflage from predators, conservation of body heat, and is an important component of fitness. Parents and offspring share the nest and both parents retrieve pups into the nest. Mutant mice related to human conditions such as schizophrenia and autism spectrum disorder have shown deficits in nest building behavior.

Nest building activity of GPR85/SREB3 DKO and control mice were assessed as follows. Mice were singly housed. A piece of nestlet, a 5cm x 5cm piece of pressed cotton, was introduced into the cage during early daytime. Afterwards, the quality of the nest built and the location of the mouse relative to the nest were recorded at 1, 2, 6 and 24 hours post insertion of nestlet. Nest quality was measured using the following scale: (0) nestlet unmodified; (1) nestlet minimally teared; (2) shallow nest with partially shredded nestlet; (3) nest relatively well developed; and (4) perfect nest with tall walls.

As shown in Figure 4, GPR85/SREB3 DKO mice were impaired in nest building. At all time points examined after a piece of nesting material was given, the DKO mice showed significant impairment in building the nest (**p<0.01, ***p<0.001, n = 7-10 per group, Mann-Whitney U test). GPR85 KO mice also showed significant impairment at 1h and 2h time points (*p<0.05, **p<0.01), while SREB3 KO mice had no significant impairment.

Old GPR85 KO mice were also impaired in nest building (Figure 6). When tested at 16-18 months of age, both male and female GPR85 KO mice showed significant impairment in building the nest at all time points examined (*p<0.05, **p<0.01, ***p<0.001, n = 6-9 per group, Mann-Whitney U test).

Resident-intruder test is a test of male social behavior and was used to test GPR85/SREB3 DKO mice and controls. A singly housed male test mouse (resident) was introduced with a stranger mouse (intruder) of wildtype genotype. Their behaviors were videotaped and observed for 12 min. Wildtype resident mice exhibited normal social/aggressive behavior, consisting of investing the intruder mouse by sniffing and other contacts followed by aggressive behavior towards the intruder such as biting and attacking.

As shown in Figure 5, GPR85/SREB3 DKO mice had reduced aggressive behavior in the resident-intruder test. Only WT and DKO, but not GPR85 KO or SREB3 KO, were tested. The total numbers of all social interactive behavior exhibited by the WT or DKO resident mice, including sniffing, tail chasing and attacking, did not differ significantly by genotype (left panel). There was a significant reduction of aggressive behavior, i.e., attacking the intruder mice, in the DKO mice (**p<0.01, n = 16DKO, 21WT, Student's t-test).

### EXAMPLE 4

### GPR85 KO AND GPR85/SREB3 DKO MICE EXHIBIT ALTERED METABOLIC CHARACTERISTICS

A variety of physiological and metabolic characteristics were analyzed in GPR85 KO and GPR85/SREB3 DKO mice, including body weight and food intake.

Growth curves were generated by measuring the animal's weight and length every one or two weeks. Both male and female GPR85 KO mice had lower body weight and length than WT, with more significant genotype difference in female mice. P values at 70 weeks were: female weight p<0.0001 (Figure 8A), male weight p=0.12 (Figure 8B), female length p<0.0001 (Figure 8C), male length p<0.05 (Figure 8D) (n = 8-10 per group, Student's t-test).

GPR85 KO mice had normal baseline food intake. There was no significant difference in the amount of daily food intake between genotypes in either male (Figure 9A) or female (Figure 9B) mice (n = 8-10 per group, Student's t-test). However, GPR85 KO mice ate less after a fasting challenge. Mice were subjected to a 24-hr fasting and then re-feeding. The amount of food eaten and the animal's body weight was measured at 1h, 4h and 24h after re-feeding. Daily food intake was also measured for Days 2-5. Both male (Figure 10A) and female (Figure 10B) KO mice had significantly less cumulative food intake during re-feeding than WT mice (*p<0.05, **p<0.01, n = 8-10 per group, Student's t-test).

GPR85 KO mice were slow to recover body weight after a fasting challenge. Body weight changes were monitored in the same experiment described above. Both male and female KO mice lost the same amount of weight as the WT mice after the fasting (Figures 11A and 11B, respectively). However, during re-feeding, both male and female KO mice were significantly slower in regaining their body weight than WT mice (*p<0.05, **p<0.01, n = 8-10 per group, Student's t-test).

GPR85 KO mice had lower baseline body temperatures. Baseline body temperatures were measured by a rectal probe on singly housed, resting mice. Both male and female KO mice had significantly lower body temperature than WT mice (p<0.05, n = 9-11 per group, Student's t-test), as shown in Figure 12.

Female GPR85 KO mice had lower percentage of white fat. DEXA analysis showed that KO mice had significantly lower total fat mass than WT mice (p<0.05, n = 10 per group, Student's t-test, Figure 13A). Mice were then dissected, and abdominal white fat and brown fat were weighed. KO mice had a significantly lower amount of white fat (p<0.01, Figure 13B) but a normal amount of brown fat (Figure 13C). The percentage of white fat versus the whole body weight was also significantly lower in KO mice (p<0.01) by ∼33% (Figure 13D).

GPR85 KO mice have slightly reduced leptin and insulin levels. Blood was collected via tail clip or retro-orbital eye bleeding, and centrifuged in microtainer tubes (Becton-Dickinson, Franklin Lakes, NJ) for plasma separation. Plasma was assayed for cholesterol, T4, triglyceride, and glucose using a Prochem V biochemistry analyzer (Drew Scientific, Oxford, CT). Leptin, insulin and glucagon were measured by Linco Diagnostics (St. Charles, MO) using a Lincoplex luminex assay. There was a trend of reduction of baseline plasma leptin and insulin levels in both male and female KO mice (n = 7-10 per group, Figure 14). There was no difference in IGF-1 level between genotypes.

Old male GPR85 KO mice had improved glucose tolerance. Old (16 months) male KO mice had significantly lower fed and fasting glucose levels compared to WT mice (Figures 15A and 15B, *p<0.05, **p<0.01, n = 7-10 per group, Student's t-test). These mice were given an insulin challenge or a glucose challenge. In the insulin challenge test (Figure 15C), mice were fasted for 4 hours and then injected with 0.75 U/kg insulin via i.p., and blood glucose levels were measured at different time points afterwards. KO mice had significantly lower glucose levels and were slower in recovery (*p<0.05, **p<0.01). In the glucose challenge test (Figure 15D), mice were injected with 2 g/kg glucose via i.p., and blood glucose levels were measured at different time points afterwards. KO mice were faster in clearing glucose than WT at 1hr post injection (p<0.05).

GPR85/SREB3 DKO mice had greatly reduced body weight and body length. Growth curves were generated by measuring the animal's weight and length every two or four weeks. Both male and female DKO mice had significantly lower body weight and length than WT, with more significant genotype difference in male mice (n = 8-10 per group, Student's t-test). Male DKO mice weighed 27% less than WT (p<0.0001, Figure 16A). Female DKO mice weighed 18% less than WT (p<0.01, Figure 16B).

GPR85/SREB3 DKO mice eat more food per gram body weight. Both male and female DKO mice had lower baseline body weight than WT (Figure 17, *p<0.05, ***p<0.001, Student's t-test, n = 6-10 per group, ages 3-5 months). When normalized to their body weights, both male and female DKO mice ate more food per gram body weight (lower panels, *p<0.05, ***p<0.001).

These results suggest that SREBs play important roles in metabolism and that targeting SREBs may be therapeutically useful in the treatment of a variety of metabolic diseases and disorders, including obesity and diabetes.

### EXAMPLE 5

### GPR88 KNOCKOUT MICE HAVE REDUCED MOTOR COORDINATION

Neurological characteristics of GPR88 knockout (KO) mice were measured using a variety of tests. Rotarod is one of the most commonly used tests of motor coordination. The rotarod used (Rotamex-5, from Columbus Instruments, Columbus, OH) had 4 channels to test 4 mice simultaneously. The rotating rod was 3 cm in diameter. Its rotating speed could be programmed in fixed or accelerating mode.

GPR88 knockout mice were generated using methods described in U.S. Patent No. 6,228,639 and tested using the roratod. An accelerating program was used, with start speed set at 1 rpm, end ppm, and acceleration rate at 1 rpm per 3 sec. Each mouse was placed on top of the rod and the program was started. The mouse had to keep walking and adjusting its pace in order to stay on the rod. The trial terminated when the mouse fell off from the rod or started to cling to the rod and rotated along with it. Infrared beam sensors were located above the rotating rod to detect animals on top of the rod, and thus are used to record the latency for the mouse to fall off or start to rotate. Each mouse was tested in 4 trials a day, with an inter-trial interval of at least 1 min, for 4-5 days.

As shown in Figure 18, GPR88 KO mice were impaired in motor coordination as assessed by the rotarod test. The KO mice (n=16) had significantly shorter latency compared to wild type (WT) mice (n=18) in each of the 4 testing days (p<0.01 for day 1, p<0.001 for days 2-4, Student's t-test). These results indicate that GPR88 is involved in motor coordination.

### EXAMPLE 6

### GPR88 KNOCKOUT MICE ARE HYPERACTIVE IN THE OPEN FIELD

Open field activity of GPR88 knock-out mice was examined to determine if loss of GPR88 is associated with neurological defects. This assay provides data on the general activity level of mice (*i.e.,* hypo- or hyper-active), and indication of anxiety towards the aversive properties of the novel, open environment.

Open field activity was monitored in VersaMax chambers (Accuscan Instruments, Columbus, OH) measuring 40 x 40 cm. Locomotor activity was detected by photobeams breaks as the animal crossed each beam. The animal was placed in the center of the field and then left undisturbed for a period of time (20 min to 2 hr) in order to measure its spontaneous activity in a novel environment. Measurements used to assess locomotor activity included: horizontal activity, total distance traveled, vertical activity (rearing events - animal raises up on hindlimbs), rotation, stereotypy, and distance traveled in the center compared to total distance traveled (center: total distance ratio).

As shown in Figure 19, compared to WT mice (n=18), the KO mice (n=18) had increased horizontal activity (p<0.05, repeated measures ANOVA, Figure 19A), total distance traveled in the chamber (p<0.05, Figure 19B), vertical/rearing activity (p<0.001, Figure 19C) and stereotypy behavior (p<0.05, Figure 19D). These results suggest that GPR88 in involved in neuropsychiatric functions and/or motor control.

### EXAMPLE 7

### GPR88 KNOCKOUT MICE ARE HYPERSENSITIVE TO AMPHETAMINE-INDUCED ACTIVITY

Psychostimulants, including indirect dopamine agonist amphetamine, induce psychosis-like conditions manifested as hyperactivity and increased stereotypic behavior. The role of GPR88 in modulating these conditions was investigated using GPR88 knockout mice.

Amphetamine-induced hyperactivity of GPR88 knockout mice was measured by injecting the animals with amphetamine and monitoring the animals' activity levels. GPR88 KO mice were injected with amphetamine (2.5 mg/kg) via i.p. The mice were placed in the activity chambers 30 minutes after amphetamine injection, and their locomotor activities were monitored for 20 min.

As shown in Figure 20, compared to baseline locomotor activity in the absence of drugs, both WT and KO mice had significantly higher activity after amphetamine administration (p<0.001 respectively). However, the activity level of KO mice (n=18) was much higher than that of the WT mice (n=18) (p<0.001 for the first 4-min block, p<0.05 for the second 4-min block, p<0.05 during the whole 20-min period). There was a strong genotype x treatment interaction for the first 4-min block (F(3,68)=10.5, p<0.01, two-way ANOVA). These results strongly suggest that GPR88 modulates hyperactivity.

### EXAMPLE 8

### GPR88 KNOCKOUT MICE ARE SENSITIVE TO AMPHETAMINE-INDUCED REDUCTION OF PREPULSE INHIBITION (PPI).

Prepulse inhibition (PPI) of the acoustic startle response evaluates the brain's sensorimotor gating function that is often disrupted in schizophrenic and other psychotic conditions. Psychostimulants such as PCP and amphetamine reduce or disrupt PPI, and many antipsychotics increase PPI and/or reverse psychostimulant-induced reduction of PPI. In addition, the startle response to the loud noise itself (in the absense of any prepulse) is a measure of the basic sensorimotor reflex.

Preppulse inhibitor testing of GPR88 knockout mice was performed using the SR-Lab System (San Diego Instruments, San Diego, CA). A test session consisted of six trial types under the background noise of 70 dB. One type used a 40 msec, 120 dB noise as the startle stimulus. Four types contained acoustic startle stimulus preceded by acoustic prepulses of different intensity: the 20-msec prepulse noise of 73, 76, 79, or 82 dB was presented 100 msec before the 120 dB startle stimulus. The last trial type used the 70 dB background noise with no startle stimulus to measure baseline reaction. Six blocks of the six trial types were presented in pseudorandom order. The startle response was recorded for 65 ms starting with the onset of the startle stimulus. Measurements used to assess PPI were the maximum startle amplitude and the percent each of the 4 prepulses inhibited the startle response.

As shown in Figure 21, GPR88 KO mice tended to be more sensitive to amphetamine-induced reduction of prepulse inhibition (PPI). GPR88 KO (n=20) and WT (n=21) mice in the inbred 129sv background were tested for baseline PPI, and there was no significant difference between genotypes (p>0.3, repeated measures ANOVA). Several weeks later, the mice were injected with amphetamine (2.5 mg/kg) via i.p. and tested for PPI 30 minutes later. The startle responses to the 120 dB stimulus of both WT and KO were significantly decreased after amphetamine treatment, and there was no significant difference between genotypes (p>0.5, Figure 21A). There was significant reduction of PPI in both WT and KO after amphetamine treatment (p<0.001 for both, Figure 21B). There was a trend of more reduction of PPI in KO than WT (p=0.091, repeated measures ANOVA).

### EXAMPLE 9

### GPR22 KO MICE ARE HYPOACTIVE AND ANXIOUS IN THE OPEN FIELD

Open field activity of GPR22 knock-out mice was examined to determine if loss of GPR22 is associated with neurological defects. This assay provides data on the general activity level of mice (*i.e.,* hypo- or hyper-active), and indication of anxiety towards the aversive properties of the novel, open environment.

GPR22 knock-out 9KO) mice were generated using methods described in U.S. Patent No. 6,228,639. Open field activity was monitored in VersaMax chambers (Accuscan Instruments, Columbus, OH) measuring 40 x 40 cm. Locomotor activity was detected by photobeams breaks as the animal crossed each beam. Mice were placed in the center of the field and then left undisturbed for a period of time (20 min to 2 hr) in order to measure their spontaneous activity in a novel environment. Measurements used to assess locomotor activity included: horizontal activity, total distance traveled, vertical activity (rearing events - animal raises up on hind limbs), rotation, and stereotypy. Measurements related to anxiety included time spent in the center arena of the chamber and distance traveled in the center compared to total distance traveled (center:total distance ratio). Data was plotted in 4-min bins.

As shown in Figure 22, GPR22 KO mice were hypoactive and more anxious in the open field activity test. Compared to wild type (WT) mice (n=23), the KO mice (n=27) traveled less distance during the testing period (p<0.05, repeated measures ANOVA), and spent less time in the center arena of the chamber (p<0.05, Student's t-test). These results indicate that GPR22 is involved in anxiety disorders, panic attacks, PTSD, stress disorders, and mood regulation.

### EXAMPLE 10

### GPR22 KO MICE HAVE INCREASED ANXIETY

A light-dark box was used to determine whether GPR22 knock-out mice were more prone to anxiety. The light-dark box measures the conflict between the natural tendencies of mice to explore a novel environment but to avoid the aversive properties of a brightly lit (anxiety-provoking) open field. It has two compartments. The brightly-lit compartment (27 cm x 20 cm x 30 cm) comprises two-thirds of the surface area, while the dark compartment (18 cm x 20 cm x 30 cm) comprises one-third of the surface area. An opening is designed to allow the mouse access to both compartments.

GPR22 knockout mice were placed individually into a light-dark box for 6 min. The timing of each transition from the dark to light or light to dark compartments, defined as all four limbs of the animal crossing the boundary, was recorded during the period using The Observer Mobile with a Psion Workabout (Noldus Information Technology, Netherlands).

As shown in Figure 23, GPR22 KO mice were more anxious in the light-dark box test. In this test, less number of transitions between the light and dark compartments and more time spent in the dark compartment were two indicators of increased anxiety in animals. Compared to WT mice (n=21), the KO mice (n=26) had a lower number of transitions between the light and dark compartments (p<0.05, Student's t-test), and spent less time in the light compartment (p<0.05). These results indicate that reduced GPR22 activity is associated with anxiety.

### EXAMPLE 11

### GPR22 KO MICE ARE HYPERACTIVE AT NIGHT

Activity of GPR22 knockout mice was determined by measuring their home cage activity. This data provides insight into the animal's circadian patterns of sleep-wake cycles.

Home cage activity of GPR22 knockout mice was monitored in MicroMax chambers (Accuscan Instruments, Columbus, OH) that were exterior to the cages. The photobeams provided information of when an animal was moving around in its home cage. Animals singly housed in their home cages were placed in the photobeam boxes for three to seven days and activity was recorded continually. Measurements used to assess home cage activity included: horizontal activity, total distance traveled, rotation, and periods of rest time and active time (onset, offset and duration). Data was plotted in 1-hr bins.

GPR22 KO mice were hyperactive at night in their home cages (Figure 24). Compared to WT mice (n=23), the KO mice (n=23) had significantly higher locomotor activity levels during the night time (p<0.01, repeated measures ANOVA). During the daytime, locomotor activity levels were not significantly different between genotypes. These results suggest that GPR22 is involved with basal nighttime activity, which may impact circadian rhythms and sleep patterns.

## Claims

1. A non-human mammal comprising a mutation in a gene encoding a polypeptide having the amino acid sequence set forth in SEQ ID NO: 1, wherein the mutation leads to a reduced or disrupted expression or activity of the gene, and wherein said non-human mammal exhibits one or more symptoms of a metabolic disease or disorder.

2. The non-human mammal of claim 1, further comprising a mutation in a second gene encoding a polypeptide having the amino acid sequence set forth in SEQ ID NO: 5, wherein the mutation of the second gene leads to a reduced or disrupted expression or activity of said second gene.

3. A cell isolated from the non-human mammal of any of claims 1-2.

4. An animal model of a metabolic disease or disorder, comprising the non-human mammal of claim 1 or 2.

5. A method for identifying a compound for the treatment of a metabolic disease or disorder, said method comprising administering a modulator to an animal of claim 1 or 2, or an animal model of claim 4, and determining whether said modulator reduces said one or more symptoms, thereby indicating that said modulator is useful for the treatment of a metabolic disease or disorder.

6. The non-human mammal of claim 1 or 2, or the animal model of claim 4, or the method of claim 5, wherein the metabolic disease or disorder is selected from the group consisting of: obesity, diabetes, metabolic syndrome, and anorexia.

## Patentansprüche

1. Ein nicht-humanes Säugetier, umfassend eine Mutation in einem Gen, das ein Polypeptid kodiert, das die Aminosäuresequenz gemäß SEQ ID NO: 1 hat, wobei die Mutation zu einer reduzierten oder unterbrochenen Expression oder Aktivität des Gens führt und wobei das nicht-humane Säugetier ein oder mehrere Symptome einer metabolischen Erkrankung oder Funktionsstörung aufweist.

2. Das nicht-humane Säugetier gemäß Anspruch 1, ferner umfassend eine Mutation in einem zweiten Gen, das ein Polypeptid kodiert, das die Aminosäuresequenz gemäß SEQ ID NO: 5 hat, wobei die Mutation des zweiten Gens zu einer reduzierten oder unterbrochenen Expression oder Aktivität des zweiten Gens führt.

3. Eine Zelle, die von dem nicht-humanen Säugetier gemäß irgendeinem der Ansprüche 1-2 isoliert ist.

4. Ein Tiermodell einer metabolischen Erkrankung oder Funktionsstörung, umfassend das nicht-humane Säugetier gemäß Anspruch 1 oder 2.

5. Ein Verfahren zum Identifizieren einer Verbindung zur Behandlung einer metabolischen Erkrankung oder Funktionsstörung, wobei das Verfahren das Verabreichen eines Modulators an ein Tier gemäß Anspruch 1 oder 2, oder an ein Tiermodell gemäß Anspruch 4 umfasst und das Bestimmen, ob der Modulator das eine oder mehrere Symptome reduziert und dadurch anzeigt, dass der Modulator verwendbar in der Behandlung einer metabolischen Erkrankung oder Funktionsstörung ist.

6. Das nicht-humane Säugetier gemäß Anspruch 1 oder 2, oder das Tiermodell gemäß Anspruch 4, oder das Verfahren gemäß Anspruch 5, wobei die metabolische Erkrankung oder Funktionsstörung ausgewählt ist aus einer Gruppe, bestehend aus Übergewicht, Diabetes, metabolisches Syndrom oder Anorexie.

## Revendications

1. Mammifère non humain comprenant une mutation dans un gène codant pour un polypeptide ayant la séquence d'acides aminés énoncée dans SEQ ID n° : 1, la mutation conduisant à une expression ou une activité réduite ou interrompue du gène, et ledit mammifère non humain faisant preuve d'un ou plusieurs symptômes de maladie ou de trouble métabolique.

2. Mammifère non humain selon la revendication 1, comprenant en outre une mutation dans un second gène codant pour un polypeptide ayant la séquence d'acides aminés énoncée dans SEQ ID n° : 5, la mutation du second gène conduisant à une expression ou une activité réduite ou interrompue dudit second gène.

3. Cellule isolée du mammifère non humain selon l'une quelconque des revendications 1 à 2.

4. Modèle animal d'une maladie ou d'un trouble métabolique, comprenant le mammifère non humain selon la revendication 1 ou 2.

5. Procédé d'identification d'un composé destiné au traitement d'une maladie ou d'un trouble métabolique, ledit procédé comprenant l'administration d'un modulateur à un animal selon la revendication 1 ou 2, ou à un modèle animal selon la revendication 4, et la détermination du fait que ledit modulateur réduit lesdits un ou plusieurs symptômes, indiquant de là que ledit modulateur est utile au traitement d'une maladie ou d'un trouble métabolique.

6. Mammifère non humain selon la revendication 1 ou 2, ou modèle animal selon la revendication 4, ou procédé selon la revendication 5, la maladie ou le trouble métabolique étant sélectionné-e dans le groupe constitué : de l'obésité, du diabète, du syndrome métabolique, et de l'anorexie.


### SEQUENCE LISTING

<110> Omeros Corporation
<120> G PROTEIN COUPLED RECEPTORS AND USES THEREOF
<130> EP65662FZpau
<140> 07 868 766.2
   <141> 2007-11-15
<150> PCT/US2007/084877
   <151> 2007-11-15
<150> 60/859,469
   <151> 2006-11-15
<150> 60/859,470
   <151> 2006-11-15
<150> 60/859,473
   <151> 2006-11-15
<160> 20
<170> FastSEQ for Windows version 4.0
<210> 1
   <211> 370
   <212> PRT
   <213> Homo sapiens
<400> 1
   Met Ala Asn Tyr Ser His Ala Ala Asp Asn Ile Leu Gln Asn Leu Ser
   1               5                   10                  15
   Pro Leu Thr Ala Phe Leu Lys Leu Thr Ser Leu Gly Phe Ile Ile Gly
               20                  25                  30
   Val Ser Val Val Gly Asn Leu Leu Ile Ser Ile Leu Leu Val Lys Asp
           35                  40                  45
   Lys Thr Leu His Arg Ala Pro Tyr Tyr Phe Leu Leu Asp Leu Cys Cys
       50                  55                  60
   Ser Asp Ile Leu Arg Ser Ala Ile Cys Phe Pro Phe Val Phe Asn Ser
   65                  70                  75                  80
   Val Lys Asn Gly Ser Thr Trp Thr Tyr Gly Thr Leu Thr Cys Lys Val
                   85                  90                  95
   Ile Ala Phe Leu Gly Val Leu Ser Cys Phe His Thr Ala Phe Met Leu
               100                 105                 110
   Phe Cys Ile Ser Val Thr Arg Tyr Leu Ala Ile Ala His His Arg Phe
           115                 120                 125
   Tyr Thr Lys Arg Leu Thr Phe Trp Thr Cys Leu Ala Val Ile Cys Met
       130                 135                 140
   Val Trp Thr Leu Ser Val Ala Met Ala Phe Pro Pro Val Leu Asp Val
   145                 150                 155                 160
   Gly Thr Tyr Ser Phe Ile Arg Glu Glu Asp Gln Cys Thr Phe Gln His
                   165                 170                 175
   Arg Ser Phe Arg Ala Asn Asp Ser Leu Gly Phe Met Leu Leu Leu Ala
               180                 185                 190
   Leu Ile Leu Leu Ala Thr Gln Leu Val Tyr Leu Lys Leu Ile Phe Phe
           195                 200                 205
   Val His Asp Arg Arg Lys Met Lys Pro Val Gln Phe Val Ala Ala Val
       210                 215                 220
   Ser Gln Asn Trp Thr Phe His Gly Pro Gly Ala Ser Gly Gln Ala Ala
   225                 230                 235                 240
   Ala Asn Trp Leu Ala Gly Phe Gly Arg Gly Pro Thr Pro Pro Thr Leu
                   245                 250                 255
   Leu Gly Ile Arg Gln Asn Ala Asn Thr Thr Gly Arg Arg Arg Leu Leu
               260                 265                 270
   Val Leu Asp Glu Phe Lys Met Glu Lys Arg Ile Ser Arg Met Phe Tyr
           275                 280                 285
   Ile Met Thr Phe Leu Phe Leu Thr Leu Trp Gly Pro Tyr Leu Val Ala
       290                 295                 300
   Cys Tyr Trp Arg Val Phe Ala Arg Gly Pro Val Val Pro Gly Gly Phe
   305                 310                 315                 320
   Leu Thr Ala Ala Val Trp Met Ser Phe Ala Gln Ala Gly Ile Asn Pro
                   325                 330                 335
   Phe Val Cys Ile Phe Ser Asn Arg Glu Leu Arg Arg Cys Phe Ser Thr
               340                 345                 350
   Thr Leu Leu Tyr Cys Arg Lys Ser Arg Leu Pro Arg Glu Pro Tyr Cys
           355                 360                 365
   Val Ile
       370
<210> 2
   <211> 1113
   <212> DNA
   <213> Homo sapiens
<400> 2
   atggcgaact atagccatgc agctgacaac attttgcaaa atctctcgcc tctaacagcc 60
   tttctgaaac tgacttcctt gggtttcata ataggagtca gcgtggtggg caacctcctg 120
   atctccattt tgctagtgaa agataagacc ttgcatagag caccttacta cttcctgttg 180
   gatctttgct gttcagatat cctcagatct gcaatttgtt tcccatttgt gttcaactct 240
   gtcaaaaatg gctctacctg gacttatggg actctgactt gcaaagtgat tgcctttctg 300
   ggggttttgt cctgtttcca cactgctttc atgctcttct gcatcagtgt caccagatac 360
   ttagctatcg cccatcaccg cttctataca aagaggctga ccttttggac gtgtctggct 420
   gtgatctgta tggtgtggac tctgtctgtg gccatggcat ttcccccggt tttagacgtg 480
   ggcacttact cattcattag ggaggaagat caatgcacct tccaacaccg ctccttcagg 540
   gctaatgatt ccttaggatt tatgctgctt cttgctctca tcctcctagc cacacagctt 600
   gtctacctca agctgatatt tttcgtccac gatcgaagaa aaatgaagcc agtccagttt 660
   gtagcagcag tcagccagaa ctggactttt catggtcctg gagccagtgg ccaggcagct 720
   gccaattggc tagcaggatt tggaaggggt cccacaccac ccaccttgct gggcatcagg 780
   caaaatgcaa acaccacagg cagaagaagg ctattggtct tagacgagtt caaaatggag 840
   aaaagaatca gcagaatgtt ctatataatg acttttctgt ttctaacctt gtggggcccc 900
   tacctggtgg cctgttattg gagagttttt gcaagagggc ctgtagtacc agggggattt 960
   ctaacagctg ctgtctggat gagttttgcc caagcaggaa tcaatccttt tgtctgcatt 1020
   ttctcaaaca gggagctgag gcgctgtttc agcacaaccc ttctttactg cagaaaatcc 1080
   aggttaccaa gggaacctta ctgtgttata tga                               1113
<210> 3
   <211> 375
   <212> PRT
   <213> Homo sapiens
<400> 3
   Met Ala Asn Ala Ser Glu Pro Gly Gly Ser Gly Gly Gly Glu Ala Ala
   1               5                   10                  15
   Ala Leu Gly Leu Lys Leu Ala Thr Leu Ser Leu Leu Leu Cys Val Ser
               20                  25                  30
   Leu Ala Gly Asn Val Leu Phe Ala Leu Leu Ile Val Arg Glu Arg Ser
           35                  40                  45
   Leu His Arg Ala Pro Tyr Tyr Leu Leu Leu Asp Leu Cys Leu Ala Asp
       50                  55                  60
   Gly Leu Arg Ala Leu Ala Cys Leu Pro Ala Val Met Leu Ala Ala Arg
   65                  70                  75                  80
   Arg Ala Ala Ala Ala Ala Gly Ala Pro Pro Gly Ala Leu Gly Cys Lys
                   85                  90                  95
   Leu Leu Ala Phe Leu Ala Ala Leu Phe Cys Phe His Ala Ala Phe Leu
               100                 105                 110
   Leu Leu Gly Val Gly Val Thr Arg Tyr Leu Ala Ile Ala His His Arg
           115                 120                 125
   Phe Tyr Ala Glu Arg Leu Ala Gly Trp Pro Cys Ala Ala Met Leu Val
       130                 135                 140
   Cys Ala Ala Trp Ala Leu Ala Leu Ala Ala Ala Phe Pro Pro Val Leu
   145                 150                 155                 160
   Asp Gly Gly Gly Asp Asp Glu Asp Ala Pro Cys Ala Leu Glu Gln Arg
                   165                 170                 175
   Pro Asp Gly Ala Pro Gly Ala Leu Gly Phe Leu Leu Leu Leu Ala Val
               180                 185                 190
   Val Val Gly Ala Thr His Leu Val Tyr Leu Arg Leu Leu Phe Phe Ile
           195                 200                 205
   His Asp Arg Arg Lys Met Arg Pro Ala Arg Leu Val Pro Ala Val Ser
       210                 215                 220
   His Asp Trp Thr Phe His Gly Pro Gly Ala Thr Gly Gln Ala Ala Ala
   225                 230                 235                 240
   Asn Trp Thr Ala Gly Phe Gly Arg Gly Pro Thr Pro Pro Ala Leu Val
                   245                 250                 255
   Gly Ile Arg Pro Ala Gly Pro Gly Arg Gly Ala Arg Arg Leu Leu Val
               260                 265                 270
   Leu Glu Glu Phe Lys Thr Glu Lys Arg Leu Cys Lys Met Phe Tyr Ala
           275                 280                 285
   Val Thr Leu Leu Phe Leu Leu Leu Trp Gly Pro Tyr Val Val Ala Ser
       290                 295                 300
   Tyr Leu Arg Val Leu Val Arg Pro Gly Ala Val Pro Gln Ala Tyr Leu
   305                 310                 315                 320
   Thr Ala Ser Val Trp Leu Thr Phe Ala Gln Ala Gly Ile Asn Pro Val
                   325                 330                 335
   Val Cys Phe Leu Phe Asn Arg Glu Leu Arg Asp Cys Phe Arg Ala Gln
               340                 345                 350
   Phe Pro Cys Cys Gln Ser Pro Arg Thr Thr Gln Ala Thr His Pro Cys
           355                 360                 365
   Asp Leu Lys Gly Ile Gly Leu
       370                 375
<210> 4
   <211> 1128
   <212> DNA
   <213> Homo sapiens
<400> 4
   atggcgaacg cgagcgagcc gggtggcagc ggcggcggcg aggcggccgc cctgggcctc 60
   aagctggcca cgctcagcct gctgctgtgc gtgagcctag cgggcaacgt gctgttcgcg 120
   ctgctgatcg tgcgggagcg cagcctgcac cgcgccccgt actacctgct gctcgacctg 180
   tgcctggccg acgggctgcg cgcgctcgcc tgcctcccgg ccgtcatgct ggcggcgcgg 240
   cgtgcggcgg ccgcggcggg ggcgccgccg ggcgcgctgg gctgcaagct gctcgccttc 300
   ctggccgcgc tcttctgctt ccacgccgcc ttcctgctgc tgggcgtggg cgtcacccgc 360
   tacctggcca tcgcgcacca ccgcttctat gcagagcgcc tggccggctg gccgtgcgcc 420
   gccatgctgg tgtgcgccgc ctgggcgctg gcgctggccg cggccttccc gccagtgctg 480
   gacggcggtg gcgacgacga ggacgcgccg tgcgccctgg agcagcggcc cgacggcgcc 540
   cccggcgcgc tgggcttcct gctgctgctg gccgtggtgg tgggcgccac gcacctcgtc 600
   tacctccgcc tgctcttctt catccacgac cgccgcaaga tgcggcccgc gcgcctggtg 660
   cccgccgtca gccacgactg gaccttccac ggcccgggcg ccaccggcca ggcggccgcc 720
   aactggacgg cgggcttcgg ccgcgggccc acgccgcccg cgcttgtggg catccggccc 780
   gcagggccgg gccgcggcgc gcgccgcctc ctcgtgctgg aagaattcaa gacggagaag 840
   aggctgtgca agatgttcta cgccgtcacg ctgctcttcc tgctcctctg ggggccctac 900
   gtcgtggcca gctacctgcg ggtcctggtg cggcccggcg ccgtccccca ggcctacctg 960
   acggcctccg tgtggctgac cttcgcgcag gccggcatca accccgtcgt gtgcttcctc 1020
   ttcaacaggg agctgaggga ctgcttcagg gcccagttcc cctgctgcca gagcccccgg 1080
   accacccagg cgacccatcc ctgcgacctg aaaggcattg gtttatga             1128
<210> 5
   <211> 373
   <212> PRT
   <213> Homo sapiens
<400> 5
   Met Ala Asn Thr Thr Gly Glu Pro Glu Glu Val Ser Gly Ala Leu Ser
   1               5                   10                  15
   Pro Pro Ser Ala Ser Ala Tyr Val Lys Leu Val Leu Leu Gly Leu Ile
               20                  25                  30
   Met Cys Val Ser Leu Ala Gly Asn Ala Ile Leu Ser Leu Leu Val Leu
           35                  40                  45
   Lys Glu Arg Ala Leu His Lys Ala Pro Tyr Tyr Phe Leu Leu Asp Leu
       50                  55                  60
   Cys Leu Ala Asp Gly Ile Arg Ser Ala Val Cys Phe Pro Phe Val Leu
   65                  70                  75                  80
   Ala Ser Val Arg His Gly Ser Ser Trp Thr Phe Ser Ala Leu Ser Cys
                   85                  90                  95
   Lys Ile Val Ala Phe Met Ala Val Leu Phe Cys Phe His Ala Ala Phe
               100                 105                 110
   Met Leu Phe Cys Ile Ser Val Thr Arg Tyr Met Ala Ile Ala His His
           115                 120                 125
   Arg Phe Tyr Ala Lys Arg Met Thr Leu Trp Thr Cys Ala Ala Val Ile
       130                 135                 140
   Cys Met Ala Trp Thr Leu Ser Val Ala Met Ala Phe Pro Pro Val Phe
   145                 150                 155                 160
   Asp Val Gly Thr Tyr Lys Phe Ile Arg Glu Glu Asp Gln Cys Ile Phe
                   165                 170                 175
   Glu His Arg Tyr Phe Lys Ala Asn Asp Thr Leu Gly Phe Met Leu Met
               180                 185                 190
   Leu Ala Val Leu Met Ala Ala Thr His Ala Val Tyr Gly Lys Leu Leu
           195                 200                 205
   Leu Phe Glu Tyr Arg His Arg Lys Met Lys Pro Val Gln Met Val Pro
       210                 215                 220
   Ala Ile Ser Gln Asn Trp Thr Phe His Gly Pro Gly Ala Thr Gly Gln
   225                 230                 235                 240
   Ala Ala Ala Asn Trp Ile Ala Gly Phe Gly Arg Gly Pro Met Pro Pro
                   245                 250                 255
   Thr Leu Leu Gly Ile Arg Gln Asn Gly His Ala Ala Ser Arg Arg Leu
               260                 265                 270
   Leu Gly Met Asp Glu Val Lys Gly Glu Lys Gln Leu Gly Arg Met Phe
           275                 280                 285
   Tyr Ala Ile Thr Leu Leu Phe Leu Leu Leu Trp Ser Pro Tyr Ile Val
       290                 295                 300
   Ala Cys Tyr Trp Arg Val Phe Val Lys Ala Cys Ala Val Pro His Arg
   305                 310                 315                 320
   Tyr Leu Ala Thr Ala Val Trp Met Ser Phe Ala Gln Ala Ala Val Asn
                   325                 330                 335
   Pro Ile Val Cys Phe Leu Leu Asn Lys Asp Leu Lys Lys Cys Leu Arg
               340                 345                 350
   Thr His Ala Pro Cys Trp Gly Thr Gly Gly Ala Pro Ala Pro Arg Glu
           355                 360                 365
   Pro Tyr Cys Val Met
       370
<210> 6
   <211> 1122
   <212> DNA
   <213> Homo sapiens
<400> 6
   atggccaaca ctaccggaga gcctgaggag gtgagcggcg ctctgtcccc accgtccgca 60
   tcagcttatg tgaagctggt actgctggga ctgattatgt gcgtgagcct ggcgggtaac 120
   gccatcttgt ccctgctggt gctcaaggag cgtgccctgc acaaggctcc ttactacttc 180
   ctgctggacc tgtgcctggc cgatggcata cgctctgccg tctgcttccc ctttgtgctg 240
   gcttctgtgc gccacggctc ttcatggacc ttcagtgcac tcagctgcaa gattgtggcc 300
   tttatggccg tgctcttttg cttccatgcg gccttcatgc tgttctgcat cagcgtcacc 360
   cgctacatgg ccatcgccca ccaccgcttc tacgccaagc gcatgacact ctggacatgc 420
   gcggctgtca tctgcatggc ctggaccctg tctgtggcca tggccttccc acctgtcttt 480
   gacgtgggca cctacaagtt tattcgggag gaggaccagt gcatctttga gcatcgctac 540
   ttcaaggcca atgacacgct gggcttcatg cttatgttgg ctgtgctcat ggcagctacc 600
   catgctgtct acggcaagct gctcctcttc gagtatcgtc accgcaagat gaagccagtg 660
   cagatggtgc cagccatcag ccagaactgg acattccatg gtcccggggc caccggccag 720
   gctgctgcca actggatcgc cggctttggc cgtgggccca tgccaccaac cctgctgggt 780
   atccggcaga atgggcatgc agccagccgg cggctactgg gcatggacga ggtcaagggt 840
   gaaaagcagc tgggccgcat gttctacgcg atcacactgc tctttctgct cctctggtca 900
   ccctacatcg tggcctgcta ctggcgagtg tttgtgaaag cctgtgctgt gccccaccgc 960
   tacctggcca ctgctgtttg gatgagcttc gcccaggctg ccgtcaaccc aattgtctgc 1020
   ttcctgctca acaaggacct caagaagtgc ctgaggactc acgccccctg ctggggcaca 1080
   ggaggtgccc cggctcccag agaaccctac tgtgtcatgt ga                    1122
<210> 7
   <211> 370
   <212> PRT
   <213> Mus musculus
<400> 7
   Met Ala Asn Tyr Ser His Ala Ala Asp Asn Ile Leu Gln Asn Leu Ser
    1               5                  10                  15
   Pro Leu Thr Ala Phe Leu Lys Leu Thr Ser Leu Gly Phe Ile Ile Gly
               20                  25                  30
   Val Ser Val Val Gly Asn Leu Leu Ile Ser Ile Leu Leu Val Lys Asp
           35                  40                  45
   Lys Thr Leu His Arg Ala Pro Tyr Tyr Phe Leu Leu Asp Leu Cys Cys
       50                  55                  60
   Ser Asp Ile Leu Arg Ser Ala Ile Cys Phe Pro Phe Val Phe Asn Ser
   65                  70                  75                  80
   Val Lys Asn Gly Ser Thr Trp Thr Tyr Gly Thr Leu Thr Cys Lys Val
                   85                  90                  95
   Ile Ala Phe Leu Gly Val Leu Ser Cys Phe His Thr Ala Phe Met Leu
               100                 105                 110
   Phe Cys Ile Ser Val Thr Arg Tyr Leu Ala Ile Ala His His Arg Phe
           115                 120                 125
   Tyr Thr Lys Arg Leu Thr Phe Trp Thr Cys Leu Ala Val Ile Cys Met
       130                 135                 140
   Val Trp Thr Leu Ser Val Ala Met Ala Phe Pro Pro Val Leu Asp Val
   145                 150                 155                 160
   Gly Thr Tyr Ser Phe Ile Arg Glu Glu Asp Gln Cys Thr Phe Gln His
                   165                 170                 175
   Arg Ser Phe Arg Ala Asn Asp Ser Leu Gly Phe Met Leu Leu Leu Ala
               180                 185                 190
   Leu Ile Leu Leu Ala Thr Gln Leu Val Tyr Leu Lys Leu Ile Phe Phe
           195                 200                 205
   Val His Asp Arg Arg Lys Met Lys Pro Val Gln Phe Val Ala Ala Val
       210                 215                 220
   Ser Gln Asn Trp Thr Phe His Gly Pro Gly Ala Ser Gly Gln Ala Ala
   225                 230                 235                 240
   Ala Asn Trp Leu Ala Gly Phe Gly Arg Gly Pro Thr Pro Pro Thr Leu
                   245                 250                 255
   Leu Gly Ile Arg Gln Asn Ala Asn Thr Thr Gly Arg Arg Arg Leu Leu
               260                 265                 270
   Val Leu Asp Glu Phe Lys Met Glu Lys Arg Ile Ser Arg Met Phe Tyr
           275                 280                 285
   Ile Met Thr Phe Leu Phe Leu Thr Leu Trp Gly Pro Tyr Leu Val Ala
       290                 295                 300
   Cys Tyr Trp Arg Val Phe Ala Arg Gly Pro Val Val Pro Gly Gly Phe
   305                 310                 315                 320
   Leu Thr Ala Ala Val Trp Met Ser Phe Ala Gln Ala Gly Ile Asn Pro
                   325                 330                 335
   Phe Val Cys Ile Phe Ser Asn Arg Glu Leu Arg Arg Cys Phe Ser Thr
               340                 345                 350
   Thr Leu Leu Tyr Cys Arg Lys Ser Arg Leu Pro Arg Glu Pro Tyr Cys
           355                 360                 365
   Val Ile
       370
<210> 8
   <211> 1482
   <212> DNA
   <213> Mus musculus
<400> 8
   aattcaaacg gaaggcaaga ggaagcatgg ccacagatcg gatctgatct ctttgtggat 60
   tacattttca gtaaaatgta tggatctatc ttttccttgt tcttatatct agatcatgag 120
   acttgactga ggctgtttcc ttatcctcca tccatctatg gcgaactata gccatgcagc 180
   cgacaacatt ttgcaaaatc tctcgcctct aacagccttt ctgaaactga cttccctggg 240
   tttcataata ggagtcagcg ttgtgggcaa ccttctgatc tccattttgc tagtgaaaga 300
   taagaccttg catagagctc cttactactt cctgctggat ctgtgctgct cagacatcct 360
   cagatctgca atttgttttc catttgtatt caactctgtc aaaaatggct ccacctggac 420
   ttacgggact ctgacttgca aagtgattgc cttcctgggg gttttgtcct gtttccacac 480
   tgccttcatg ctcttctgca tcagcgtcac cagatactta gccatcgccc atcaccgctt 540
   ctatacaaag aggctgacct tttggacgtg tttggctgtg atctgcatgg tgtggactct 600
   gtccgtggcc atggcattcc ccccagtttt agatgtaggc acctactcct tcattaggga 660
   ggaggatcaa tgcaccttcc aacaccgctc cttcagggct aacgattccc taggatttat 720
   gctgctcctt gctctcatcc tcctagccac acagcttgtc tacctcaagc tgatattttt 780
   tgtccacgat cgaaggaaaa tgaagccagt ccagtttgta gcagcagtga gtcagaactg 840
   gacctttcat ggccctggag ctagcggcca ggcagctgcc aattggctag caggatttgg 900
   aaggggtccc acaccaccca ccttgctggg catcaggcaa aatgcgaata ccacaggcag 960
   aaggcggcta ttggtcttgg atgagttcaa aatggagaaa agaatcagca gaatgtttta 1020
   tataatgact ttcctcttcc taaccttgtg gggtccctac ctggtggcct gctattggag 1080
   agtttttgca cgagggcctg tagtaccagg gggatttcta acagccgctg tctggatgag 1140
   tttcgcccaa gcaggaatca atccctttgt ctgcattttc tccaacaggg agctgaggcg 1200
   ctgtttcagc acaacccttc tttactgcag aaaatccagg ttaccaaggg aaccttactg 1260
   tgttatatga gggagcatct gtaaatcttt agccttgtga aacactaacc ctctctgcta 1320
   agcaattgtg gcctgtagcc atgtctggag aagaaaacca aggatgggaa tcagcagtga 1380
   caaggatttg ggcaacattc tgcagtcttt gcaatagttc acctataatc atattttaaa 1440
   tttcggagtg atcctgctga ctgctggcga aggtttgtaa tt                    1482
<210> 9
   <211> 379
   <212> PRT
   <213> Mus musculus
<400> 9
   Met Ala Asn Ala Ser Glu Pro Gly Gly Gly Gly Ser Gly Gly Gly Ala
    1               5                  10                  15
   Glu Ala Ala Ala Leu Gly Leu Arg Leu Ala Thr Leu Ser Leu Leu Leu
               20                  25                  30
   Cys Val Ser Leu Ala Gly Asn Val Leu Phe Ala Leu Leu Ile Val Arg
           35                  40                  45
   Glu Arg Ser Leu His Arg Ala Pro Tyr Tyr Leu Leu Leu Asp Leu Cys
       50                  55                  60
   Leu Ala Asp Gly Leu Arg Ala Leu Ala Cys Leu Pro Ala Val Met Leu
   65                  70                  75                  80
   Ala Ala Arg Arg Ala Ala Ala Ala Ala Gly Thr Pro Pro Gly Ala Leu
                   85                  90                  95
   Gly Cys Lys Leu Leu Ala Phe Leu Ala Ala Leu Phe Cys Phe His Ala
               100                 105                 110
   Ala Phe Leu Leu Leu Gly Val Gly Val Thr Arg Tyr Leu Ala Ile Ala
           115                 120                 125
   His His Arg Phe Tyr Ala Glu Arg Leu Ala Gly Trp Pro Cys Ala Ala
       130                 135                 140
   Met Leu Val Cys Ala Ala Trp Ala Leu Ala Leu Ala Ala Ala Phe Pro
   145                 150                 155                 160
   Pro Val Leu Asp Gly Gly Gly Ala Asp Asp Glu Asp Ala Pro Cys Ala
                   165                 170                 175
   Leu Glu Gln Arg Pro Asp Gly Ala Pro Gly Ala Leu Gly Phe Leu Leu
               180                 185                 190
   Leu Leu Ala Ala Val Val Gly Ala Thr His Leu Val Tyr Leu Arg Leu
           195                 200                 205
   Leu Phe Phe Ile His Asp Arg Arg Lys Met Arg Pro Ala Arg Leu Val
       210                 215                 220
   Pro Ala Val Ser His Asp Trp Thr Phe His Gly Pro Gly Ala Thr Gly
   225                 230                 235                 240
   Gln Ala Ala Ala Asn Trp Thr Ala Gly Phe Gly Arg Gly Pro Thr Pro
                   245                 250                 255
   Pro Ala Leu Val Gly Ile Arg Pro Ala Gly Pro Gly Arg Gly Ala Arg
               260                 265                 270
   Arg Leu Leu Val Leu Glu Glu Phe Lys Thr Glu Lys Arg Leu Cys Lys
           275                 280                 285
   Met Phe Tyr Ala Ile Thr Leu Leu Phe Leu Leu Leu Trp Gly Pro Tyr
       290                 295                 300
   Val Val Ala Ser Tyr Leu Arg Val Leu Val Arg Pro Gly Ala Val Pro
   305                 310                 315                 320
   Gln Ala Tyr Leu Thr Ala Ser Val Trp Leu Thr Phe Ala Gln Ala Gly
                   325                 330                 335
   Ile Asn Pro Val Val Cys Phe Leu Phe Asn Arg Glu Leu Arg Asp Cys
               340                 345                 350
   Phe Arg Ala Gln Phe Pro Cys Cys Gln Ser Pro Gln Ala Thr Gln Ala
           355                 360                 365
   Thr Leu Pro Cys Asp Leu Lys Gly Ile Gly Leu
       370                 375
<210> 10
   <211> 1140
   <212> DNA
   <213> Mus musculus
<400> 10
   atggcgaacg ctagtgagcc gggcggcggc ggcagcggcg gaggggccga ggctgccgcg 60
   ctgggcctta ggctggccac gctcagcctg ctgctgtgcg tgagcctggc gggcaacgtg 120
   ctgttcgcgc tgctcatcgt gcgggagcgc agcctgcacc gcgcgccgta ctacctgctg 180
   ctcgacctgt gcctggccga cgggctgcgc gcgctcgcct gcctcccggc cgtcatgctg 240
   gcggcgcggc gcgcggcggc cgcggcgggg acgccgccgg gcgcgctggg ctgcaagctg 300
   ctggccttcc tggccgcact cttctgcttc cacgcggcct tcctgctgct gggcgtgggc 360
   gtcacccgct acctggccat cgcgcaccac cgcttctacg ccgagcgcct ggccggctgg 420
   ccgtgcgccg cgatgctggt gtgcgccgcc tgggcgctgg ctttggccgc ggccttcccg 480
   ccggtgctgg acggcggtgg cgcggacgac gaggacgcgc cgtgcgccct ggagcagcga 540
   cccgacggcg ccccgggtgc gctaggcttc ctgctgctcc tggccgcggt ggtgggcgcc 600
   acgcacctcg tctacctccg cctgctcttc ttcatccacg accgccgcaa gatgcgaccc 660
   gcgcgcctgg tgcccgccgt cagccacgac tggaccttcc acggcccggg cgccaccggt 720
   caggcggccg ccaactggac ggcgggcttc ggccgcgggc ccacgccacc tgcgctcgtg 780
   ggcatcaggc ctgcaggccc gggccgcggc gcccggcgcc tcctggtgct ggaggagttc 840
   aagacggaga agaggctgtg caagatgttc tacgccatca cgctgctctt cctgctcctc 900
   tggggcccct atgtggtggc cagttacctg cgcgtcctgg tgcgcccggg agctgtcccg 960
   caggcctacc tgacagcctc ggtgtggctg acattcgcgc aggccggcat caaccccgtg 1020
   gtgtgtttcc tcttcaaccg ggagctgagg gactgtttcc gagcccagtt tccctgctgc 1080
   cagagccccc aggccacgca ggccaccctc ccctgcgacc tgaaaggcat tggtttgtga 1140
<210> 11
   <211> 373
   <212> PRT
   <213> Mus musculus
<400> 11
   Met Ala Asn Thr Thr Gly Glu Pro Glu Glu Val Ser Gly Ala Leu Ser
   1               5                   10                  15
   Leu Pro Ser Ala Ser Ala Tyr Val Lys Leu Val Leu Leu Gly Leu Ile
               20                  25                  30
   Met Cys Val Ser Leu Ala Gly Asn Ala Ile Leu Ser Leu Leu Val Leu
           35                  40                  45
   Lys Glu Arg Ala Leu His Lys Ala Pro Tyr Tyr Phe Leu Leu Asp Leu
       50                  55                  60
   Cys Leu Ala Asp Gly Ile Arg Ser Ala Ile Cys Phe Pro Phe Val Leu
   65                  70                  75                  80
   Ala Ser Val Arg His Gly Ser Ser Trp Thr Phe Ser Ala Leu Ser Cys
                   85                  90                  95
   Lys Ile Val Ala Phe Met Ala Val Leu Phe Cys Phe His Ala Ala Phe
               100                 105                 110
   Met Leu Phe Cys Ile Ser Val Thr Arg Tyr Met Ala Ile Ala His His
           115                 120                 125
   Arg Phe Tyr Ala Lys Arg Met Thr Leu Trp Thr Cys Ala Ala Val Ile
       130                 135                 140
   Cys Met Ala Trp Thr Leu Ser Val Ala Met Ala Phe Pro Pro Val Phe
   145                 150                 155                 160
   Asp Val Gly Thr Tyr Lys Phe Ile Arg Glu Glu Asp Gln Cys Ile Phe
                   165                 170                 175
   Glu His Arg Tyr Phe Lys Ala Asn Asp Thr Leu Gly Phe Met Leu Met
               180                 185                 190
   Leu Ala Val Leu Met Ala Ala Thr His Ala Val Tyr Gly Lys Leu Leu
           195                 200                 205
   Leu Phe Glu Tyr Arg His Arg Lys Met Lys Pro Val Gln Met Val Pro
       210                 215                 220
   Ala Ile Ser Gln Asn Trp Thr Phe His Gly Pro Gly Ala Thr Gly Gln
   225                 230                 235                 240
   Ala Ala Ala Asn Trp Ile Ala Gly Phe Gly Arg Gly Pro Met Pro Pro
                   245                 250                 255
   Thr Leu Leu Gly Ile Arg Gln Asn Gly His Ala Ala Ser Arg Arg Leu
               260                 265                 270
   Leu Gly Met Asp Glu Val Lys Gly Glu Lys Gln Leu Gly Arg Met Phe
           275                 280                 285
   Tyr Ala Ile Thr Leu Leu Phe Leu Leu Leu Trp Ser Pro Tyr Ile Val
       290                 295                 300
   Ala Cys Tyr Trp Arg Val Phe Val Lys Ala Cys Ala Val Pro His Arg
   305                 310                 315                 320
   Tyr Leu Ala Thr Ala Val Trp Met Ser Phe Ala Gln Ala Ala Val Asn
                   325                 330                 335
   Pro Ile Val Cys Phe Leu Leu Asn Lys Asp Leu Lys Lys Cys Leu Arg
               340                 345                 350
   Thr His Ala Pro Cys Trp Gly Thr Gly Gly Ala Pro Ala Pro Arg Glu
           355                 360                 365
   Pro Tyr Cys Val Met
       370
<210> 12
   <211> 1119
   <212> DNA
   <213> Mus musculus
<400> 12
   atggccaaca ccaccggaga gcccgaagag gtgagcggcg cactgtctct gccatcagca 60
   tcggcttatg tgaagctggt gctgctggga ctgatcatgt gtgtaagcct ggcaggcaat 120
   gccatcttgt ccctgctggt gctcaaggag cgtgccctgc acaaggctcc ttactacttt 180
   ctgctggacc tgtgcctagc cgatggcata cgctctgcca tctgcttccc ctttgtactg 240
   gcttctgtgc gccatggctc ctcatggacc ttcagtgcac tcagctgtaa gattgtggcc 300
   tttatggctg tgctcttttg cttccatgcg gccttcatgc tgttctgcat cagcgtcacc 360
   cgctacatgg ccatcgccca ccaccgcttc tatgccaagc gcatgacact ctggacatgc 420
   gcagctgtca tctgcatggc ctggaccttg tctgtggcca tggctttccc acctgtcttt 480
   gatgtgggca cctacaagtt tatccgagag gaggaccagt gcatctttga gcatcgctac 540
   ttcaaagcaa atgacacact gggctttatg cttatgttgg ctgtgctcat ggcagccaca 600
   catgctgtct atggcaagct gctactcttc gagtatcgtc accgcaagat gaagccagtg 660
   cagatggtgc cagccatcag ccaaaactgg acattccatg gccctggggc taccggccag 720
   gctgctgcca actggatcgc tggttttggc cgtgggccca tgccaccaac tctgctgggt 780
   atccggcaga atgggcatgc agctagccgg cggctactgg gcatggacga ggtcaagggt 840
   gaaaagcagc tgggccgaat gttctacgcg attacactgc tcttcctgct cctctggtca 900
   ccatacattg tggcctgcta ctggcgagtg tttgtgaaag cctgcgctgt gccccaccgc 960
   tacttggcca ctgctgtttg gatgagcttc gcccaggctg ccgtcaaccc aatcgtctgc 1020
   ttcctgctta acaaggacct caagaagtgc ctgaggactc atgccccttg ctggggcaca 1080
   ggaggtgccc cagctcccag agaaccctac tgtgtcatg                        1119
<210> 13
   <211> 384
   <212> PRT
   <213> Homo sapiens
<400> 13
   Met Thr Asn Ser Ser Ser Thr Ser Thr Ser Ser Thr Thr Gly Gly Ser
    1               5                   10                  15
   Leu Leu Leu Leu Cys Glu Glu Glu Glu Ser Trp Ala Gly Arg Arg Ile
               20                  25                  30
   Pro Val Ser Leu Leu Tyr Ser Gly Leu Ala Ile Gly Gly Thr Leu Ala
           35                  40                  45
   Asn Gly Met Val Ile Tyr Leu Val Ser Ser Phe Arg Lys Leu Gln Thr
       50                  55                  60
   Thr Ser Asn Ala Phe Ile Val Asn Gly Cys Ala Ala Asp Leu Ser Val
   65                  70                  75                  80
   Cys Ala Leu Trp Met Pro Gln Glu Ala Val Leu Gly Leu Leu Pro Thr
                   85                  90                  95
   Gly Ser Ala Glu Pro Pro Ala Asp Trp Asp Gly Ala Gly Gly Ser Tyr
               100                 105                 110
   Arg Leu Leu Arg Gly Gly Leu Leu Gly Leu Gly Leu Thr Val Ser Leu
           115                 120                 125
   Leu Ser His Cys Leu Val Ala Leu Asn Arg Tyr Leu Leu Ile Thr Arg
       130                 135                 140
   Ala Pro Ala Thr Tyr Gln Ala Leu Tyr Gln Arg Arg His Thr Ala Gly
   145                 150                 155                 160
   Met Leu Ala Leu Ser Trp Ala Leu Ala Leu Gly Leu Val Leu Leu Leu
                   165                 170                 175
   Pro Pro Trp Ala Pro Arg Pro Gly Ala Ala Pro Pro Arg Ile His Tyr
               180                 185                 190
   Pro Ala Leu Leu Ala Ala Ala Ala Leu Leu Ala Gln Thr Ala Leu Leu
           195                 200                 205
   Leu His Cys Tyr Leu Gly Ile Val Arg Arg Val Arg Val Ser Val Lys
       210                 215                 220
   Arg Val Ser Val Leu Asn Phe His Leu Leu His Gln Leu Pro Gly Cys
   225                 230                 235                 240
   Ala Ala Ala Ala Ala Ala Phe Pro Gly Ala Gln His Ala Pro Gly Pro
                   245                 250                 255
   Gly Gly Ala Ala His Pro Ala Gln Ala Gln Pro Leu Pro Pro Ala Leu
               260                 265                 270
   His Pro Arg Arg Ala Gln Arg Arg Leu Ser Gly Leu Ser Val Leu Leu
           275                 280                 285
   Leu Cys Cys Val Phe Leu Leu Ala Thr Gln Pro Leu Val Trp Val Ser
       290                 295                 300
   Leu Ala Ser Gly Phe Ser Leu Pro Val Pro Trp Gly Val His Ala Ala
   305                 310                 315                 320
   Ser Trp Leu Leu Cys Cys Ala Leu Ser Ala Leu Asn Pro Leu Leu Tyr
                   325                 330                 335
   Thr Trp Arg Asn Glu Glu Phe Arg Arg Ser Val Arg Ser Val Leu Pro
               340                 345                 350
   Gly Val Gly Asp Ala Ala Ala Ala Ala Val Ala Ala Thr Ala Val Pro
           355                 360                 365
   Ala Val Ser Gln Ala Gln Leu Gly Thr Arg Ala Ala Gly Gln His Trp
       370                 375                 380
<210> 14
   <211> 1155
   <212> DNA
   <213> Homo sapiens
<400> 14
   atgaccaact cctcctccac atccacctcc tccaccaccg gtggctcgct gctgctgctc 60
   tgcgaggaag aggagtcgtg ggcgggccgg cgcatcccgg tgtcactcct gtattcgggc 120
   ctggccatcg ggggcacgct ggccaacggc atggtcatct atctcgtgtc gtccttccga 180
   aagctgcaga ccaccagcaa cgccttcatt gtgaacggct gcgccgccga cctcagcgtc 240
   tgcgccctct ggatgccgca ggaggcggtg ctcgggctcc tgcccaccgg ctctgcggag 300
   ccccccgcag actgggacgg cgctgggggc agctaccgcc tgctacgggg tgggctgctg 360
   ggcctcggac tcacggtgtc cctcctctcc cactgcctcg tggccctgaa ccgctacctg 420
   ctcatcaccc gggcgcccgc cacctaccag gcgctgtacc agaggcgcca cacggcgggc 480
   atgctggcgc tgtcctgggc gctcgccctg ggcctcgtgc tgctgctccc gccctgggca 540
   ccgcggcccg gcgccgcgcc accgcgaatc cactacccgg cgctgctggc cgccgcggcg 600
   ctgctggcgc agacagctct gctgctgcac tgctacctgg gcatcgtgcg ccgcgtgcgt 660
   gtcagcgtca agcgggtcag cgtgctcaac ttccacctgc tgcaccagtt gcccggctgc 720
   gccgccgccg ccgccgcctt cccgggcgcc cagcacgcgc cgggccccgg tggcgccgcg 780
   cacccggcgc aggcccagcc cctgccgccc gcgctgcacc cgcggcgggc acagcggcgt 840
   ctcagcggcc tgtcggtgct gctgctctgc tgcgtcttcc tgctggccac gcagccactg 900
   gtgtgggtga gcctggccag cggcttctcg ctgccggtgc cctggggagt gcacgcggcc 960
   agctggctcc tgtgctgcgc cctgtccgcg ctcaacccgc tgctctacac gtggaggaac 1020
   gaggagttcc gccgctccgt gcgctcagtc ctgccgggcg tcggcgacgc ggcggccgct 1080
   gccgttgccg ccacagccgt gcccgcagtg tcccaggcgc aactgggcac ccgcgccgcg 1140
   ggccagcact ggtaa                                                  1155
<210> 15
   <211> 384
   <212> PRT
   <213> Mus musculus
<400> 15
   Met Thr Asn Ser Ser Ser Thr Ser Thr Ser Thr Thr Thr Gly Gly Ser
   1               5                   10                  15
   Leu Leu Leu Leu Cys Glu Glu Glu Glu Ser Trp Ala Gly Arg Arg Ile
               20                  25                  30
   Pro Val Ser Leu Leu Tyr Ser Gly Leu Ala Ile Gly Gly Thr Leu Ala
           35                  40                  45
   Asn Gly Met Val Ile Tyr Leu Val Ser Ser Phe Arg Lys Leu Gln Thr
       50                  55                  60
   Thr Ser Asn Ala Phe Ile Val Asn Gly Cys Ala Ala Asp Leu Ser Val
   65                  70                  75                  80
   Cys Ala Leu Trp Met Pro Gln Glu Ala Val Leu Gly Leu Leu Pro Ser
                   85                  90                  95
   Gly Ser Ala Glu Pro Pro Gly Asp Trp Asp Gly Gly Gly Gly Ser Tyr
               100                 105                 110
   Arg Leu Leu Arg Gly Gly Leu Leu Gly Leu Gly Leu Thr Val Ser Leu
           115                 120                 125
   Leu Ser His Cys Leu Val Ala Leu Asn Arg Tyr Leu Leu Ile Thr Arg
       130                 135                 140
   Ala Pro Ala Thr Tyr Gln Val Leu Tyr Gln Arg Arg His Thr Val Gly
   145                 150                 155                 160
   Met Leu Ala Leu Ser Trp Ala Leu Ala Leu Gly Leu Val Leu Leu Leu
                   165                 170                 175
   Pro Pro Trp Ala Pro Lys Pro Gly Ala Glu Pro Pro Gln Val His Tyr
               180                 185                 190
   Pro Ala Leu Leu Ala Ala Gly Ala Leu Leu Ala Gln Thr Ala Leu Leu
           195                 200                 205
   Leu His Cys Tyr Leu Gly Ile Val Arg Arg Val Arg Val Ser Val Lys
       210                 215                 220
   Arg Val Ser Val Leu Asn Phe His Leu Leu His Gln Leu Pro Gly Cys
   225                 230                 235                 240
   Ala Ala Ala Ala Ala Ala Phe Pro Ala Ala Pro His Ala Pro Gly Pro
                   245                 250                 255
   Gly Gly Ala Ala His Pro Ala Gln Pro Gln Pro Leu Pro Ala Ala Leu
               260                 265                 270
   Gln Pro Arg Arg Ala Gln Arg Arg Leu Ser Gly Leu Ser Val Leu Leu
           275                 280                 285
   Leu Cys Cys Val Phe Leu Leu Ala Thr Gln Pro Leu Val Trp Val Ser
       290                 295                 300
   Leu Ala Ser Gly Phe Ser Leu Pro Val Pro Trp Gly Val Gln Ala Ala
   305                 310                 315                 320
   Ser Trp Leu Leu Cys Cys Ala Leu Ser Ala Leu Asn Pro Leu Leu Tyr
                   325                 330                 335
   Thr Trp Arg Asn Glu Glu Phe Arg Arg Ser Val Arg Ser Val Leu Pro
               340                 345                 350
   Gly Val Gly Asp Ala Ala Ala Ala Ala Ala Ala Ala Thr Ala Val Pro
           355                 360                 365
   Ala Met Ser Gln Ala Gln Leu Gly Thr Arg Ala Ala Gly Gln His Trp
       370                 375                 380
<210> 16
   <211> 1155
   <212> DNA
   <213> Mus musculus
<400> 16
   atgaccaact cctcctccac ttcgacctcc accactaccg ggggatcgct gctgctgctc 60
   tgcgaggaag aagaatcgtg ggcgggccgg cggatcccgg tgtctctcct gtactcgggc 120
   ctggccatcg ggggcacgct ggccaacggt atggtcatct atctcgtgtc gtccttccga 180
   aagctgcaga ccaccagcaa cgccttcatc gtgaatggct gcgccgcgga tctcagcgtc 240
   tgcgccctct ggatgccaca ggaggcggtg ctggggctcc tgccctcggg ctccgcggag 300
   cccccggggg actgggacgg tggcggaggc agctaccgcc tgctccgggg cgggttgttg 360
   ggcctcgggc tcactgtgtc ccttctgtcc cattgcctcg tggcgctgaa ccgctacctg 420
   ctcatcacca gggcgcctgc cacctaccag gtgctctacc aacggcgcca cacggtgggc 480
   atgctggcac tgtcctgggc gctcgccttg ggtctggtgc tgctgctccc gccctgggct 540
   cctaagcccg gagccgagcc accgcaagtc cactaccccg cgctcctggc ggcgggagcg 600
   ctgcttgcgc agacggcgct gctgttgcac tgctacctgg gcatcgtgcg ccgcgtgcgc 660
   gtcagcgtca agcgggtcag cgtgctcaac ttccacctgc tgcaccagct acccggctgc 720
   gccgccgccg ccgccgcctt ccccgccgcc ccgcacgcgc cgggtccggg aggcgccgcg 780
   catcccgcgc agcctcaacc cctgccggcc gctctgcagc ctcgccgggc gcagcggcgt 840
   ctcagcggct tgtcggtgct gctgctctgc tgcgtcttcc tgctggccac gcagccgctg 900
   gtgtgggtga gcctggccag tggcttctcc ctcccggtgc cctggggcgt gcaggcggcc 960
   agctggctcc tgtgctgcgc cctgtcggcg ctcaacccgc tgctctacac gtggaggaac 1020
   gaggagttcc gcagatccgt gcgatctgtc ctgccgggcg tcggggacgc ggctgcggcc 1080
   gccgcggccg ccaccgcggt gccagccatg tcccaggcgc aactgggtac ccgggcggct 1140
   ggccagcact ggtaa                                                  1155
<210> 17
   <211> 433
   <212> PRT
   <213> Homo sapiens
<400> 17
   Met Cys Phe Ser Pro Ile Leu Glu Ile Asn Met Gln Ser Glu Ser Asn
    1               5                  10                  15
   Ile Thr Val Arg Asp Asp Ile Asp Asp Ile Asn Thr Asn Met Tyr Gln
               20                  25                  30
   Pro Leu Ser Tyr Pro Leu Ser Phe Gln Val Ser Leu Thr Gly Phe Leu
           35                  40                  45
   Met Leu Glu Ile Val Leu Gly Leu Gly Ser Asn Leu Thr Val Leu Val
       50                  55                  60
   Leu Tyr Cys Met Lys Ser Asn Leu Ile Asn Ser Val Ser Asn Ile Ile
   65                  70                  75                  80
   Thr Met Asn Leu His Val Leu Asp Val Ile Ile Cys Val Gly Cys Ile
                   85                  90                  95
   Pro Leu Thr Ile Val Ile Leu Leu Leu Ser Leu Glu Ser Asn Thr Ala
               100                 105                 110
   Leu Ile Cys Cys Phe His Glu Ala Cys Val Ser Phe Ala Ser Val Ser
           115                 120                 125
   Thr Ala Ile Asn Val Phe Ala Ile Thr Leu Asp Arg Tyr Asp Ile Ser
       130                 135                 140
   Val Lys Pro Ala Asn Arg Ile Leu Thr Met Gly Arg Ala Val Met Leu
   145                 150                 155                 160
   Met Ile Ser Ile Trp Ile Phe Ser Phe Phe Ser Phe Leu Ile Pro Phe
                   165                 170                 175
   Ile Glu Val Asn Phe Phe Ser Leu Gln Ser Gly Asn Thr Trp Glu Asn
               180                 185                 190
   Lys Thr Leu Leu Cys Val Ser Thr Asn Glu Tyr Tyr Thr Glu Leu Gly
           195                 200                 205
   Met Tyr Tyr His Leu Leu Val Gln Ile Pro Ile Phe Phe Phe Thr Val
       210                 215                 220
   Val Val Met Leu Ile Thr Tyr Thr Lys Ile Leu Gln Ala Leu Asn Ile
   225                 230                 235                 240
   Arg Ile Gly Thr Arg Phe Ser Thr Gly Gln Lys Lys Lys Ala Arg Lys
                   245                 250                 255
   Lys Lys Thr Ile Ser Leu Thr Thr Gln His Glu Ala Thr Asp Met Ser
               260                 265                 270
   Gln Ser Ser Gly Gly Arg Asn Val Val Phe Gly Val Arg Thr Ser Val
           275                 280                 285
   Ser Val Ile Ile Ala Leu Arg Arg Ala Val Lys Arg His Arg Glu Arg
       290                 295                 300
   Arg Glu Arg Gln Lys Arg Val Phe Arg Met Ser Leu Leu Ile Ile Ser
   305                 310                 315                 320
   Thr Phe Leu Leu Cys Trp Thr Pro Ile Ser Val Leu Asn Thr Thr Ile
                   325                 330                 335
   Leu Cys Leu Gly Pro Ser Asp Leu Leu Val Lys Leu Arg Leu Cys Phe
               340                 345                 350
   Leu Val Met Ala Tyr Gly Thr Thr Ile Phe His Pro Leu Leu Tyr Ala
           355                 360                 365
   Phe Thr Arg Gln Lys Phe Gln Lys Val Leu Lys Ser Lys Met Lys Lys
       370                 375                 380
   Arg Val Val Ser Ile Val Glu Ala Asp Pro Leu Pro Asn Asn Ala Val
   385                 390                 395                 400
   Ile His Asn Ser Trp Ile Asp Pro Lys Arg Asn Lys Lys Ile Thr Phe
                   405                 410                 415
   Glu Asp Ser Glu Ile Arg Glu Lys Arg Leu Val Pro Gln Val Val Thr
               420                 425                 430
   Asp
<210> 18
   <211> 1302
   <212> DNA
   <213> Homo sapiens
<400> 18
   atgtgttttt ctcccattct ggaaatcaac atgcagtctg aatctaacat tacagtgcga 60
   gatgacattg atgacatcaa caccaatatg taccaaccac tatcatatcc gttaagcttt 120
   caagtgtctc tcaccggatt tcttatgtta gaaattgtgt tgggacttgg cagcaacctc 180
   actgtattgg tactttactg catgaaatcc aacttaatca actctgtcag taacattatt 240
   acaatgaatc ttcatgtact tgatgtaata atttgtgtgg gatgtattcc tctaactata 300
   gttatccttc tgctttcact ggagagtaac actgctctca tttgctgttt ccatgaggct 360
   tgtgtatctt ttgcaagtgt ctcaacagca atcaacgttt ttgctatcac tttggacaga 420
   tatgacatct ctgtaaaacc tgcaaaccga attctgacaa tgggcagagc tgtaatgtta 480
   atgatatcca tttggatttt ttcttttttc tctttcctga ttccttttat tgaggtaaat 540
   tttttcagtc ttcaaagtgg aaatacctgg gaaaacaaga cacttttatg tgtcagtaca 600
   aatgaatact acactgaact gggaatgtat tatcacctgt tagtacagat cccaatattc 660
   tttttcactg ttgtagtaat gttaatcaca tacaccaaaa tacttcaggc tcttaatatt 720
   cgaataggca caagattttc aacagggcag aagaagaaag caagaaagaa aaagacaatt 780
   tctctaacca cacaacatga ggctacagac atgtcacaaa gcagtggtgg gagaaatgta 840
   gtctttggtg taagaacttc agtttctgta ataattgccc tccggcgagc tgtgaaacga 900
   caccgtgaac gacgagaaag acaaaagaga gtcttcagga tgtctttatt gattatttct 960
   acatttcttc tctgctggac accaatttct gttttaaata ccaccatttt atgtttaggc 1020
   ccaagtgacc ttttagtaaa attaagattg tgttttttag tcatggctta tggaacaact 1080
   atatttcacc ctctattata tgcattcact agacaaaaat ttcaaaaggt cttgaaaagt 1140
   aaaatgaaaa agcgagttgt ttctatagta gaagctgatc ccctgcctaa taatgctgta 1200
   atacacaact cttggataga tcccaaaaga aacaaaaaaa ttacctttga agatagtgaa 1260
   ataagagaaa aacgtttagt gcctcaggtt gtcacagact ag                    1302
<210> 19
   <211> 432
   <212> PRT
   <213> Mus musculus
<400> 19
   Met Cys Phe Ser Pro Val Leu Glu Ile Asn Met Gln Ser Glu Ser Asn
   1               5                   10                  15
   Val Thr Val Arg Asp Asp Ile Asp Asp Ile Asp Thr Asn Met Tyr Gln
               20                  25                  30
   Pro Leu Ser Tyr Pro Leu Ser Phe Gln Val Ser Leu Thr Gly Phe Leu
           35                  40                  45
   Met Leu Glu Ile Val Leu Gly Leu Gly Ser Asn Leu Thr Val Leu Val
       50                  55                  60
   Leu Tyr Cys Met Lys Ser Asn Leu Ile Asn Ser Val Ser Asn Ile Ile
   65                  70                  75                  80
   Thr Met Asn Leu His Val Leu Asp Val Ile Ile Cys Val Gly Cys Ile
                   85                  90                  95
   Pro Leu Thr Ile Val Ile Leu Leu Leu Ser Leu Glu Ser Asn Thr Ala
               100                 105                 110
   Leu Ile Cys Cys Phe His Glu Ala Cys Val Ser Phe Ala Ser Val Ser
           115                 120                 125
   Thr Ala Ile Asn Val Phe Ala Ile Thr Leu Asp Arg Tyr Asp Ile Ser
       130                 135                 140
   Val Lys Pro Ala Asn Arg Ile Leu Thr Met Gly Arg Ala Val Met Leu
   145                 150                 155                 160
   Met Thr Ser Ile Trp Ile Phe Ser Phe Phe Ser Phe Leu Ile Pro Phe
                   165                 170                 175
   Ile Glu Val Asn Phe Phe Ser Leu Gln Ser Gly Asn Thr Trp Ala Asn
               180                 185                 190
   Lys Thr Leu Leu Cys Val Ser Thr Ser Glu Tyr Tyr Thr Glu Leu Gly
           195                 200                 205
   Met Tyr Tyr His Leu Leu Val Gln Ile Pro Ile Phe Phe Phe Thr Val
       210                 215                 220
   Ile Val Met Leu Ile Thr Tyr Thr Lys Ile Leu Gln Ala Leu Asn Ile
   225                 230                 235                 240
   Arg Ile Gly Thr Arg Phe Ser Thr Gly Gln Lys Lys Lys Ala Arg Lys
                   245                 250                 255
   Lys Lys Thr Ile Ser Leu Ala Thr His Glu Thr Thr Asp Met Ser Gln
               260                 265                 270
   Ser Ser Gly Gly Arg Asn Val Val Phe Gly Val Arg Thr Ser Val Ser
           275                 280                 285
   Val Ile Ile Ala Leu Arg Arg Ala Val Lys Arg His Arg Glu Arg Arg
       290                 295                 300
   Glu Arg Gln Lys Arg Val Phe Lys Met Ser Leu Leu Ile Ile Ser Thr
   305                 310                 315                 320
   Phe Leu Leu Cys Trp Thr Pro Ile Ser Val Leu Asn Thr Thr Ile Leu
                   325                 330                 335
   Cys Leu Gly Pro Ser Asp Leu Leu Val Lys Leu Arg Leu Cys Phe Leu
               340                 345                 350
   Val Met Ala Tyr Gly Thr Thr Ile Phe His Pro Leu Leu Tyr Ala Phe
           355                 360                 365
   Thr Arg Gln Lys Phe Gln Lys Val Leu Lys Ser Lys Met Lys Lys Arg
       370                 375                 380
   Val Val Ser Ile Val Glu Ala Asp Pro Met Pro Asn Asn Ala Val Ile
   385                 390                 395                 400
   His Asn Ser Trp Ile Asp Pro Lys Arg Asn Lys Lys Val Thr Tyr Glu
                   405                 410                 415
   Asp Ser Glu Ile Arg Glu Lys Cys Leu Val Pro Gln Val Val Thr Asp
               420                 425                 430
<210> 20
   <211> 1296
   <212> DNA
   <213> Mus musculus
<400> 20
   atgtgttttt ctcctgttct ggaaatcaac atgcagtctg aatcaaacgt cacggtgcga 60
   gatgacattg atgacatcga caccaatatg taccaaccac tgtcataccc actaagcttt 120
   caagtgtctc tcactggatt tctcatgtta gagatcgtgc tggggcttgg cagcaacctt 180
   accgtcctgg tactttactg catgaaatcc aacttaatca actctgtcag taacattatt 240
   acaatgaacc tccatgtact tgatgtcata atttgtgtgg gatgcattcc tctaactata 300
   gtgatccttc tgctctcact ggagagtaac actgctctca tctgctgttt ccacgaagct 360
   tgtgtttcct ttgcaagtgt ttcgacagca atcaacgttt ttgctattac tctggacaga 420
   tatgacatct ctgtaaaacc tgcaaacaga attctgacaa tgggcagagc tgtaatgcta 480
   atgacatcca tttggatttt ttctttcttc tcattcctga ttcccttcat tgaagtaaat 540
   tttttcagtc ttcaaagtgg aaatacatgg gcaaacaaga cactgctgtg tgtcagtaca 600
   agtgaatact atactgagct cgggatgtac tatcaccttt tggtgcagat ccccatcttc 660
   ttcttcacag ttatagtcat gttgatcaca tacactaaga tactccaggc tcttaacatc 720
   cgcataggca ctagattctc aacaggacag aagaagaaag cccgaaagaa aaagacaatc 780
   tctctagcta cacatgagac cacagacatg tcacaaagca gtggtgggag gaatgtcgtg 840
   tttggtgtga gaacttcagt ttctgtaata attgccctcc ggcgagccgt gaaacgccac 900
   cgggaacgac gagaacggca gaaaagagtc ttcaaaatgt cgttattgat tatttctaca 960
   tttcttctct gttggacacc aatttctgtt ttaaatacca ccattctatg tttaggccca 1020
   agtgaccttt tagtaaaatt aagattgtgt tttctagtca tggcttatgg aacaacgata 1080
   ttccaccctc tcttgtatgc attcaccaga caaaagtttc aaaaggtctt aaagagtaag 1140
   atgaaaaagc gagttgtttc catagttgaa gctgatccca tgcctaataa cgctgtaata 1200
   cacaactcat ggatagatcc taaaagaaac aaaaaggtta cctatgaaga cagtgaaata 1260
   agagagaaat gtttagtacc tcaggttgtc acagac                           1296